# EUROPEAN PATENT SPECIFICATION

(11) **EP 3 099 792 B1**
(45) Date of publication and mention of the grant of the patent: **17.10.2018**
(21) Application number: 15703352.3
(22) Date of filing: 15.01.2015
(51) Int. Cl.: C12N 9/24, C12P 7/64, C11B 1/02

(54) **METHODS FOR IMPROVING BY-PRODUCTS FROM FERMENTATION PROCESSES USING XYLANASE**
VERFAHREN ZUR VERBESSERUNG VON NEBENPRODUKTEN FERMENTATIONSVERFAHREN MIT XYLANASE
PROCÉDÉ POUR AMÉLIORER LES PRODUITS SECONDAIRES DE PROCÉDÉS DE FERMENTATION À L'AIDE DE XYLANASE

(30) Priority: 31.01.2014 US 201461934612 P
(43) Date of publication of application: 07.12.2016
(73) Proprietor: Danisco US Inc., Palo Alto, California 94304 (US)
(72) Inventor: TEUNISSEN, Paula Johanna Maria, Palo Alto, California 94304 (US); PEPSIN, Michael Jay, Palo Alto, California 94304 (US); SHARMA, Vivek, Palo Alto, California 94304 (US)
(74) Representative: Kiddle, Simon John
(86) International application number: PCT/US2015/011527
(87) International publication number: WO 2015/116395

(56) References cited:
- WO-A1-2014/020142
- WO-A2-03/106654
- DATABASE UniProt [Online] 26 June 2013 (2013-06-26), "RecName: Full=Beta-xylanase {ECO:0000256|RuleBase:RU361174}; EC=3.2.1.8 {ECO:0000256|RuleBase:RU361174};", XP002738576, retrieved from EBI accession no. UNIPROT:N4V5T0 Database accession no. N4V5T0
- DATABASE UniProt [Online] 26 June 2013 (2013-06-26), "RecName: Full=Beta-xylanase {ECO:0000256|RuleBase:RU361174}; EC=3.2.1.8 {ECO:0000256|RuleBase:RU361174};", XP002738577, retrieved from EBI accession no. UNIPROT:N4WYQ3 Database accession no. N4WYQ3
- DATABASE UniProt [Online] 13 October 2009 (2009-10-13), "RecName: Full=Beta-xylanase {ECO:0000256|RuleBase:RU361174}; EC=3.2.1.8 {ECO:0000256|RuleBase:RU361174};", XP002738578, retrieved from EBI accession no. UNIPROT:C7Z894 Database accession no. C7Z894
- DATABASE UniProt [Online] 28 November 2012 (2012-11-28), "RecName: Full=Beta-xylanase {ECO:0000256|RuleBase:RU361174}; EC=3.2.1.8 {ECO:0000256|RuleBase:RU361174};", XP002738579, retrieved from EBI accession no. UNIPROT:K3VD03 Database accession no. K3VD03
- DATABASE UniProt [Online] 14 May 2014 (2014-05-14), "RecName: Full=Beta-xylanase {ECO:0000256|RuleBase:RU361174}; EC=3.2.1.8 {ECO:0000256|RuleBase:RU361174};", XP002738580, retrieved from EBI accession no. UNIPROT:X0C0Q4 Database accession no. X0C0Q4
- DATABASE UniProt [Online] 26 June 2013 (2013-06-26), "RecName: Full=Beta-xylanase {ECO:0000256|RuleBase:RU361174}; EC=3.2.1.8 {ECO:0000256|RuleBase:RU361174};", XP002738581, retrieved from EBI accession no. UNIPROT:N4UXB3 Database accession no. N4UXB3

## Description

### CROSS REFERENCE TO RELATED APPLICATIONS

This application claims benefit of priority from United States Provisional Patent Application Serial No. 61/934,612, filed on 31 January 2014.

### FIELD OF THE INVENTION

The present invention relates to methods for improving by-products (e.g., oil) from fermentation processes including use of xylanases, including modified xylanases, e.g., in the treatment of arabinoxylan containing raw materials like grain-based materials, e.g. in the production of biofuel or other fermentation products, including biochemicals.

### BACKGROUND OF THE INVENTION

For many years, endo-β-1,4-xylanases (EC 3.2.1.8) (referred to herein as xylanases) have been used for the modification of complex carbohydrates derived from plant cell wall material. It is well known in the art that the functionality of different xylanases (derived from different microorganisms or plants) differs enormously. Xylanase is the name given to a class of enzymes which degrade the linear polysaccharide beta-1,4-xylan into xylooligosaccharides or xylose, thus breaking down hemicellulose, one of the major components of plant cell walls.

Based on structural and genetic information, xylanases have been classified into different Glycoside Hydrolase (GH) families (Henrissat, (1991) Biochem. J. 280, 309-316).

Initially all known and characterized xylanases belonged to the families GH10 or GH11. Further work then identified numerous other types of xylanases belonging to the families GH5, GH7, GH8 and GH43 (Collins et al (2005) FEMS Microbiol Rev., 29 (1), 3-23).

Until now the GH11 family differs from all other GH's, being the only family solely consisting of xylan specific xylanases. The structure of the GH11 xylanases can be described as a β-Jelly roll structure or an all β-strand sandwich fold structure (Himmel et al 1997 Appl. Biochem. Biotechnol. 63-65, 315-325). GH11 enzymes have a catalytic domain of around 20kDa.

GH10 xylanases have a catalytic domain with molecular weights in the range of 32-39kDa. The structure of the catalytic domain of GH10 xylanases consists of an eightfold β/α barrel (Harris et al 1996 - Acta. Crystallog. Sec. D 52, 393-401).

Three-dimensional structures are available for a large number of Family GH10 enzymes, the first solved being those of the *Streptomyces lividans* xylanase A (Derewenda et al J Biol Chem 1994 Aug 19; 269(33) 20811-4), the *C. fimi* endo-glycanase Cex (White et al Biochemistry 1994 Oct 25; 33(42) 12546-52), and the *Cellvibrio japonicus* Xyn10A (previously *Pseudomonas fluorescens* subsp. xylanase A) (Harris et al Structure 1994 Nov 15; 2(11) 1107-16.). As members of Clan GHA they have a classical (α/β)₈ TIM barrel fold with the two key active site glutamic acids located at the C-terminal ends of beta-strands 4 (acid/base) and 7 (nucleophile) (Henrissat et al Proc Natl Acad Sci U S A 1995 Jul 18; 92(15) 7090-4).

Comprehensive studies characterising the functionality of xylanases have been done on well characterised and pure substrates (Kormelink et al., 1992 Characterisation and mode of action of xylanases and some accessory enzymes. Ph.D. Thesis, Agricultural University Wageningen, Holland (175 pp., English and Dutch summaries)). These studies show that different xylanases have different specific requirements with respect to substitution of the xylose backbone of the arabinoxylan (AX). Some xylanases require three un-substituted xylose residues to hydrolyse the xylose backbone; others require only one or two. The reasons for these differences in specificity are thought to be due to the three dimensional structure within the catalytic domains, which in turn is dependent on the primary structure of the xylanase, i.e. the amino acid sequence. However, the translation of these differences in the amino acid sequences into differences in the functionality of the xylanases, has up until now not been documented when the xylanase acts in a complex environment, such as a plant material, e.g. in a feedstuff.

The xylanase substrates in plant material, e.g. in wheat, have traditionally been divided into two fractions: The water un-extractable AX (WU-AX) and the water extractable AX (WE-AX). There have been numerous explanations as to why there are two different fractions of AX. The older literature (D'Appolonia and MacArthur - (1976, Cereal Chem. 53. 711-718) and Montgomery and Smith (1955, J. Am. Chem. Soc. 77. 3325-332) describes quite high differences in the substitution degree between WE-AX and WU-AX. The highest degree of substitution was found in WE-AX. This was used to explain why some of the AX was extractable. The high degree of substitution made the polymer soluble, compared to a lower substitution degree, which would cause hydrogen bonding between polymers and consequently precipitation.

The difference between the functionality of different xylanases has been thought to be due to differences in xylanase specificity and thereby their preference for the WU-AX or the WE-AX substrates.

WO 03/106654 describes a series of xylanases screened from a lambda library.

Xylanase enzymes have been reported from nearly 100 different organisms, including plants, fungi and bacteria. The xylanase enzymes are classified into several of the more than 40 families of glycosyl hydrolase enzymes. The glycosyl hydrolase enzymes, which include xylanases, mannanases, amylases, β-glucanases, cellulases, and other carbohydrases, are classified based on such properties as the sequence of amino acids, their three dimensional structure and the geometry of their catalytic site (Gilkes, et al., 1991, Microbiol. Reviews 55: 303-315).

### BRIEF DESCRIPTION OF THE DRAWINGS

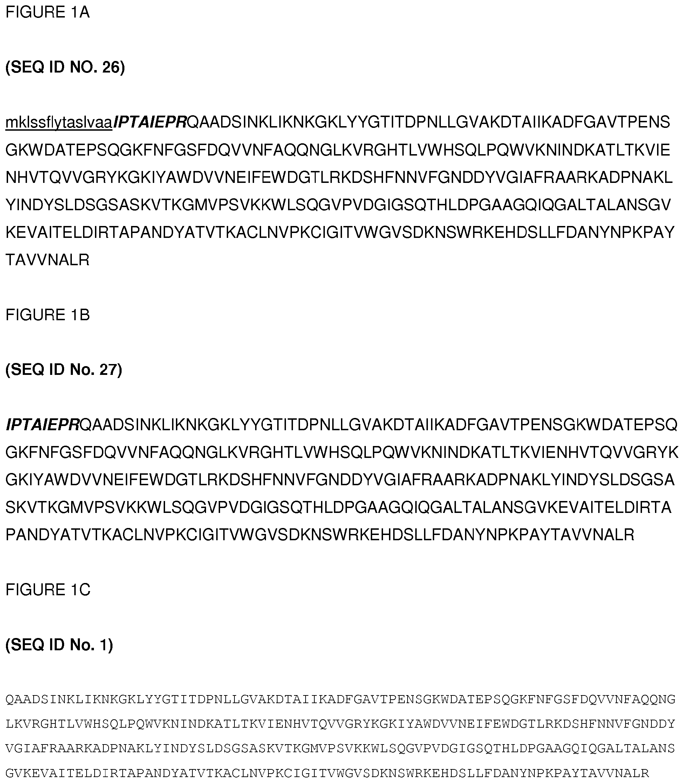

Figure 1A shows a polypeptide sequence (SEQ ID No. 26) of a xylanase of the present invention (FveXyn4). Underlined (lower case) portion of the sequence reflects an N terminal signal peptide can be cleaved before the enzyme is matured. The amino acids shown in bold and italicized may also be cleaved by post-translational modification before the enzyme is fully matured. In some embodiments this sequence may be the backbone sequence.
Figure 1B shows a polypeptide sequence (SEQ ID No. 27) of a xylanase of the present invention (FveXyn4). The amino acids shown in bold and italicized may also be cleaved by post-translational modification before the enzyme is fully matured. In some embodiments this sequence may be the backbone sequence.
Figure 1C shows a polypeptide sequence (SEQ ID No. 1) of a xylanase referred to herein as FveXyn4. This is the active form of the enzyme. This may be referred to herein as the mature form of the enzyme (in some embodiments this sequence a backbone sequence).

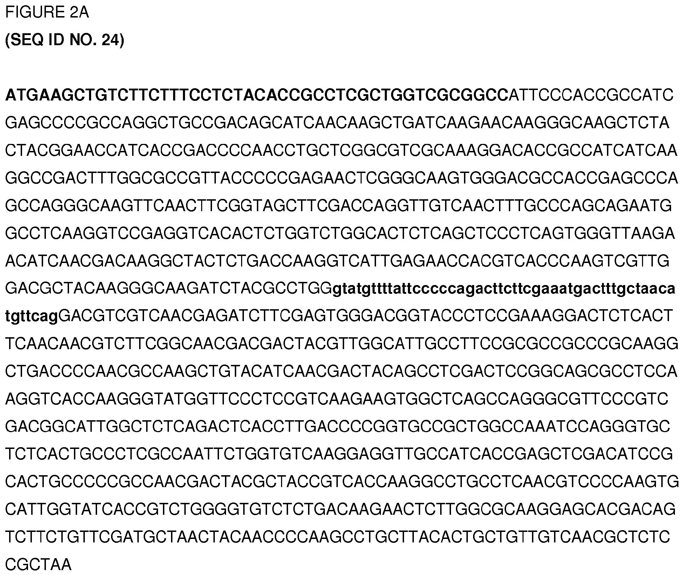

Figure 2A shows a nucleotide sequence (SEQ ID No. 24) encoding a xylanase of the present invention (FveXyn4). The lower case nucleotides which are in bold show the intron sequence. The sequence that encodes the signal sequence is shown bold (upper case).

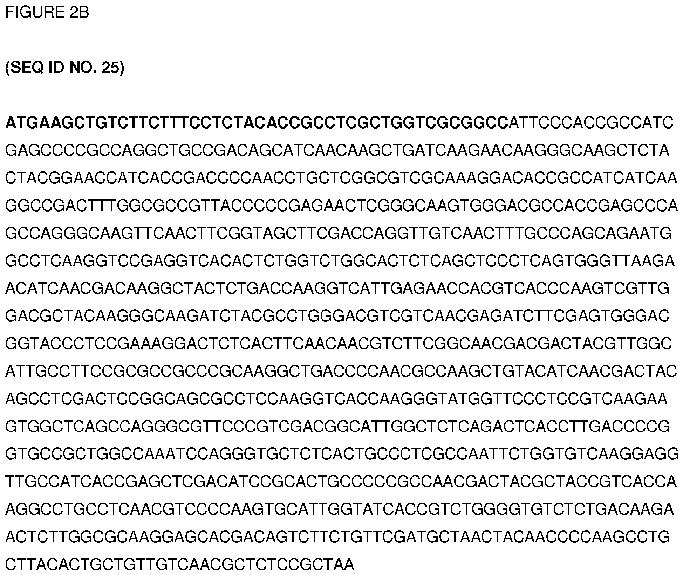

Figure 2B shows a nucleotide sequence (SEQ ID No. 25) encoding a xylanase of the present invention (FveXyn4). The sequence that encodes the signal sequence is shown bold (upper case).

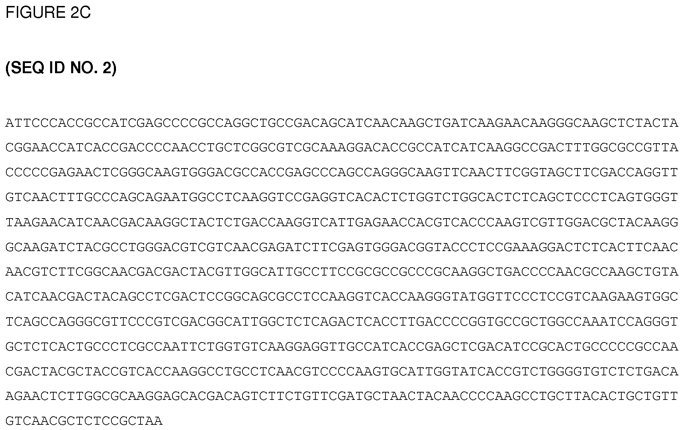

Figure 2C shows a nucleotide sequence (SEQ ID No. 2) encoding a xylanase referred to herein as FveXyn4.
Figure 3A shows a polypeptide sequence (SEQ ID No. 28) of a xylanase of the present invention (FoxXyn2). Underlined (lower case) portion of the sequence may reflect an N terminal signal peptide which can be cleaved before the enzyme is matured. The amino acids shown in bold and italicized may also be cleaved by post-translational modification before the enzyme is fully matured. In some embodiments this sequence is a backbone sequence.
Figure 3B shows a polypeptide sequence (SEQ ID No. 29) of a xylanase of the present invention (FoxXyn2). The amino acids shown in bold and italicized may also be cleaved by post-translational modification before the enzyme is fully matured. This sequence may be an active form of the protein and may be one active form of the protein. This may be referred to herein as the mature form of the enzyme. In some embodiments this sequence is a backbone sequence.
Figure 3C shows a polypeptide sequence (SEQ ID No. 3) of a xylanase referred to herein as FoxXyn2. This is another active form of the enzyme. In some embodiments, this may be referred to herein as the mature form of the enzyme. In some embodiments this sequence is a backbone sequence.
Figure 4A shows a nucleotide sequence (SEQ ID No. 30) encoding a xylanase of the present invention (FoxXyn2). The lower case nucleotides which are in bold show the intron sequence. The sequence that encodes the signal sequence is shown bold (upper case).
Figure 4B shows a nucleotide sequence (SEQ ID No. 31) encoding a xylanase of the present invention (FoxXyn2). The sequence that encodes the signal sequence is shown bold (upper case).
Figure 4C shows a nucleotide sequence (SEQ ID No. 4) encoding a xylanase referred to herein as FoxXyn2.

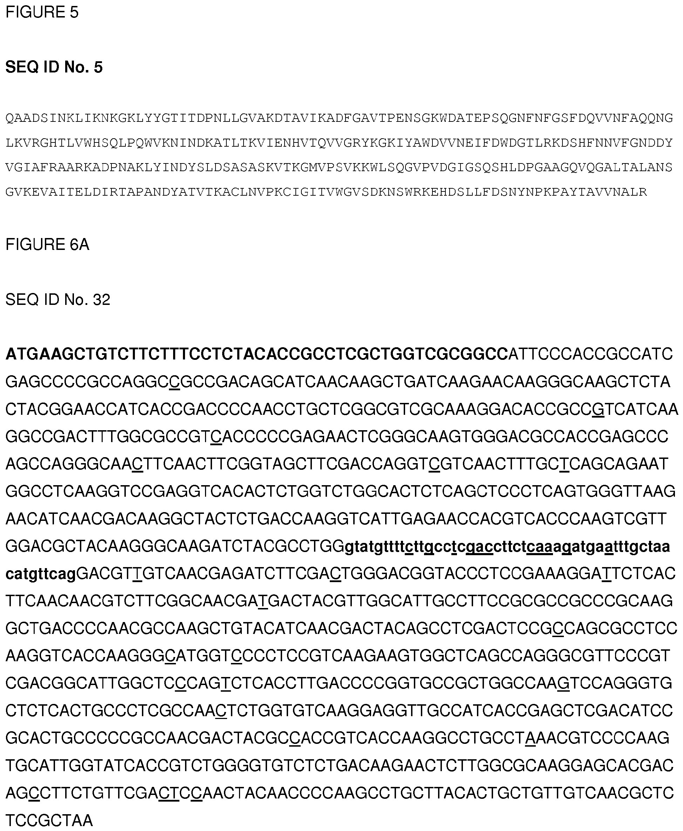

Figure 5 shows a polypeptide sequence (SEQ ID No. 5) of a xylanase from Fusarium - Fusarium Comparative Sequencing Project, Broad Institute of Harvard and MIT (http://www.broadinstitute.org/)). In some embodiments, this sequence is a backbone sequence.
Figure 6A shows a nucleotide sequence (SEQ ID No. 32) encoding a xylanase for use in the present invention from Fusarium - obtained from Fusarium Comparative Sequencing Project, Broad Institute of Harvard and MIT (http://www.broadinstitute.org/)). The lower case nucleotides which are in bold show the intron sequence. The sequence that encodes the signal sequence is shown bold (upper case). Changes compared with SEQ ID No. 24 are underlined.

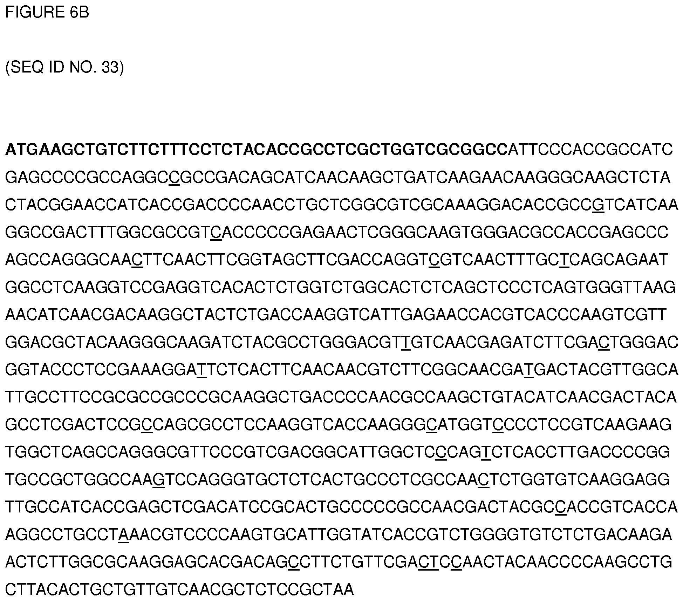

Figure 6B shows a nucleotide sequence (SEQ ID No. 33) encoding a xylanase for use in the present invention from Fusarium - obtained from Fusarium Comparative Sequencing Project, Broad Institute of Harvard and MIT (http://www.broadinstitute.org/)). The sequence that encodes the signal sequence is shown bold (upper case). Changes compared with SEQ ID No. 25 are underlined.

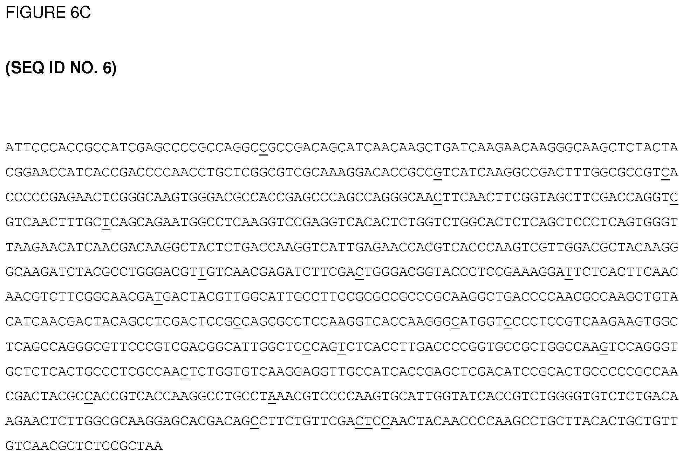

Figure 6C shows a nucleotide sequence (SEQ ID No. 6) encoding a xylanase for use in the present invention from Fusarium - obtained from Fusarium Comparative Sequencing Project, Broad Institute of Harvard and MIT (http://www.broadinstitute.org/)), changes compared with SEQ ID No. 4 are underlined.

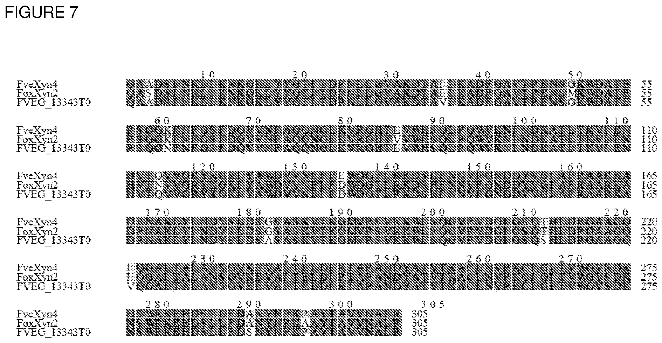

Figure 7 shows an alignment of the mature proteins for FveXyn4 (SEQ ID No. 1), FoxXyn2 (SEQ ID No. 3) and the xylanase shown herein as SEQ ID No. 5 (FVEG_13343T0).

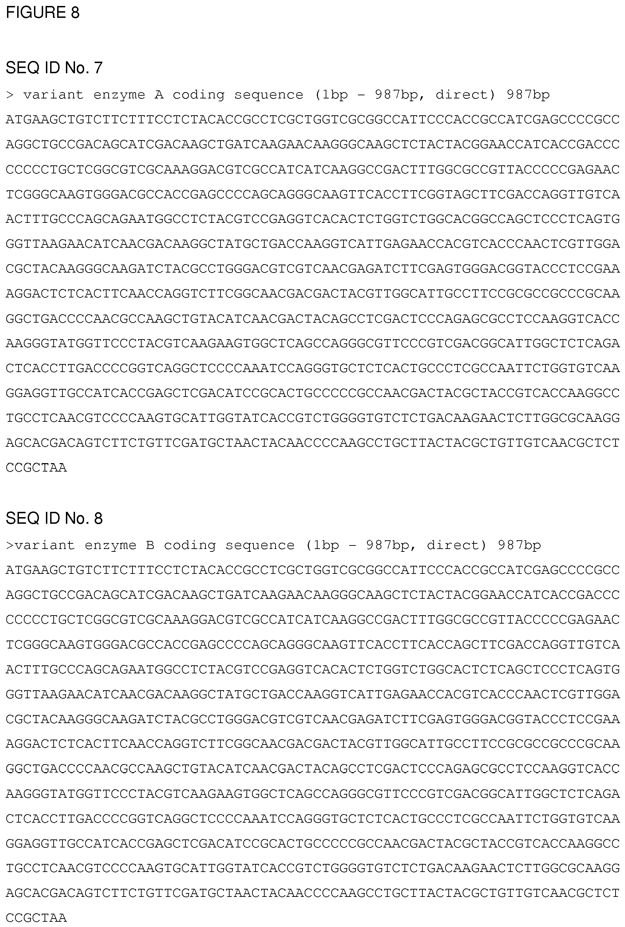

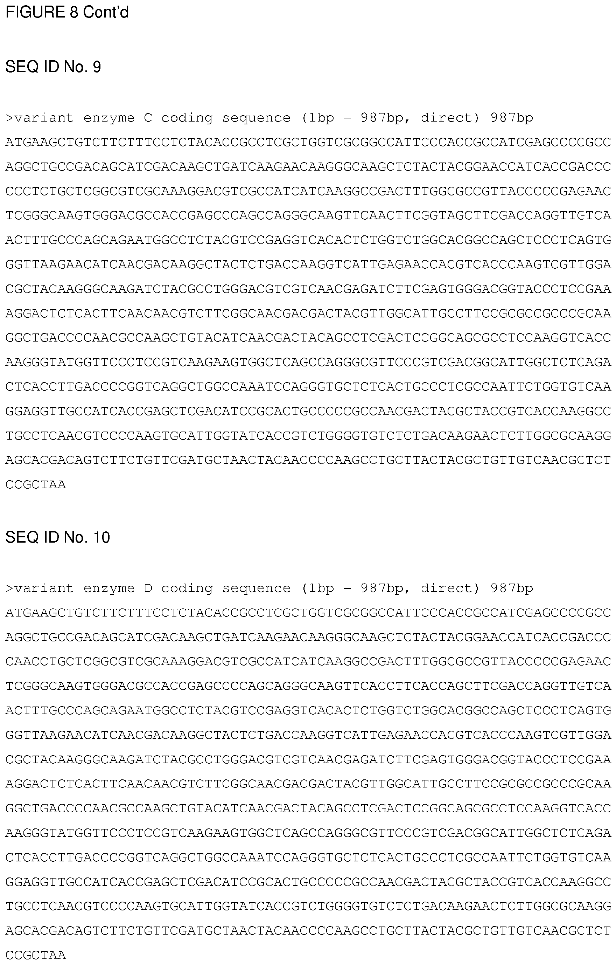

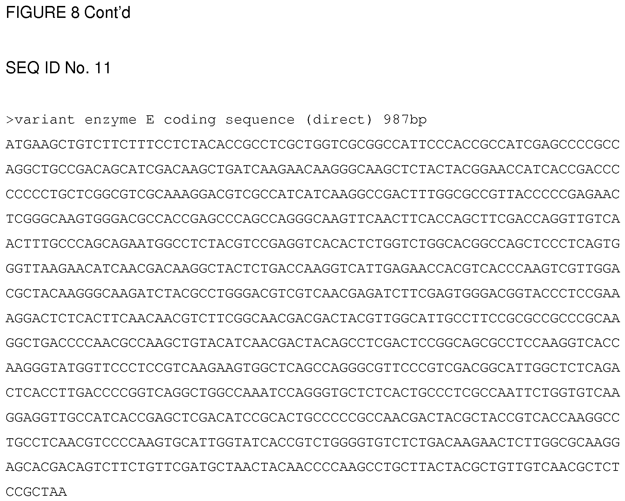

Figure 8 shows nucleotide sequences (without introns) of the coding sequences of variant GH10 xylanases in accordance with the present invention (SEQ ID No. 7, SEQ ID No. 8, SEQ ID No. 9, SEQ ID No. 10, and SEQ ID No. 11).

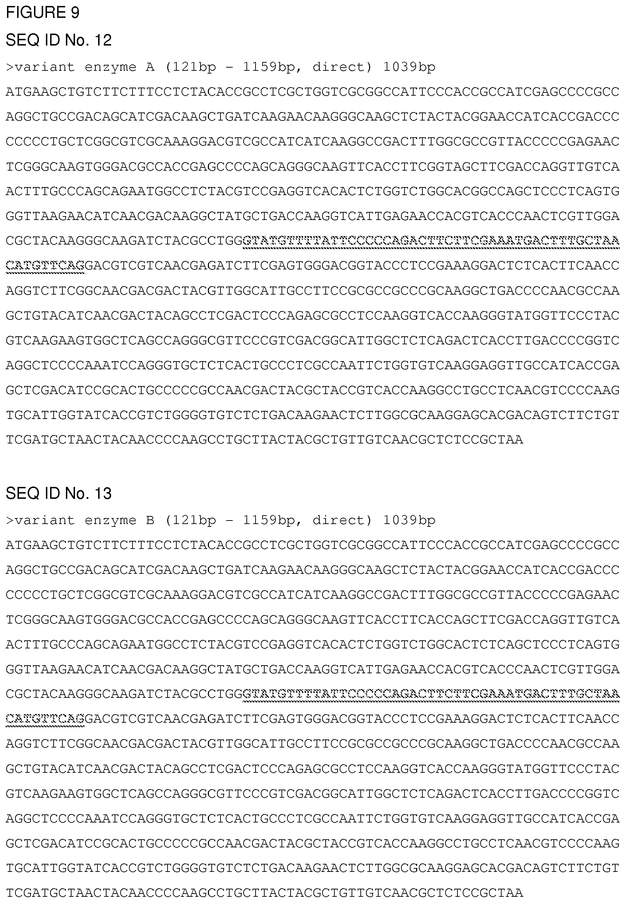

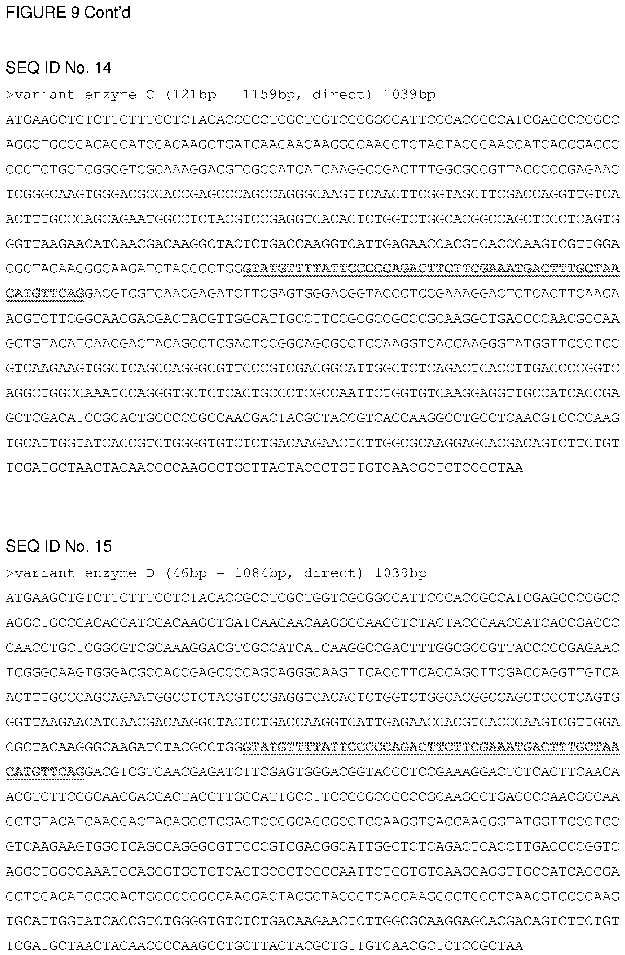

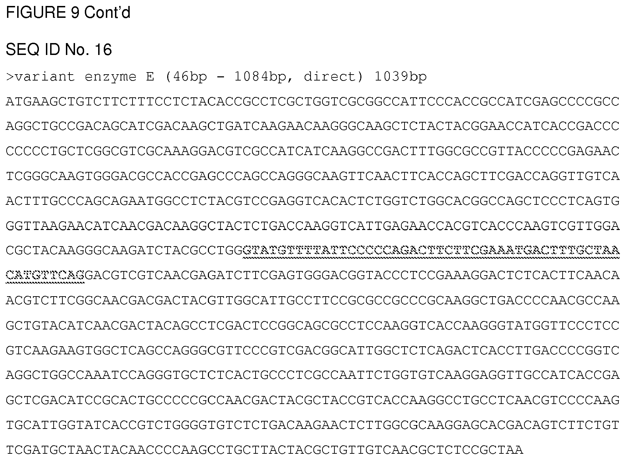

Figure 9 shows nucleotide sequences (with introns shown underlined) of the coding sequences of variant GH10 xylanases in accordance with the present invention (SEQ ID No. 12, SEQ ID No. 13, SEQ ID No. 14, SEQ ID No. 15, and SEQ ID No. 16).

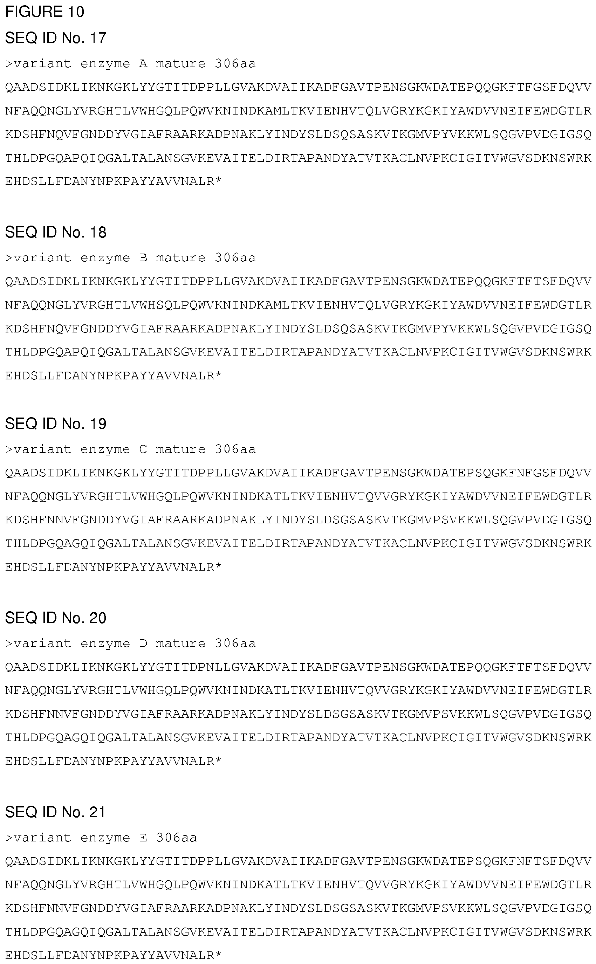

Figure 10 shows amino acid sequences of mature variant GH10 xylanases in accordance with the present invention (SEQ ID No. 17, SEQ ID No. 18, SEQ ID No. 19, SEQ ID No. 20, and SEQ ID No. 21).
Figure 11 shows the Tm value of the 5 variants A, B, C, D, and E compared to FveXyn4. Tm value is measured as the temperature at which 50 % residual activity is obtained after 10 min incubation.
Figure 12 shows pH profile of the five variants measured at pH 4.0, 5.0 and 6.0 and all data are relative to FveXyn4 at pH 5.0.
Figures 13a and b show solubilisation of pentosan from cDDGS (top - Figure 13a) and wheat bran (bottom Figure 13b) as function of xylanase dosage.
Figure 14 shows viscosity reduction in the Viscosity reduction in *in vitro* animal model assay taught in Example 1 by the variants of the present invention and compared to FveXyn4 and the benchmark Econase XT. The viscosity reduction is measured on high viscosity wheat.
Figure 15 shows xylanase recovery after in-feed pelleting at 90 and 95 °C. Activity is shown as relative to mash sample. Samples containing FveXyn4 was analyzed using the extract method, whereas samples containing variant A-E was analyzed using the slurry method, both methods described in materials and methods of Example 1.
Figure 16 shows a plasmid map of pZZH254.
Figure 17 shows the temperature profile of FveXyn4.
Figure 18 shows a plasmid map of pZZH135.
Figure 19 shows the temperature profile of FoxXyn2.
Figure 20 shows the schematic map of pEntry-FveXyn4.
Figure 21 shows schematic maps of the destination pTTTpyr2 vector and expression vectors for the FveXyn4 (pTTTpyr2-FveXyn4) and FveXyn4 variants (pTTTpyr2-FveXyn4_VAR).
Figure 22 shows viscosity reduction in grain based material of a backbone (parent) enzyme FveXyn4 compared with thermostable variants A, B, C, D, and E according to the present invention. Fve Xyn4 and the variants perform in a similar way, showing viscosity reduction of 55-67% compared to blank (only SPEZYME® CL).
Figure 23 shows the effect on oil recovery of variant E xylanase compared with FveXyn4 and DISTILLASE® SSF.
Figure 24 shows the effect on oil recovery by FveXyn4 xylanse in an oil quantification assay.

### SUMMARY OF THE INVENTION

In a first aspect, the present invention provides a xylanase enzyme having at least 70% amino acid sequence identity to the amino acid sequence of SEQ ID NO: 21, the xylanase enzyme comprising amino acids 7D, 33V, 79Y, 217Q and 298Y at positions corresponding to the amino acid numbering of SEQ ID NO: 21 and wherein the xylanase enzyme has xylanase activity and is capable of increased oil recovery from grain-based material as compared to the xylanase enzyme of SEQ ID NO: 1.

An additional finding of the present invention is the modification of a GH10 xylanase to render the GH10 xylanase improved over the parent GH10 xylanase in that the modified xylanase enzyme provides for increased oil recovery from a grain-based material.

In a further aspect, the present invention provides processes for recovering oil using the xylanases of the present invention as set out in the accompanying claims.

The other properties of the xylanases of the present invention is its ability to break down (solubilising) insoluble arabinoxylans (AXinsol).

In particular the variant xylanases of the present invention efficiently breakdown (solubilise) AXinsol from a wide range of substrates, including corn, wheat, DDGS, etc., in particular corn and corn based substrates, in particular both wheat (including wheat-based) products and corn (including corn-based products). This contrasts with prior-known enzymes, which are often inferior at solubilising AXinsol in corn or corn-based substrates or which are not efficient in both wheat- and corn-based substrates.

In addition, the variant xylanases of the present invention are particularly good at not only breaking down (solubilising) AXinsol, but also breaking down (or degrading) the solubilized polymers efficiently. By being able to efficiently (quickly) breakdown (degrade) the solubilized polymers (obtained from dissolving AXinsol), a (fast) reduction in viscosity is obtained or the solubilized polymers (obtained from dissolving AXinsol) cannot contribute to increasing viscosity. This latter effect is essential in some of the claimed applications.

Without wishing to be bound by theory, the variant enzyme of the present invention mainly releases polymers, which do not contribute to viscosity, because the released polymers are short.

Typically, conventional xylanases may breakdown AXinsol, but will often lead to an increase in viscosity of the mixture. This increased viscosity is disadvantageous in many applications.

Without wishing to be bound by theory, although some conventional xylanases breakdown AXinsol, they lead to an increase in soluble degradation products of high molecular weight, which leads to an increase in viscosity in the mixture.

Furthermore or alternatively and again without wishing to be bound by theory, conventional xylanase enzymes may breakdown AXinsol, but because they do not degrade the solubilised products of high molecular weight fast enough the viscosity in the mixture is not ideal. In contrast, with the methods and uses of the present invention, the variant xylanases breakdown AXinsol without increasing viscosity and/or whilst reducing viscosity quickly compared with conventional enzymes. Without wishing to be being bound by theory, it is believed that high molecular weight products are not formed by the enzymes of the present invention.

The enzymes of the present invention and as described herein have been found to not only breakdown (solubilise) insoluble arabinoxylans (AXinsol) from a wide range of substrates, including corn, wheat, DDGS, etc., in particular corn and corn-based substrates, in particular both wheat (including wheat-based) products and corn (including corn-based products), but also efficiently ensuring that viscosity is not raised and/or reducing viscosity. Without wishing to be being bound by theory, it is believed that high molecular weight products are not formed by the enzymes of the present invention.

Thus the present invention relates to enzymes capable of solubilising pentosans, in particular xylan-containing materials, such as arabinoxylans, in particular insoluble arabinoxylans. In particular the enzyme is particularly good at solubilising pentosans in particular xylan-containing materials, such as arabinoxylans, in particular insoluble arabinoxylans, in a broad spectrum of substrates, including corn based substrates.

The present invention further relates to enzymes capable of degrading AXsol or the breakdown products of AXinsol to ensure viscosity is not increased and/or is reduced in the reaction mixture.

Many of the xylanases commercialized for use in feedstuffs for solubilizing pentosans are GH11 enzymes. It had been considered by those skilled in the art that GH10 xylanases were not as strong at solublizing pentosans, particularly AXinsol, compared with GH11 xylanases. Surprisingly it has been found that the novel xylanase disclosed herein which is a GH10 xylanase is particularly good at degrading AXinsol in a broad spectrum of substrates, including corn based substrates. Surprisingly, the present inventors have found that the variant GH10 xylanases of the present invention outperform commercial GH11 xylanases in their ability to solubilize pentosans. In addition the variant GH10 xylanases are thermostable.

The fact that the present enzymes efficiently degrade AXinsol from corn and corn-based substrates is significantly advantageous as corn holds much more AX in the insoluble form compared with other cereals, such as wheat and rye for example. Therefore only xylanases that can breakdown AXinsol can show significant benefit to animals fed on corn-based diet, such as corn-soy diet for example.

It was completely unexpected for a GH10 xylanase to be so good at degrading AXinsol in cereals, particularly in corn or corn-based substrates.

The enzymes of the present invention are able to efficiently (and quickly) degrade the polymers and oligomers that are produced from degradation of AXinsol or that are present in grain-based material. This leads to an unexpected advantage for the GH10 xylanases taught herein in that they are particularly good in a number of applications to keep viscosity low or to reduce viscosity, e.g. in feedstuffs; in brewing and/or malting; in grain-based production of glucose, e.g. for further processing to biofuels and/or biochemicals (e.g. bio-based isoprene); or in the wheat gluten-starch separation industry for the production of starch for example.

Notably it has been found that the degradation product on average is shorter for the GH10 enzymes tested herein compared with GH11 enzymes. This means that the degradation products do not contribute to or cause an increase in viscosity.

Based on these findings, the variant xylanases according to the present invention can be used to degrade a xylan-containing material, particularly arabinoxylans, particularly AXinsol. In addition or alternatively, the xylanases according to the present invention can be used to degrade soluble polymers (e.g. oligomers) that are produced from degradation of AXinsol or that are (naturally) present in grain-based materials. Surprisingly it has been found that the variant xylanases according the present invention can be used to both degrade a xylan-containing material, particularly arabinoxylans, particularly AXinsol, and to degrade soluble polymers (e.g. oligomers) that are produced from degradation of AXinsol.

Such enzymes find useful application in many industries, including feedstuffs, malting and brewing, in the treatment of arabinoxylan containing raw materials like grain-based materials, herein grain-based materials includes grains and cereals, in the wheat gluten-starch separation industry, in the production of starch derived syrups, in biofuel production, and the like.

The term "variant xylanase(s)" as used herein may be used interchangeably with "modified xylanase(s)".

### DETAILED DISCLOSURE OF THE PREFERRED EMBODIMENTS OF THE INVENTION

Unless defined otherwise, all technical and scientific terms used herein have the same meaning as commonly understood by one of ordinary skill in the art to which this disclosure belongs. Singleton, et al., DICTIONARY OF MICROBIOLOGY AND MOLECULAR BIOLOGY, 20 ED., John Wiley and Sons, New York (1994), and Hale & Marham, THE HARPER COLLINS DICTIONARY OF BIOLOGY, Harper Perennial, NY (1991) provide one of skill with a general dictionary of many of the terms used in this disclosure.

This disclosure is not limited by the exemplary methods and materials disclosed herein, and any methods and materials similar or equivalent to those described herein can be used in the practice or testing of embodiments of this disclosure. Numeric ranges are inclusive of the numbers defining the range. Unless otherwise indicated, any nucleic acid sequences are written left to right in 5' to 3' orientation; amino acid sequences are written left to right in amino to carboxy orientation, respectively.

The headings provided herein are not limitations of the various aspects or embodiments of this disclosure which can be had by reference to the specification as a whole. Accordingly, the terms defined immediately below are more fully defined by reference to the specification as a whole.

Amino acids are referred to herein using the name of the amino acid, the three letter abbreviation or the single letter abbreviation.

The term "protein", as used herein, includes proteins, polypeptides, and peptides.

As used herein, the term "amino acid sequence" is synonymous with the term "polypeptide" and/or the term "protein". In some instances, the term "amino acid sequence" is synonymous with the term "peptide". In some instances, the term "amino acid sequence" is synonymous with the term "enzyme".

The terms "protein" and "polypeptide" are used interchangeably herein. In the present disclosure and claims, the conventional one-letter and three-letter codes for amino acid residues may be used. The 3-letter code for amino acids as defined in conformity with the IUPACIUB Joint Commission on Biochemical Nomenclature (JCBN). It is also understood that a polypeptide may be coded for by more than one nucleotide sequence due to the degeneracy of the genetic code.

As used herein, "starch" refers to any material comprised of the complex polysaccharide carbohydrates of plants, comprised of amylose and amylopectin with the formula (C6H10O5)x, wherein "X" can be any number. In particular, the term refers to any plant-based material including but not limited to grains, grasses, tubers and roots and more specifically wheat, barley, corn, rye, rice, sorghum, brans, cassava, millet, potato, sweet potato, and tapioca. "Granular starch" refers to uncooked (raw) starch, which has not been subject to gelatinization, where "starch gelatinization" means solubilization of a starch molecule to form a viscous suspension.

As used herein, "hydrolysis of starch" and the like refers to the cleavage of glucosidic bonds with the addition of water molecules. Thus, enzymes having "starch hydrolysis activity" catalyze the cleavage of glucosidic bonds with the addition of water molecules
As used herein, "fermentable sugars" refer to saccharides that are capable of being metabolized under fermentation conditions. These sugars typically refer to glucose, maltose, and maltotriose (DP1, DP2 and DP3).

Other definitions of terms may appear throughout the specification. Before the exemplary embodiments are described in more detail, it is to understand that this disclosure is not limited to particular embodiments described, as such may, of course, vary. It is also to be understood that the terminology used herein is for the purpose of describing particular embodiments only, and is not intended to be limiting, since the scope of the present disclosure will be limited only by the appended claims.

Where a range of values is provided, it is understood that each intervening value, to the tenth of the unit of the lower limit unless the context clearly dictates otherwise, between the upper and lower limits of that range is also specifically disclosed. Each smaller range between any stated value or intervening value in a stated range and any other stated or intervening value in that stated range is encompassed within this disclosure. The upper and lower limits of these smaller ranges may independently be included or excluded in the range, and each range where either, neither or both limits are included in the smaller ranges is also encompassed within this disclosure, subject to any specifically excluded limit in the stated range. Where the stated range includes one or both of the limits, ranges excluding either or both of those included limits are also included in this disclosure.

It must be noted that as used herein and in the appended claims, the singular forms "a", "an", and "the" include plural referents unless the context clearly dictates otherwise. Thus, for example, reference to "an enzyme" includes a plurality of such candidate agents and reference to "the feed" includes reference to one or more feeds and equivalents thereof known to those skilled in the art, and so forth.

Increasing prices of raw material traditionally used as energy source in animal feed, as a feedstock in biofuel production, as an ingredient in brewing or malting, or as a feedstock in wheat gluten-starch separation processes for instance have resulted in inclusion of low-cost fibrous materials in the starting substrates for these industries, particularly the use of low-cost fibrous by-products in animal feed.

Fibre addition may cause several disadvantageous effects. For example in animal feed fibre addition may cause anti-nutritional effects. The presence of un-degraded polymers present in the animal's intestine causes a highly viscous content and impeded diffusion with reduced nutrient absorption as a result. Also, the polymers possess a high water holding capacity hindering an effective re-absorption of water, and the water retention increases the volume of the gut content, which leads to a decrease intestinal transit time (Englyst & Kingman (1993) in Human Nutrition and Dietetics, 9th edition (Garrow J. S., James W. P. T., eds.) p. 53).

In grain-based materials and feedstuffs, hemicellulose and cellulose (including insoluble arabinoxylan) can form physical barriers encapsulating (or entrapping) nutrients like starch, oil and protein and thereby restrict access to these components.

Hemicellulose and cellulose (including insoluble arabinoxylans (AXinsol)) by themselves are also potential energy sources, as they consist of C5- and C6-saccharides. Mono C6-saccharides can be used as energy source by the animal, while oligo C5-saccharides can be transformed into short chain fatty acids by the micro flora present in the animal gut (van den Broek et al., 2008 Molecular Nutrition & Food Research, 52, 146-63), which short chain fatty acids can be taken up and digested by the animal's gut.

Release of nutrients and water from feedstuffs as a consequence of physical barrier degradation is dependent on the ability of the xylanase to degrade insoluble fibre components (e.g. insoluble arabanoxylans (AXinsol)).

The present invention provides a xylanase enzyme having at least 70% amino acid sequence identity to the amino acid sequence of SEQ ID NO: 21, the xylanase enzyme comprising amino acids 7D, 33V, 79Y, 217Q and 298Y at positions corresponding to the amino acid numbering of SEQ ID NO: 21 and wherein the xylanase enzyme has xylanase activity and is capable of increased oil recovery from grain-based material as compared to the xylanase enzyme of SEQ ID NO: 1.

In one embodiment the enzyme having xylanase activity, e.g. the GH10 xylanase enzyme (such as the parent or modified GH10 xylanase enzyme) or a fragment thereof, according to the present invention comprises the following amino acids at the positions indicated:
7D;
33V;
79Y;
217Q; and
298Y.

The term "parent" means a xylanase, preferably a GH10 xylanase, to which an alteration is made to produce a modified enzyme of the present invention. In one embodiment the parent enzyme is a GH10 xylanase. Suitably the parent enzyme may be a naturally occurring (wild-type) polypeptide or a variant or fragment thereof. In a preferred embodiment the parent enzyme is a naturally occurring (wild-type polypeptide).

The modified GH10 xylanase or GH10 xylanase according to the present invention (and claimed herein) suitably has about at least 90% sequence identity (preferably at least 93%, suitably at least 97%, suitably at least 99% sequence identity to the parent enzyme.

The term "backbone" as used herein means a polypeptide sequence that is a GH10 xylanase polypeptide, which is modified to comprise the following amino acids at at least one or more of the positions indicated: 7D; 25P; 33V; 64T; 79Y;; 89G; 217Q; and 298Y wherein the numbering is based on the amino acid numbering of FveXyn4 (SEQ ID No. 1).

The enzyme having xylanase activity, e.g. the GH10 xylanase enzyme, of the present invention (e.g. the modified GH10 xylanase enzyme) preferably comprises a polypeptide having at least 70% (suitably at least 80%, suitably at least 90%, suitably at least 95%, suitably at least 98%, suitably at least 99%) identity to a GH10 xylanase (e.g. a parent or backbone GH1 0 xylanase); and comprises the following amino acids at two or more (preferably at three or more, more preferably at all) of the positions indicated: 7D; 33V; 79Y, V, F, I, L or M (preferably 79Y, F or V, more preferably Y); 217Q, E, P, D or M (preferably 217Q, E or P, more preferably Q); and 298Y, F or W (preferably Y or F, more preferably Y) wherein the numbering is based on the amino acid numbering of FveXyn4 (SEQ ID No. 1).

The enzyme having xylanase activity, e.g. the GH10 xylanase enzyme, of the present invention (e.g. the modified GH10 xylanase enzyme) preferably comprises a polypeptide having at least at least 95% (suitably at least 98%, suitably at least 99%) identity to a GH10 xylanase (e.g. a parent or backbone GH10 xylanase); and comprises the following amino acids at at least one or more of the positions indicated: 7D; 25P; 33V; 64T; 79Y; 89G; 217Q; and 298Y wherein the numbering is based on the amino acid numbering of FveXyn4 (SEQ ID No. 1).

Suitably, the parent or backbone GH10 xylanase may be obtainable (suitably obtained) from a *Fusarium* organism.

Suitably the parent or backbone xylanase is an endo-1,4-β-d-xylanase.

The modified xylanase or GH10 xylanase according to the present invention is preferably an endo-1,4-β-d-xylanase.
In a preferred embodiment, the enzyme having xylanase activity, e.g. the GH10 xylanase enzyme (such as the parent or modified GH10 xylanase enzyme) or a fragment thereof according to the present invention has a Tm value of more than 70°C (preferably more than 75°C), wherein the Tm value is measured as the temperature at which 50% residual activity is obtained after 10 min incubation.

The thermostability of a xylanase (e.g. a modified xylanase) in accordance with the present invention may be determined using the "Assay for measurement of thermostability" (see below).

### Assay for measurement of thermostability

The thermal denaturation profiles of the FveXyn4 variants was measured by diluting and pre-incubating the enzyme samples in 25 mM MES buffer, pH 6.0 for 10 min at varying temperatures (63, 65.5, 66.7, 68.2, 70.6, 73.5, 76, 76.5, 76.8, 79.7, 81.9, 83.5, 84.6, and 85 °C, respectively) and subsequently measuring the residual activity by the xylanase activity method described in Example 1. Activity measured without pre-incubation was set to 100 % and the residual activity of each variant at each temperature was calculated as relative to this. Tm value is calculated from the thermal denaturation profiles as the temperature at which 50 % residual activity is obtained.

In one embodiment, an enzyme is considered to be thermostable in accordance with the present invention if it has a Tm value of more than 70°C, wherein the Tm value is the temperature at which 50% residual activity is obtained after 10 min incubation. This Tm value may be measured in accordance with the assay for measurement of thermostability as taught herein.

In one embodiment, an enzyme is considered to be thermostable in accordance with the present invention if it has a Tm value of more than 76°C, wherein the Tm value is the temperature at which 50% residual activity is obtained after 10 min incubation. This Tm value may be measured in accordance with the assay for measurement of thermostability as taught herein.

In one embodiment, an enzyme is considered to be thermostable in accordance with the present invention if it has a Tm value of more than 85°C, wherein the Tm value is the temperature at which 50% residual activity is obtained after 10 min incubation. This Tm value may be measured in accordance with the assay for measurement of thermostability as taught herein.

Preferably, the enzyme having xylanase activity, e.g. the GH10 xylanase enzyme (such as the parent or modified GH10 xylanase enzyme) or a fragment thereof according to the present invention (or composition comprising same) can withstand a heat treatment (e.g. during the pelleting process for example) of up to about 70°C; e.g. up to 75°C, e.g. up to 76°C, e.g. up to about 85°C; e.g. or up to about 95°C. The heat treatment may be performed for up to about 1 minute; up to about 5 minutes; up to about 10 minutes; up to about 30 minutes; up to about 60 minutes. To withstand such heat treatment means that at least about 50% of the enzyme that was present/active in the additive before heating to the specified temperature is still present/active after it cools to room temperature. Preferably, at least about 80% of the enzyme that is present and active in the additive before heating to the specified temperature is still present and active after it cools to room temperature.

The term "thermostability" is the ability of an enzyme to resist irreversible inactivation (usually by denaturation) at a relatively high temperature.

There are many ways of measuring thermostabiliy. By way of example, enzyme samples maybe incubated without substrate for a defined period of time (e.g. 10 min or 1 to 30 min) at an elevated temperature compared to the temperature at which the enzyme is stable for a longer time (days). Following the incubation at elevated temperature the enzyme sample is assayed for residual activity at the permissive temperature of e.g. 30°C (alternatively 25-50°C or even up to 70°C). Residual activity is calculated as relative to a sample of the enzyme that has not been incubated at the elevated temperature.

Thermostability can also be measured as enzyme inactivation as function of temperature. Here enzyme samples are incubated without substrate for a defined period of time (e.g. 10 min or 1 to 30 min) at various temperatures and following incubation assayed for residual activity at the permissive temperature of e.g. 30°C (alternatively 25-70°C or even higher). Residual activity at each temperature is calculated as relative to a sample of the enzyme that has not been incubated at the elevated temperature. The resulting thermal denaturation profile (temperature versus residual activity) can be used to calculate the temperature at which 50% residual activity is obtained. This value is defined as the Tm value.

Even further, thermostability can be measured as enzyme inactivation as function of time. Here enzyme samples are incubated without substrate at a defined elevated temperature (e.g. 76°C) for various time periods (e.g. between 10 sec and 30 min) and following incubation assayed for residual activity at the permissive temperature of e.g. 30°C (alternatively 25-70°C or even higher). Residual activity at each temperature is calculated as relative to an enzyme sample that has not been incubated at the elevated temperature. The resulting inactivation profile (time versus residual activity) can be used to calculate the time at which 50 % residual activity is obtained. This is usually given as T1/2.

These are examples of how to measure thermostability. Thermostability can also be measured by other methods. Preferably thermostability is assessed by use of the "Assay for measurement of thermostability" as taught herein.

In contradistinction to thermostability, thermoactivity is enzyme activity as a function of temperature. To determine thermoactivity enzyme samples may be incubated (assayed) for the period of time defined by the assay at various temperatures in the presence of substrate. Enzyme activity is obtained during or immediately after incubation as defined by the assay (e.g. reading an OD-value which reflects the amount of formed reaction product). The temperature at which the highest activity is obtained is the temperature optimum of the enzyme at the given assay conditions. The activity obtained at each temperature can be calculated relative to the activity obtained at optimum temperature. This will provide a temperature profile for the enzyme at the given assay conditions.

In the present application thermostability is not the same as thermoactivity.

Suitably the modified xylanase according to the present invention has a pH optimum in the range of 4.6 to 7, preferably about 5 to 6.

In a preferred embodiment, the modified xylanase according to the present invention comprises one of the amino acid sequences shown herein as SEQ ID No. 17, SEQ ID No. 18, SEQ ID No. 19, SEQ ID No. 20, or SEQ ID No. 21, or a fragment thereof having xylanase activity.

In one embodiment the modifications in the backbone polynucleotide sequence are such to render the above detailed modifications in the encoded amino acid sequence:
The methods of the present invention are suitable to render the modifications as taught above in the polynucleotide or amino acid sequence.

The host cell of the present invention may be selected from the group consisting of a bacterial cell, fungal cell, a yeast cell, a filamentous fungal cell and a plant cell. Preferably the host cell is a bacterial or fungal cell.

In some embodiments the enzyme having xylanase activity, e.g. the GH10 xylanase enzyme (such as the parent or modified GH10 xylanase enzyme) or a fragment thereof according to the present invention may be used directly as a fermentate without isolation and/or purification of the enzyme.

In some embodiments the feed additive composition according to the present invention or the premix according to the present invention further comprises one or more of the enzymes selected from the group consisting of a protease (e.g. subtilisin (E.C. 3.4.21.62) or a bacillolysin (E.C. 3.4.24.28) or an alkaline serine protease (E.C. 3.4.21.x) or a keratinase (E.C. 3.4.x.x) or an acid stable protease) and/or an amylase (including α-amylases (E.C. 3.2.1.1), G4-forming amylases (E.C. 3.2.1.60), β-amylases (E.C. 3.2.1.2) and γ-amylases (E.C. 3.2.1.3)).

The enzyme having xylanase activity, e.g. the GH10 xylanase enzyme (such as the parent or modified GH10 xylanase enzyme) or a fragment thereof according to the present invention may be used in a method for degrading arabinoxylan-containing material in a xylan-containing material.

Suitably, the arabinoxylan may be insoluble arabinoxylan (AXinsol).

In one embodiment the xylan-containing material is selected from one or more of the group consisting of: a feed or feedstuff; a feed component; a grain-based material; a mash; a wort; a malt; malted barley; an adjunct, a barley mash; and a cereal flour.

In a preferred embodiment the arabinoxylans are solubilized without increasing viscosity in the reaction medium.

In one embodiment of the present invention the feed or feedstuff or feed component comprises or consists of corn, DDGS (such as cDDGS), wheat, wheat bran or a combination thereof.

In one preferred embodiment the feed or feedstuff is a corn-based feedstuff.

The enzyme having xylanase activity, e.g. the GH10 xylanase enzyme (such as the parent or modified GH10 xylanase enzyme) or a fragment thereof according to the present invention according to the present invention may be used in combination with one or more of the enzymes selected from the group consisting of a protease (e.g. subtilisin (E.C. 3.4.21.62) or a bacillolysin (E.C. 3.4.24.28) or an alkaline serine protease (E.C. 3.4.21.x) or a keratinase (E.C. 3.4.x.x) or an acid stable protease) and/or an amylase (including α-amylases (E.C. 3.2.1.1), G4-forming amylases (E.C. 3.2.1.60), β-amylases (E.C. 3.2.1.2) and γ-amylases (E.C. 3.2.1.3)).

In one embodiment the method or use according to the present invention comprises administering a subject with an enzyme having xylanase activity, e.g. the GH10 xylanase enzyme (such as the parent or modified GH10 xylanase enzyme) or a fragment thereof according to the present invention, or a fermentate comprising said enzyme according to the present invention, or an enzyme composition comprising said xylanase enzyme according to the present invention, or a feed additive composition comprising said xylanase enzyme according to the present invention, or a premix comprising said xylanase enzyme according to the present invention or a feedstuff comprising said xylanase enzyme according to the present invention.

In one embodiment the method or use of the present invention is (or is part of) a wheat gluten-starch separation process.

In another embodiment, the method or use of the present invention is (or is part of) a biofuel (e.g. bioethanol) or biochemical (e.g. bio-based isoprene) production process.

In another embodiment, the method or use of the present invention is (or is part of) a malting or brewing process.

Suitably, a fermented beverage, e.g. beer, produced by a method according to the present invention in envisaged by the present invention.

In one embodiment the parent xylanase enzyme of the present invention may be referred to herein as FveXyn4.

Both the polypeptide sequences and the nucleic acid sequences taught herein are preferably isolated.

The xylanase of the present invention is preferably a GH10 xylanase. In other words the xylanase may have a molecular weight in the range of 32-39 kDa and/or the catalytic domain of the xylanase consists of an eightfold β/α barrel structure (as taught in Harris et al 1996 - Acta. Crystallog. Sec. D 52, 393-401).

In one aspect of the invention, the xylanase of the invention is a xylanase of Glycoside Hydrolyase (GH) Family 10. The term "of Glycoside Hydrolyase (GH) Family 10" means that the xylanase in question is or can be classified in the GH family 10.

Protein similarity searches (e.g. protein blast at http://blast.ncbi.nlm.nih.gov/Blast.cgi?CMD=Web&PAGE_TYPE=BlastHome) may determine whether an unknown sequence falls under the term of a GH10 xylanase family member, particularly the GH families may be categorised based on sequence homology in key regions. In addition or alternatively, to determine whether an unknown protein sequence is a xylanase protein within the GH10 family, the evaluation can be done, not only on sequence similarity/homology/identity, but also on 3D structure similarity. The classification of GH-families is often based on the 3D fold. Software that will predict the 3D fold of an unknown protein sequence is HHpred (http://toolkit.tuebingen.mpg.de/hhpred). The power of this software for protein structure prediction relies on identifying homologous sequences with known structure to be used as template. This works so well because structures diverge much more slowly than primary sequences. Proteins of the same family may have very similar structures even when their sequences have diverged beyond recognition.

In practice, an unknown sequence can be pasted into the software (http://toolkit.tuebingen.mpg.de/hhpred) in FASTA format. Having done this, the search can be submitted. The output of the search will show a list of sequences with known 3D structures. To confirm that the unknown sequence indeed is a GH10 xylanase, GH10 xylanases may be found within the list of homologues having a probability of > 90. Not all proteins identified as homologues will be characterised as GH10 xylanases, but some will. The latter proteins are proteins with a known structure and biochemically characterisation identifying them as xylanases. The former have not been biochemically characterised as GH10 xylanases. Several references describes this protocol such as Söding J. (2005) Protein homology detection by HMM-HMM comparison - Bioinformatics 21, 951-960 (doi:10.1093/bioinformatics/bti125) and Söding J, Biegert A, and Lupas AN. (2005) The HHpred interactive server for protein homology detection and structure prediction - Nucleic Acids Research 33, W244--W248 (Web Server issue) (doi:10.1093/nar/gki40).

According to the Cazy site (http://www.cazy.org/), Family 10 glycoside hydrolases can be characterised as follows:
Known Activities: endo-1,4-β-xylanase (EC 3.2.1.8); endo-1,3-β-xylanase (EC 3.2.1.32); tomatinase (EC 3.2.1.-)
Mechanism: Retaining
Clan: GH-A
Catalytic Nucleophile/Base: Glu (experimental)
Catalytic Proton Donor: Glu (experimental)
3D Structure Status: (β/α)₈

The GH10 xylanase of the present invention may have a catalytic domain with molecular weights in the range of 32-39kDa. The structure of the catalytic domain of the GH10 xylanase of the present invention consists of an eightfold β/α barrel (Harris et al 1996 - Acta. Crystallog. Sec. D 52, 393-401).

Three-dimensional structures are available for a large number of Family GH10 enzymes, the first solved being those of the *Streptomyces lividans* xylanase A (Derewenda et al J Biol Chem 1994 Aug 19; 269(33) 20811-4), the *C. fimi* endo-glycanase Cex (White et al Biochemistry 1994 Oct 25; 33(42) 12546-52), and the *Cellvibrio japonicus* Xyn10A (previously *Pseudomonas fluorescens* subsp. xylanase A) (Harris et al Structure 1994 Nov 15; 2(11) 1107-16.). As members of Clan GHA they have a classical (α/β)₈ TIM barrel fold with the two key active site glutamic acids located at the C-terminal ends of beta-strands 4 (acid/base) and 7 (nucleophile) (Henrissat et al Proc Natl Acad Sci U S A 1995 Jul 18; 92(15) 7090-4).

The term "GH10 xylanase" as used herein means a polypeptide having xylanase activity and having a (α/β)₈ TIM barrel fold with the two key active site glutamic acids located at the C-terminal ends of beta-strands 4 (acid/base) and 7 (nucleophile).

The backbone (or parent) xylanase enzyme used herein may be referred to as FveXyn4 or FoxXyn 2 (these terms refer to the active proteins, e.g. the mature proteins).

In one embodiment preferably the xylanase is a fungal xylanase.

The enzyme having xylanase activity, e.g. the GH10 xylanase enzyme (such as the parent or modified GH10 xylanase enzyme) or a fragment thereof according to the present invention and/or parent enzyme is a GH10 xylanase.

In one embodiment preferably the enzyme having xylanase activity, e.g. the GH10 xylanase enzyme (such as the parent or modified GH10 xylanase enzyme) or a fragment thereof according to the present invention (and/or parent xylanase) is a fungal GH10 xylanase.

In one embodiment preferably the enzyme having xylanase activity, e.g. the GH10 xylanase enzyme (such as the parent or modified GH10 xylanase enzyme) or a fragment thereof according to the present invention (and/or parent xylanase) is an endoxylanase, e.g. an endo-1,4-β-d-xylanase. The classification for an endo-1,4-β-d-xylanase is E.C. 3.2.1.8.

In some embodiments the enzyme having xylanase activity, e.g. the GH10 xylanase enzyme (such as the parent or modified GH10 xylanase enzyme) or a fragment thereof of the present invention has an optimum pH at about 6.

Preferably the enzyme having xylanase activity, e.g. the GH10 xylanase enzyme (such as the parent or modified GH1 0 xylanase enzyme) or a fragment thereof according to the present invention retains greater than 70% of maximum activity between pH4 and 8, suitably between pH 4.6 and 7.

In some embodiments, e.g. in feed applications, the enzyme having xylanase activity, e.g. the GH10 xylanase enzyme (such as the parent or modified GH10 xylanase enzyme) or a fragment thereof according to the present invention preferably retains greater than 70% of maximum activity between 5.1 and 7.

Without wishing to be bound by theory, pH may also have an important effect on enzyme efficacy and efficiency. For feed applications in particular the pH profile of the xylanases of the present invention favor activity in the small intestine, under neutral conditions.

In one embodiment, the enzyme having xylanase activity, e.g. the GH10 xylanase enzyme (such as the parent or modified GH10 xylanase enzyme) or a fragment thereof according to the present invention is capable of degrading (or degrades) a xylan-containing material, particularly arabinoxylans, particularly insoluble arabinoxylans (AXinsol).

In another embodiment the enzyme having xylanase activity, e.g. the GH10 xylanase enzyme (such as the parent or modified GH10 xylanase enzyme) or a fragment thereof according to the present invention is capable of degrading (or degrades) soluble polymers (e.g. oligomers) that are produced from degradation of AXinsol or that are (naturally) present in grain-based material.

In a further embodiment the enzyme having xylanase activity, e.g. the GH10 xylanase enzyme (such as the parent or modified GH10 xylanase enzyme) or a fragment thereof according to the present invention is capable of degrading (or degrades) both a xylan-containing material, particularly arabinoxylans, particularly AXinsol, and soluble polymers (e.g. oligomers) that are produced from degradation of AXinsol.

In one embodiment the enzyme having xylanase activity, e.g. the GH10 xylanase enzyme (such as the parent or modified GH10 xylanase enzyme) or a fragment thereof according to the present invention are unaffected by wheat xylanases inhibitors, e.g. proteinaceous inhibitors, e.g. TAXI-like proteinaceous inhibitors in wheat. Prior art fungal xylanases can be inhibited by as much as 70-95% by wheat proteinaceous inhibitors. Preferably the xylanases of the present invention are only inhibited by 20-30% at most in wheat applications.

TAXI are *Triticum aestivum* xylanases inhibitors, present in cereals.

The term "consisting essentially of" as used herein means that unspecified components may be present if the characteristics of the claimed composition are thereby not materially affected.

The term "consisting of" means that the proportions of the specific ingredients must total 100%.

The term "comprising" used herein may be amended in some embodiments to refer to consisting essentially of or consisting of (both having a more limited meaning than "comprising").

In one embodiment the insoluble arabinoxylan containing material is not wheat straw.

The term "fragment thereof" as used herein means an active fragment. In other words the fragment is one which has xylanase activity. Suitably the fragment may have the same xylanase activity as the full length modified GH10 xylanase enzyme from which the fragment is derived. Alternatively, the fragment may have a modified activity (e.g. enhanced specificity, specific activity, pH or temperature profile) compared with the full length modified GH10 xylanase enzyme from which the fragment is derived. In addition the fragment must retain the thermostable properties of the modified GH10 xylanase enzyme of which it is a fragment.

Also described is an enzyme having xylanase activity, e.g. the GH10 xylanase enzyme (such as the parent or modified GH10 xylanase enzyme) or a fragment, thereof that
a) comprises one of the amino acid sequences shown herein as SEQ ID No. 17, SEQ ID No. 18, SEQ ID No. 19, SEQ ID No. 20, or SEQ ID No. 21, or b) comprises an amino acid sequence which is at least 96%, preferably at least 98.5%, identical with the amino acid sequences shown herein as SEQ ID No. 17, SEQ ID No. 18, SEQ ID No. 19, SEQ ID No. 20, or SEQ ID No. 21 so long as the amino acids at positions 7, 33, 79, 217 and 298 are identical with those shown in SEQ ID No. 17, SEQ ID No. 18, SEQ ID No. 19, SEQ ID No. 20, or SEQ ID No. 21.

Also described is a nucleic acid molecule or a vector or construct comprising same, wherein the nucleotide sequence is selected from the group consisting of: SEQ ID No. 7, SEQ ID No. 8, SEQ ID No. 9, SEQ ID No. 10, SEQ ID No. 11, SEQ ID No. 12. SEQ ID No. 13, SEQ ID No.14, SEQ ID No. 15 and SEQ ID No. 16; or a nucleotide sequence which is at least 96%, preferably 98.5%, identical with the nucleotide sequences shown herein as SEQ ID No. 7, SEQ ID No. 8, SEQ ID No. 9, SEQ ID No. 10, SEQ ID No. 11, SEQ ID No. 12. SEQ ID No. 13, SEQ ID No.14, SEQ ID No. 15 or SEQ ID No. 16 so long as the codons encoding amino acid positions 7, 33, 79, 217 and 298 in the mature protein the same as those of SEQ ID No. 7, SEQ ID No. 8, SEQ ID No. 9, SEQ ID No. 10, SEQ ID No. 11, SEQ ID No. 12. SEQ ID No. 13, SEQ ID No.14, SEQ ID No. 15 or SEQ ID No. 16.

The term "modifying" as used herein means changing or altering. In particular, the term "modifying" as used herein means altering from the naturally occurring. In other words, when modifying the enzyme, one changes the enzyme in such a way that renders the enzyme altered from the parent backbone enzyme. Preferably the modified enzyme does not exist itself in nature. Thus the modified enzyme is a non-naturally-occurring enzyme.

The term "modified" as used herein means altered, e.g. from its naturally occurring form. The modified enzymes according to the present invention are preferably not naturally occurring enzymes or naturally occurring variants. In other words, the modified enzymes according to the present invention are preferably modified enzymes that have not been found in nature. The modified enzymes of the present invention have preferably not occurred spontaneously.

The enzyme having xylanase activity, e.g. the GH10 xylanase enzyme (incuding a parent or modified GH10 xylanase enzyme) or a fragment thereof of the present invention can be suitably used in any one of the following applications:
a) An additive in animal feedstuffs; and/or
b) A feed supplement for an animal; and/or
c) Breakdown of grain-based material (e.g. this can be whole grain or part of grain). The breakdown products (e.g. glucose) can be used as a feedstock for any fermentation process, such as in biofuel (e.g. bioethanol) production or in the production of other products such as biochemicals (e.g., bio-based isoprene). In such processes the modified xylanase can improve by-product recovery, e.g., oil recovery. Therefore in one embodiment the present invention relates to the production of biofuel (e.g. bioethanol), e.g., by improved oil recovery and to the enhanced utilisation of grain-based material in the biofuel industry; and/or
d) Cereal (e.g. wheat) gluten-starch separation industry. The resultant product(s) may be starch (e.g. purified starch) and/or gluten and/or fibres and/or water solubles (such as soluble pentosans). Improved by-product recovery, e.g., oil recovery is also envisioned in conjuction with such a process. In one embodiment the present invention relates to the production of starch and/or gluten; and/or
e) Improving malting and brewing, e.g. by breaking down grain-based material (e.g. malted barley). Improved by-product recovery, e.g., oil recovery is also envisioned in conjunction with such a process; and/or
f) to degrade AXsol or the breakdown products of AXinsol to ensure viscosity is not increased and/or viscosity is reduced in the reaction mixture;
g) to reducing viscosity when degrading grain-based materials, e.g. in biofuel (e.g. bioethanol) production processes.

In one embodiment the enzyme having xylanase activity, e.g. the GH10 xylanase enzyme (such as the parent or modified GH10 xylanase enzyme) or a fragment thereof of the present invention is used in a feedstuff. Preferably a feedstuff comprising corn or is a corn-based feedstuff.

In one embodiment the enzyme having xylanase activity, e.g. the GH10 xylanase enzyme (such as the modified GH10 xylanase enzyme) or a fragment thereof of the present invention is used in malting or brewing.

In a further embodiment the enzyme having xylanase activity, e.g. the GH10 xylanase enzyme (such as the parent or modified GH10 xylanase enzyme) or a fragment thereof of the present invention is used in wheat gluten-starch separation.

In a yet further embodiment the enzyme having xylanase activity, e.g. the GH10 xylanase enzyme (such as the parent or modified GH10 xylanase enzyme) or a fragment thereof of the present invention is used in the breakdown of grain-based material and may be part of the biofuel (e.g. bioethanol) production process.

### ADVANTAGES

The novel enzyme having xylanase activity, e.g. the GH10 xylanase enzyme (including the parent or the modified GH10 xylanase enzyme) or a fragment thereof taught herein has many advantages compared with known xylanases.

The enzyme having xylanase activity, e.g. the GH10 xylanase enzyme (such as the parent or modified GH10 xylanase enzyme) or a fragment thereof of the present invention is thermostable. For example the enzyme having xylanase activity, e.g. the GH10 xylanase enzyme (such as the parent or modified GH10 xylanase enzyme) or a fragment thereof of the present invention is significantly more stable than the parent (backbone) xylanase before modification. Suitably the modified xylanase has a Tm value of more than 70°C (preferably more than 75°C), wherein the Tm value is measured as the temperature at which 50 % residual activity is obtained after 10 min incubation.

The enzyme having xylanase activity, e.g. the GH10 xylanase enzyme (including the parent or the modified GH10 xylanase enzyme) or a fragment thereof of the present invention are also unexpectedly good at improved oil recovery.

The enzyme having xylanase activity, e.g. the GH10 xylanase enzyme (including the parent or the modified GH10 xylanase enzyme) or a fragment thereof of the present invention are also unexpectedly good at solubilising pentosans.

The enzyme having xylanase activity, e.g. the GH10 xylanase enzyme (including the parent or the modified GH10 xylanase enzyme) or a fragment thereof of the present invention are unexpectedly good at solubilising AXinsol.

Surprisingly it has been found that the enzyme having xylanase activity, e.g. the GH10 xylanase enzyme (including the parent or the modified GH10 xylanase enzyme) or a fragment thereof of the present invention is particularly good at degrading xylan-containing materials, such as arabinoxylans, e.g. AXinsol, in a broad spectrum of substrates, corn, wheat, DDGS, etc., in particular corn and corn based substrates, in particular both wheat (including wheat-based) products and corn (including corn-based products). Compared with the benchmark xylanases which are all commercially produced and marketed xylanases, the novel xylanase taught herein was capable of much more efficient degradation and pentosan release from more plant based materials (in particular corn-based substrates) compared with the marketed xylanases. This was completely unexpected. This contrasts with prior-known enzymes, which are often inferior at solubilising AXinsol in corn or corn-based substrates or which are not as efficient in both wheat- and corn-based substrates.

In addition, the enzyme having xylanase activity, e.g. the GH1 0 xylanase enzyme (including the parent orthe modified GH10 xylanase enzyme) or a fragment thereof, of the present invention is particularly good at not only breaking down (solubilising) AXinsol, but also breaking down (or degrading) the solubilized polymers efficiently. By being able to efficiently (quickly) breakdown (degrade) the solubilized polymers (obtained from dissolving AXinsol) a reduction in viscosity is obtained. This latter effect is essential in some of the claimed applications.

Typically, conventional xylanases may breakdown AXinsol, but will lead to an increase is the polymer production products which will lead to an increase in viscosity of the mixture. This increased viscosity is disadvantageous in many applications.

The enzyme having xylanase activity, e.g. the GH10 xylanase enzyme (including the parent or the modified GH10 xylanase enzyme) or a fragment thereof, of the present invention and as described herein have been found to not only breakdown (solubilise) insoluble arabinoxylans (AXinsol) from a wide range of substrates, including corn, wheat, DDGS, etc., in particular corn and corn-based substrates, in particular both wheat (including wheat-based) products and corn (including corn-based products), but also efficiently breakdown the thus solubilised polymers to ensure viscosity is not raised and/or to reduce viscosity.

The enzyme having xylanase activity, e.g. the GH10 xylanase enzyme (including the parent or the modified GH10 xylanase enzyme) or a fragment thereof, of the present invention and as described herein are capable of degrading AXsol or the breakdown products of AXinsol to ensure viscosity is not increased and/or viscosity is reduced in the reaction mixture.

Many of the xylanases commercialized for use in feedstuffs for solubilizing pentosans are GH11 enzymes. It had been considered by those skilled in the art that GH10 xylanases were not as strong at solubilizing pentosans, particularly AXinsol, compared with GH11 xylanases. Surprisingly it has been found that the novel modified xylanase(s) disclosed herein which is a/are GH10 xylanase(s) is/are particularly good at solubilizing AXinsol in a broad spectrum of substrates, including corn based substrates. Surprisingly, the present inventors have found that the modified GH10 xylanases of the present invention (and taught herein) outperform commercial GH11 xylanases in their ability to solubilize pentosans.

The fact that the present enzymes efficiently solubilize AXinsol from corn and corn-based substrates is significantly advantageous as corn holds much more AX in the insoluble form compared with other cereals, such as wheat and rye for example. Therefore only xylanases that can breakdown AXinsol can show significant benefit to animals fed on corn-soy diet for example.

It was completely unexpected for a GH10 xylanase to be so good on solubilizing AXinsol in cereals, particularly in corn or corn-based substrates.

The enzymes of the present invention are able to efficiently (and quickly) degrade the polymers and/or oligomers that are produced from solubilisation of AXinsol or that are present in grain-based materials. This leads to an unexpected advantage for the modified GH10 xylanases taught herein in that they are particularly good in a number of applications to keep viscosity low or to reduce viscosity, e.g. in feedstuffs; in brewing and/or malting; in grain-based production of glucose, e.g. for further processing to biofuels and/or biochemicals (e.g., bio-based isoprene); or in the wheat gluten-starch separation industry for the production of starch for example.

In addition, the modified GH10 xylanase of the present invention is particularly thermostable. This provides significant advantages in some applications. In particular, in feed applications, enzymes can be subject to heat treatment, e.g. during pelleting processes. Thus the enzymes need to be able to maintain their activity after such processing. The modified xylanases of the present invention are particularly and unexpectedly thermostable.

Furthermore, an improved thermostability is also very beneficial during degradation of starch, which takes place at elevated temperatures during liquefaction (around 85-95C). Being thermostable allows the addition of the enzyme during this step.

Notably it has been found that the degradation product from use of the modified xylanase on average is shorter for the GH10 enzymes tested herein compared with GH11 enzymes. This enhances the lowering of viscosity effect.

In addition, a further advantage of the enzyme having xylanase activity, e.g. the GH10 xylanase enzyme (including the parent or the modified GH10 xylanase enzyme) or a fragment thereof, of the present invention (unlike many GH11 xylanases) are unaffected by wheat xylanase inhibitors, e.g. TAXI like proteinaceous inhibitors, which occur in wheat.

One advantage of the present invention is that it improves wheat gluten-starch separation.

The enzyme of the present invention is particularly effective at enhancing the performance of a subject or improving the digestibility of a raw material in a feed and/or for improving feed efficiency in a subject.

### XYLAN-CONTAINING MATERIAL

The enzyme having xylanase activity, e.g. the GH10 xylanase enzyme (such as the parent or modified GH10 xylanase enzyme) or a fragment thereof, of the present invention (or composition comprising the modified xylanase of the present invention) may be used to degrade any xylan-containing material.

In one embodiment the xylan-containing material is any plant material comprising arabinoxylan.

In one embodiment the xylan-containing material is any plant material comprising insoluble arabinoxylan (AXinsol).

In one embodiment the xylan-containing material is a feedstuff or feed component.

In one embodiment the xylan-containing material is a grain-based material (including whole grains or partial grains or malted grains, e.g. malted barley). When the method relates to biofuel production (e.g. bioethanol production) then preferably the xylan-containing material is a grain-based material.

In another embodiment the xylan-containing material may be a barley malt or mash, or malted barley or combinations thereof.

In a yet further embodiment the xylan-containing material may be a cereal flour (e.g. wheat, oat, rye or barley flour). When the method relates to a gluten-starch separation process preferably the xylan-containing material is a cereal flour (e.g. wheat oat, rye or barley flour).

### BREAKDOWN OR DEGRADATION

The enzyme having xylanase activity, e.g. the GH10 xylanase enzyme (such as the parent or modified GH10 xylanase enzyme) or a fragment thereof, of the present invention (or composition comprising the enzyme) may be used to breakdown (degrade) AXinsol or AXsol or degradation products of AXinsol.

The term "breakdown" or "degrade" in synonymous with hydrolyses.

### SOLUBILISATION / DEGRADATION

The present invention relates to a method of degrading a xylan-containing material (preferably an arabinoxylan-containing material, preferably an insoluble arabinoxylan (AXinsol)-containing material) to produce soluble pentosans (which can be polymeric, oligomeric or monomeric).

This method may be described herein as pentosan solubilisation or arabinoxylan solubilisation or AXinsol solubilisation or degradation of AXinsol.

In one embodiment, the present invention relates to a method of degrading (or breaking down) insoluble arabinoxylan (AXinsol). This can also be referred to as solubilisation of insoluble arabinoxylan and/or solubilisation of pentosans.

In a further embodiment of the present invention the method relates to degrading (e.g. breaking down) polymers derived from the degradation of insoluble arabinoxylans.

### ARABINOXYLAN (AX)

The term "arabinoxylans" (AX) as used herein means a polysaccharide consisting of a xylan backbone (1,4-linked xylose units) with L-arabinofuranose (L-arabinose in its 5-atom ring form) attached randomly by 1α→2 and/or 1α→3 linkages to the xylose units throughout the chain. Arabinoxylan is a hemicellulose found in both the primary and secondary cell walls of plants. Arabinoxylan can be found in the bran of grains such as wheat, maize (corn), rye, and barley.

Arabinoxylan (AX) is found in close association with the plant cell wall, where it acts as a glue linking various building blocks of the plant cell wall and tissue, give it both structural strength and rigidity.

The term "pentosan" as used herein is any of a group of carbohydrates which yield pentoses on complete hydrolysis.

Since xylose and arabinose (the constituents of arabinoxylans) are both pentoses, arabinoxylans are usually classified as pentosans.

AX is the principal Non Starch Polysaccharide (NSP)-fraction in several of the most important feed raw material, including wheat and corn.

Its abundance, location within vegetable material and molecular structure cause AX to have a severe, negative impact on feed digestibility, effectively reducing the nutritional value of the raw materials in which it is present. This makes AX an important anti-nutritional factor, reducing animal production efficiency.

In addition AX can have a severe, negative impact when trying to breakdown plant material for example in processes such as brewing, malting, biofuel manufacture, effectively reducing the amount of substrate accessible in the raw plant material.

AXs can also hold substantial amounts of water (which can be referred to as their water holding capacity) - this can cause soluble arabinoxylans to result in (high) viscosity - which is a disadvantage in many applications.

The term "Hemicellulose" - as used herein means the polysaccharide components of plant cell walls other than cellulose. The term "hemicellulose" as used herein may mean polysaccharides in plant cell walls which are extractable by dilute alkaline solutions. Hemicelluloses comprise almost one-third of the carbohydrates in woody plant tissue. The chemical structure of hemicelluloses consists of long chains of a variety of pentoses, hexoses, and their corresponding uronic acids. Hemicelluloses may be found in fruit, plant stems, and grain hulls. Xylan is an example of a pentosan consisting of D-xylose units with 1β→4 linkages.

### WATER INSOLUBLE ARABINOXYLAN (AXinsol)

Water-insoluble arabinoxylan (AXinsol) also known as water-unextractable arabinoxylan (WU-AX) constitutes a significant proportion of the dry matter of plant material.

In wheat AXinsol can account for 6.3% of the dry matter. In wheat bran and wheat DDGS AXinsol can account for about 20.8% or 13.4% of the dry matter (w/w).

In rye AXinsol can account for 5.5% of the dry matter.

In corn AXinsol can account for 3.5-6% (e.g. 5.1%) of the dry matter. In corn DDGS AXinsol can account for 10-20% (e.g. 12.6%) of the dry matter.

AXinsol causes nutrient entrapment in feed. Large quantities of well digestible nutrients such as starch and proteins remain either enclosed in clusters of cell wall material or bound to side chains of the AX. These entrapped nutrients will not be available for digestion and subsequent absorption in the small intestine.

### WATER-SOLUBLE ARABINOXYLAN (AXsol)

Water-soluble arabinoxylan (AXsol) also known as water extractable arabinoxylan (WE-AX) can cause problems in biofuel production, biochemical production, carbohydrate processing and/or malting and/or brewing and/or in feed as they can cause increased viscosity due to the water-binding capacity of AXsol.

In feed AXsol can have an anti-nutritional effect particularly in monogastrics as they cause a considerable increase of the viscosity of the intestinal content, caused by the extraordinary water-binding capacity of AXsol. The increase viscosity can affect feed digestion and nutrient use as it can prevent proper mixing of feed with digestive enzymes and bile salts and/or it slows down nutrient availability and absorption and/or it stimulates fermentation in the hindgut.

In wheat AXsol can account for 1.8% of the dry matter. In wheat bran and wheat DDGS AXsol can account for about 1.1% or 4.9% of the dry matter (w/w).

In rye AXsol can account for 3.4% of the dry matter.

In barley AXsol can account for 0.4-0.8% of the dry matter.

In corn AXsol can account for 0.1-0.4% (e.g. 0.1%) of the dry matter. In corn DDGS AXinsol can account for 0.3-2.5% (e.g. 0.4%) of the dry matter.

In addition, however, to the amount of AXsol present in plant material, when a xylanase solubilises AXinsol in the plant material this can release pentosans and/or oligomers which contribute to AXsol content of the plant material.

One significant advantage of the modified xylanases disclosed herein is that they have the ability to solubilise AXinsol without increasing viscosity. It is presently believed that high molecular weight products are not formed
A breakdown of AXsol can decrease viscosity.

A breakdown of AXsol can release nutrients.

### VISCOSITY

The present invention can be used to ensure that the viscosity is not increased and/or to reduce viscosity in any process where the water-binding capacity of AXsol causes an undesirable increase in viscosity.

The present invention relates to ensuring that viscosity is not increased and/or to reducing viscosity by breaking down (degrading) AXsol or by breaking down (degrading) the polymers and/or oligomers produced by solubilising AXinsol.

Without wishing to be bound by theory, by being able to efficiently (quickly) breakdown (degrade) the solubilized polymers (e.g. oligomers) obtained from dissolving AXinsol an undesirable increase in viscosity can be avoided and/or a reduction in viscosity can be obtained. The term "efficiently" as used herein means that the enzyme is capable of degrading the polymers (e.g. oligomers) being formed by solubilisation of the AXinsol faster than the speed with which the AXinsol is degraded (or solubilized).

Reducing viscosity has advantages in many applications as taught herein.

An in vitro assay which attempts to mimic the environment in the small intestine of a chicken was originally described by Bedford & Classen (1993 Poultry Sci., 72, 137-143). The assay consists of a two step incubation of the feed first at low pH with pepsin followed by incubation with pancreatin at neutral pH. It is generally accepted that the viscosity of the supernatant after end incubation correlates with the viscosity created in vivo in broilers.

Without increasing viscosity and/or a reduction in viscosity as taught herein for feed applications means that addition of the xylanase will result in an unchanged or lower viscosity measured by the method described in Example 1. By unchanged it is meant that the measured value, being the average of three repetitions, falls within two standard deviation of the measured value for a wheat sample without xylanase addition.

Viscosity can be measured using the following devices: Rapid ViscoAnalyzer (RVA) (e.g. in bioethanol processing) and Haake VT550 viscometer (Thermofisher) (e.g. is wheat-gluten starch processing). Both devices can monitor viscosity profiles of fuel ethanol processes and wheat starch separation processes, of which the experimental conditions are taught in Example 6 and 7, respectively.

In the present invention a reduction in viscosity can be calculated by comparing one sample comprising the xylanase of the present invention (or taught herein) compared with another comparable sample without the xylanase of the present invention (or taught herein).

Comparing the viscosity reduction profiles of the xylanase of the present invention with those of the market benchmark xylanase(s) demonstrates the enzyme performance. The aim is to improve enzyme performance compared to the market benchmark. The benchmark enzyme(s) for the individual applications are provided in the examples below.

The benchmark enzyme for comparing viscosity reduction in feed applications may be Econase®XT.

An example of a xylanase used in the bioethanol industry is Xylathin™.

An example of a Xylanase used in the wheat gluten-starch separation Industry is Shearzyme™.

The benchmark enzyme for review of thermostability may be the parent (backbone) xylanase (e.g. before modification).

In one embodiment of the present invention the xylanases taught herein are viscosity reducers.

Generally, wheat (or other cereal) is first dry-milled to separate the bran and germ from the endosperm, which is ground into flour. This endosperm flour is then further fractionated through a wheat starch separation process into several product streams of varying commercial value. The major aim is to produce a refined grade of A-starch, consisting of large, lenticular granules of 15-40 µm. The second stream B-starch consists of less purified starch granules, which are spherical and small (1-10 µm). (C.C. Maningat, P.A. Seib, S.D. Bassi, K.S. Woo, G.D. Lasater, Chapter 10 from the book "Starch" (2009) 441-451, Wheat starch: production, properties, modification and uses). Isolated wheat starch forms the starting material for modified starch production with applications in both food- and nonfood-applications. Vital gluten is the third product of added-value in wheat separation processes. The vitality of the isolated wheat gluten is determined by the ability to form viscoelastic networks, required for breadmaking. Vital gluten encapsulate the carbon dioxide formed in dough preparation during baking, and consequently increase the bread volume. *(*Anne van der Borght, Hans Goesaert, Wim S. Veraverbeke, Jan A. Delcour, Journal of Cereal Science 41 (2005) 221-237, Fractionation of wheat and wheat flour into starch and gluten: overview of the main processes and the factors involved*.)* It is therefore often used to enrich flours for bread making, to achieve improved bread products. Other markets for gluten include as an additive in vegetarian, meat, fish or poultry products, including those in pet-food industry; in cereal breakfast; or in soy sauce. Due to its thermoplasticity and good film-forming properties, gluten is also used in non-food markets as adhesives. *(*L. Day, M.A. Augustin, I.L. Batey, C.W. Wrigley, Trends in Food Science & Technology 17 (2006) 82-90*, Wheat-gluten uses and industry needs.).*

The modified xylanases taught herein can be used to reduce the viscosity (or not increase viscosity) in processes for separating cereal flour (e.g. wheat, oat, rye or barley flour) into starch and gluten fractions and to improve the separation by degrading oligosaccharides that hinder gluten agglomeration.

Wort viscosity, and the viscosity of barley mash and barley malt in brewing and malting can cause significant disadvantages during brewing and/or malting. The present invention relates to reducing the viscosity (or not increase the viscosity) of wort, barley mash, barley malt or a combination thereof.

### FEED OR FEEDSTUFF

The enzyme having xylanase activity, e.g. the GH10 xylanase enzyme (such as the parent or modified GH10 xylanase enzyme) or a fragment thereof, of the present invention or feed additive composition of the present invention may be used as - or in the preparation of - a feed.

The term "feed" is used synonymously herein with "feedstuff".

Preferably the arabinoxylan-containing material of the present invention is a feedstuff, or a constituent of a feedstuff, or a feed component.

The feed may be in the form of a solution or as a solid or as a semi-solid - depending on the use and/or the mode of application and/or the mode of administration.

When used as - or in the preparation of - a feed - such as functional feed - the enzyme or composition of the present invention may be used in conjunction with one or more of: a nutritionally acceptable carrier, a nutritionally acceptable diluent, a nutritionally acceptable excipient, a nutritionally acceptable adjuvant, a nutritionally active ingredient.

In a preferred embodiment the enzyme or feed additive composition of the present invention is admixed with a feed component to form a feedstuff.

The term "feed component" as used herein means all or part of the feedstuff. Part of the feedstuff may mean one constituent of the feedstuff or more than one constituent of the feedstuff, e.g. 2 or 3 or 4. In one embodiment the term "feed component" encompasses a premix or premix constituents.

Preferably the feed may be a fodder, or a premix thereof, a compound feed, or a premix thereof. In one embodiment the feed additive composition according to the present invention may be admixed with a compound feed, a compound feed component or to a premix of a compound feed or to a fodder, a fodder component, or a premix of a fodder.

The term "fodder" as used herein means any food which is provided to an animal (rather than the animal having to forage for it themselves). Fodder encompasses plants that have been cut.

The term fodder includes silage, compressed and pelleted feeds, oils and mixed rations, and also sprouted grains and legumes.

Fodder may be obtained from one or more of the plants selected from: corn (maize), alfalfa (Lucerne),http://en.wildpedia.org/wiki/Barley barley, birdsfoot trefoil, brassicas, Chau moellier, kale, rapeseed (canola), rutabaga (swede), turnip, clover, alsike clover, red clover, subterranean clover, white clover, fescue, brome, millet, oats, sorghum, soybeans, trees (pollard tree shoots for tree-hay), wheat, and legumes.

The term "compound feed" means a commercial feed in the form of a meal, a pellet, nuts, cake or a crumble. Compound feeds may be blended from various raw materials and additives. These blends are formulated according to the specific requirements of the target animal.

Compound feeds can be complete feeds that provide all the daily required nutrients, concentrates that provide a part of the ration (protein, energy) or supplements that only provide additional micronutrients, such as minerals and vitamins.

The main ingredients used in compound feed are the feed grains, which include corn, wheat, canola meal, rapeseed meal, lupin, soybeans, sorghum, oats, and barley.

Suitably a premix as referred to herein may be a composition composed of microingredients such as vitamins, minerals, chemical preservatives, antibiotics, fermentation products, and other essential ingredients. Premixes are usually compositions suitable for blending into commercial rations.

Any feedstuff of the present invention may comprise one or more feed materials selected from the group comprising a) cereals, such as small grains (e.g., wheat, barley, rye, oats, triticale and combinations thereof) and/or large grains such as maize or sorghum; b) by products from cereals, such as corn gluten meal, wet-cake (particularly corn based wet-cake), Distillers Dried Grain (DDG) (particularly corn based Distillers Dried Grain (cDDG)), Distillers Dried Grain Solubles (DDGS) (particularly corn based Distillers Dried Grain Solubles (cDDGS)), wheat bran, wheat middlings, wheat shorts, rice bran, rice hulls, oat hulls, palm kernel, and citrus pulp; c) protein obtained from sources such as soya, sunflower, peanut, lupin, peas, fava beans, cotton, canola, fish meal, dried plasma protein, meat and bone meal, potato protein, whey, copra, sesame; d) oils and fats obtained from vegetable and animal sources; e) minerals and vitamins.

In one embodiment the feedstuff comprises or consists of corn, DDGS (such as cDDGS), wheat, wheat bran or a combination thereof.

In one embodiment the feed component may be corn, DDGS (e.g. cDDGS), wheat, wheat bran or a combination thereof.

In one embodiment the feedstuff comprises or consists of corn, DDGS (such as cDDGS) or a combination thereof.

In one embodiment a feed component may be corn, DDGS (such as cDDGS) or a combination thereof.

A feedstuff of the present invention may contain at least 30%, at least 40%, at least 50% or at least 60% by weight corn and soybean meal or corn and full fat soy, or wheat meal or sunflower meal.

A feedstuff of the present invention may contain between about 5 to about 40% corn DDGS. For poultry - the feedstuff on average may contain between about 7 to 15% corn DDGS. For swine (pigs) - the feedstuff may contain on average 5 to 40% corn DDGS.

A feedstuff of the present invention may contain corn as a single grain, in which case the feedstuff may comprise between about 35% to about 80% corn.

In feedstuffs comprising mixed grains, e.g. comprising corn and wheat for example, the feedstuff may comprise at least 10% corn.

In addition or in the alternative, a feedstuff of the present invention may comprise at least one high fibre feed material and/or at least one by-product of the at least one high fibre feed material to provide a high fibre feedstuff. Examples of high fibre feed materials include: wheat, barley, rye, oats, by products from cereals, such as corn gluten meal, corn gluten feed, wet-cake, Distillers Dried Grain (DDG), Distillers Dried Grain Solubles (DDGS), wheat bran, wheat middlings, wheat shorts, rice bran, rice hulls, oat hulls, palm kernel, and citrus pulp. Some protein sources may also be regarded as high fibre: protein obtained from sources such as sunflower, lupin, fava beans and cotton.

In one embodiment the feedstuff of the present invention comprises at least one high fibre material and/or at least one by-product of the at least one high fibre feed material selected from the group consisting of Distillers Dried Grain Solubles (DDGS) - particularly cDDGS, wet-cake, Distillers Dried Grain (DDG) - particularly cDDG, wheat bran, and wheat for example.

In one embodiment the feedstuff of the present invention comprises at least one high fibre material and/or at least one by-product of the at least one high fibre feed material selected from the group consisting of Distillers Dried Grain Solubles (DDGS) - particularly cDDGS, wheat bran, and wheat for example.

In the present invention the feed may be one or more of the following: a compound feed and premix, including pellets, nuts or (cattle) cake; a crop or crop residue: corn, soybeans, sorghum, oats, barley copra, straw, chaff, sugar beet waste; fish meal; meat and bone meal; molasses; oil cake and press cake; oligosaccharides; conserved forage plants: silage; seaweed; seeds and grains, either whole or prepared by crushing, milling etc.; sprouted grains and legumes; yeast extract.

The term "feed" in the present invention encompasses in some embodiments pet food. A pet food is plant or animal material intended for consumption by pets, such as dog food or cat food. Pet food, such as dog and cat food, may be either in a dry form, such as kibble for dogs, or wet canned form. Cat food may contain the amino acid taurine.

The term "feed" in the present invention encompasses in some embodiments fish food. A fish food normally contains macro nutrients, trace elements and vitamins necessary to keep captive fish in good health. Fish food may be in the form of a flake, pellet or tablet. Pelleted forms, some of which sink rapidly, are often used for larger fish or bottom feeding species. Some fish foods also contain additives, such as beta carotene or sex hormones, to artificially enhance the colour of ornamental fish.

The term "feed" in the present invention encompasses in some embodiment bird food. Bird food includes food that is used both in birdfeeders and to feed pet birds. Typically bird food comprises of a variety of seeds, but may also encompass suet (beef or mutton fat).

As used herein the term "contacted" refers to the indirect or direct application of the enzyme (or composition comprising the enzyme) of the present invention to the product (e.g. the feed). Examples of the application methods which may be used, include, but are not limited to, treating the product in a material comprising the feed additive composition, direct application by mixing the feed additive composition with the product, spraying the feed additive composition onto the product surface or dipping the product into a preparation of the feed additive composition.

In one embodiment the feed additive composition of the present invention is preferably admixed with the product (e.g. feedstuff). Alternatively, the feed additive composition may be included in the emulsion or raw ingredients of a feedstuff.

For some applications, it is important that the composition is made available on or to the surface of a product to be affected/treated. This allows the composition to impart one or more of the following favourable characteristics: performance benefits.

The modified enzyme (or composition comprising the modified enzyme) of the present invention may be applied to intersperse, coat and/or impregnate a product (e.g. feedstuff or raw ingredients of a feedstuff) with a controlled amount of said enzyme.

In a particularly preferred embodiment the enzyme (or composition comprising the enzyme) of the present invention is homogenized to produce a powder.

In an alternative preferred embodiment, the enzyme (or composition comprising the enzyme) of the present invention is formulated to granules as described in WO2007/044968 (referred to as TPT granules) or WO1997/016076 or WO1992/012645.

In another preferred embodiment when the feed additive composition is formulated into granules the granules comprise a hydrated barrier salt coated over the protein core. The advantage of such salt coating is improved thermo-tolerance, improved storage stability and protection against other feed additives otherwise having adverse effect on the enzyme.

Preferably, the salt used for the salt coating has a water activity greater than 0.25 or constant humidity greater than 60 % at 20 °C.

Preferably, the salt coating comprises a Na₂SO₄.

The method of preparing an enzyme (or composition comprising the enzyme) of the present invention may also comprise the further step of pelleting the powder. The powder may be mixed with other components known in the art. The powder, or mixture comprising the powder, may be forced through a die and the resulting strands are cut into suitable pellets of variable length.

Optionally, the pelleting step may include a steam treatment, or conditioning stage, prior to formation of the pellets. The mixture comprising the powder may be placed in a conditioner, e.g. a mixer with steam injection. The mixture is heated in the conditioner up to a specified temperature, such as from 60-100°C, typical temperatures would be 70°C, 80°C, 85°C, 90°C or 95°C. The residence time can be variable from seconds to minutes and even hours. Such as 5 seconds, 10 seconds, 15 seconds, 30 seconds, 1 minutes 2 minutes., 5 minutes, 10 minutes, 15 minutes, 30 minutes and 1 hour.

It will be understood that the enzyme (or composition comprising the enzyme) of the present invention is suitable for addition to any appropriate feed material.

It will be understood by the skilled person that different animals require different feedstuffs, and even the same animal may require different feedstuffs, depending upon the purpose for which the animal is reared.

Optionally, the feedstuff may also contain additional minerals such as, for example, calcium and/or additional vitamins.

Preferably, the feedstuff is a corn soybean meal mix.

In one embodiment, preferably the feed is not pet food.

In another aspect there is provided a method for producing a feedstuff. Feedstuff is typically produced in feed mills in which raw materials are first ground to a suitable particle size and then mixed with appropriate additives. The feedstuff may then be produced as a mash or pellets; the later typically involves a method by which the temperature is raised to a target level and then the feed is passed through a die to produce pellets of a particular size. The pellets are allowed to cool. Subsequently liquid additives such as fat and enzyme may be added. Production of feedstuff may also involve an additional step that includes extrusion or expansion prior to pelleting - in particular by suitable techniques that may include at least the use of steam.

The feedstuff may be a feedstuff for a monogastric animal, such as poultry (for example, broiler, layer, broiler breeders, turkey, duck, geese, water fowl), and swine (all age categories), a ruminant such as cattle (e.g. cows or bulls (including calves)), horses, sheep, a pet (for example dogs, cats) or fish (for example agastric fish, gastric fish, freshwater fish such as salmon, cod, trout and carp, e.g. koi carp, marine fish such as sea bass, and crustaceans such as shrimps, mussels and scallops). Preferably the feedstuff is for poultry.

### CORN BASED FEEDSTUFF

In a preferred embodiment the feedstuff may be a corn based feedstuff. The term "corn based feedstuff" as used herein means a feedstuff which comprises or consists of corn (maize) or a by-product of corn.

Preferably the corn based feedstuff comprises corn or a by-product of corn as the major constituent. For example the corn based feedstuff may comprise at least 35% corn or a by-product of corn, such as at least 40% corn or a by-product of corn, such as at least 50% corn or a by-product of corn, such as at least 60% corn or a by-product of corn, such as at least 70% corn or a by-product of corn, such as at least 80% or a by-product of corn, such as at least 90% corn or a by-product of corn, for example 100% corn or a by-product of corn.

In some embodiments the corn based feedstuff may comprise corn or a by-product of corn as a minor constituent; in which case the feedstuff may be supplemented with corn or a by-product of corn. By way of example only the feedstuff may comprise for example wheat supplemented with corn or a by-product of corn.

When corn or the by-product of corn is a minor constituent of the feedstuff, the corn or by-product of corn is at least 5%, preferably at least 10%, preferably at least 20%, preferably at least 30% of the feedstuff.

For the avoidance of doubt the term "corn" as used herein is synonymous with maize, e.g. *Zea mays.*

In one embodiment the by-product of corn may be corn Distillers Dried Grain Solubles (cDDGS) or corn wet-cake or corn Distillers Dried Grain (DDG) or corn gluten meal or corn gluten feed or combinations thereof.

In one embodiment preferably the arabinoxylan-containing material of the present invention comprises a by-product of corn, such as corn Distillers Dried Grain Solubles (cDDGS) or corn wet-cake or corn Distillers Dried Grain (DDG) or corn gluten meal or corn gluten feed or combinations thereof.

### WHEAT BASED FEEDSTUFF

In a preferred embodiment the feedstuff may be a wheat based feedstuff. The term "wheat based feedstuff" as used herein means a feedstuff which comprises or consists of wheat or a by-product of wheat.

Preferably the wheat based feedstuff comprises wheat or a by-product of wheat as the major constituent. For example the wheat based feedstuff may comprise at least 40% wheat or a by-product of wheat, such as at least 60% wheat or a by-product of wheat, such as at least 80% or a by-product of wheat, such as at least 90% wheat or a by-product of wheat, for example 100% wheat or a by-product of wheat.

In some embodiments the wheat based feedstuff may comprise wheat or a by-product of wheat as a minor constituent; in which case the feedstuff may be supplemented with wheat or a by-product of wheat. By way of example only the feedstuff may comprise for example wheat supplemented with wheat or a by-product of wheat.

When wheat or the by-product of wheat is a minor constituent of the feedstuff, the wheat or by-product of wheat is at least 5%, preferably at least 10%, preferably at least 20%, preferably at least 30% of the feedstuff.

In one embodiment the by-product of wheat may be wheat bran, wheat middlings, wheat fibres for example.

Bran is the hard outer layer of grain and consists of combined aleurone and pericarp. Along with germ, it is an integral part of whole grains, and is often produced as a by-product of milling in the production of refined grains. When bran is removed from grains, the grains lose a portion of their nutritional value. Bran is present in and may be milled from any cereal grain, including rice, corn (maize), wheat, oats, barley and millet. Bran is particularly rich in dietary fibre and essential fatty acids and contains significant quantities of starch, protein, vitamins and dietary minerals.

Wheat middlings is coarse and fine particles of wheat bran and fine particles of wheat shorts, wheat germ, wheat flour and offal from the "tail of the mill".

Wheat middlings is an inexpensive by-product intermediate of human food and animal feed. In one embodiment preferably the arabinoxylan-containing material of the present invention comprises wheat bran and/or wheat middlings.

### WET-CAKE, DISTILLERS DRIED GRAINS (DDG) AND DISTILLERS DRIED GRAIN SOLUBLES (DDGS)

Wet-cake, Distillers Dried Grains and Distillers Dried Grains with Solubles are products obtained after the removal of ethyl alcohol by distillation from yeast fermentation of a grain or a grain mixture by methods employed in the grain distilling industry.

Stillage coming from the distillation (e.g. comprising water, remainings of the grain, yeast cells etc.) is separated into a "solid" part and a liquid part.

The solid part is called "wet-cake" and can be used as animal feed as such.

The liquid part is (partially) evaporated into a syrup (solubles).

When the wet-cake is dried it is Distillers Dried Grains (DDG).

When the wet-cake is dried together with the syrup (solubles) it is Distillers Dried Grans with Solubles (DDGS).

Wet-cake may be used in dairy operations and beef cattle feedlots.

The dried DDGS may be used in livestock, e.g. dairy, beef and swine) feeds and poultry feeds.

Corn DDGS is a very good protein source for dairy cows.

### CORN GLUTEN MEAL

In one aspect, the by-product of corn may be corn gluten meal (CGM).

CGM is a powdery by-product of the corn milling industry. CGM has utility in, for example, animal feed. It can be used as an inexpensive protein source for feed such as pet food, livestock feed and poultry feed. It is an especially good source of the amino acid cysteine, but must be balanced with other proteins for lysine.

### FEED ADDITIVE COMPOSITION

The feed additive composition of the present invention and/or the feedstuff comprising same may be used in any suitable form.

The feed additive composition of the present invention may be used in the form of solid or liquid preparations or alternatives thereof. Examples of solid preparations include powders, pastes, boluses, capsules, pellets, tablets, dusts, and granules which may be wettable, spray-dried or freeze-dried. Examples of liquid preparations include, but are not limited to, aqueous, organic or aqueous-organic solutions, suspensions and emulsions.

In some applications, the feed additive compositions of the present invention may be mixed with feed or administered in the drinking water.

In one aspect the present invention relates to a method of preparing a feed additive composition, comprising admixing a xylanase as taught herein with a feed acceptable carrier, diluent or excipient, and (optionally) packaging.

### PREMIX

The feedstuff and/or feed additive composition may be combined with at least one mineral and/or at least one vitamin. The compositions thus derived may be referred to herein as a premix.

### MALTING AND BREWING

The enzyme having xylanase activity, e.g. the GH10 xylanase enzyme (such as the parent or modified GH10 xylanase enzyme) or a fragment thereof, of the present invention (or composition comprising the enzyme) of the present invention may be used in malting and brewing.

Barley grains contain 1.7 to 4.1% (w/w) water-extractable and 3.6 to 6.4% (w/w) total beta - glucan (Anderson, M.A., Cook, J.A., & Stone, B.A., Journal of the Institute of Brewing, 1978, 84, 233-239; Henry, J., Journal of the Science of Food and Agriculture, 1985, 36, 1243).

Wheat grains contain 0.1 to 0.8% (w/w) water-extractable and 0.6 to 1.4% (w/w) total beta - glucan (Anderson, M.A. *et al* (1978) *supra*).

Efficient hydrolysis of arabinoxylans (AXsol) and beta-glucan is important because such compounds can be involved in production problems such as wort viscosity (Ducroo, P. & Frelon, P.G., Proceedings of the European Brewery Convention Congress, Zurich, 1989, 445; Viëtor, R.J. & Voragen, A.G.J., Journal of the Institute of Brewing, 1993, 99, 243) and filterability and haze formation (Coote, N. & Kirsop, B.H. 1976., Journal of the Institute of Brewing, 1976, 82, 34; Izawa, M., Kano, Y. & Kanimura, M. 1991. Proceedings Aviemore Conference on Malting, brewing and Distillling, 1990, 427).

The present invention provides a method of hydrolysing arabinoxylans (e.g. AXinsol and AXsol) during malting and brewing wherein wheat grains, barley grains or a combination thereof, or portions of the wheat and/or barley grains, are admixed with the modified xylanase of the present invention.

In one aspect of the present invention may relate to a food composition that is a beverage, including, but not limited to, a fermented beverage such as beer and wine, comprising an enzyme having xylanase activity, e.g. the GH10 xylanase enzyme (such as the parent or modified GH10 xylanase enzyme) or a fragment thereof, of the present invention.

In another aspect of the present invention may relate to a food composition that is a beverage, including, but not limited to, a fermented beverage such as beer and wine, comprising a modified xylanase according to the present invention.

In the context of the present invention, the term "fermented beverage" is meant to comprise any beverage produced by a method comprising a fermentation process, such as a microbial fermentation, such as a bacterial and/or yeast fermentation.

In an aspect of the invention the fermented beverage is beer. The term "beer" is meant to comprise any fermented wort produced by fermentation/brewing of a starch-containing plant material. Often, beer is produced from malt or adjunct, or any combination of malt and adjunct as the starch-containing plant material. As used herein the term "malt" is understood as any malted cereal grain, such as malted barley or wheat.

As used herein the term "adjunct" refers to any starch and/or sugar containing plant material which is not malt, such as barley or wheat malt. As examples of adjuncts, mention can be made of materials such as common corn grits, refined corn grits, brewer's milled yeast, rice, sorghum, refined corn starch, barley, barley starch, dehusked barley, wheat, wheat starch, torrified cereal, cereal flakes, rye, oats, corn (maize), potato, tapioca, cassava and syrups, such as corn syrup, sugar cane syrup, inverted sugar syrup, barley and/or wheat syrups, and the like may be used as a source of starch.

As used herein, the term "mash" refers to an aqueous slurry of any starch and/or sugar containing plant material such as grist, e. g. comprising crushed barley malt, crushed barley, and/or other adjunct or a combination hereof, mixed with water later to be separated into wort and spent grains.

As used herein, the term "wort" refers to the unfermented liquor run-off following extracting the grist during mashing.

In another aspect the invention relates to a method of preparing a fermented beverage such as beer comprising mixing the modified xylanase of the present invention with malt or adjunct.

Examples of beers comprise: full malted beer, beer brewed under the "Reinheitsgebot", ale, IPA, lager, bitter, Happoshu (second beer), third beer, dry beer, near beer, light beer, low alcohol beer, low calorie beer, porter, bock beer, stout, malt liquor, non-alcoholic beer, non-alcoholic malt liquor and the like, but also alternative cereal and malt beverages such as fruit flavored malt beverages, e. g. citrus flavored, such as lemon-, orange-, lime-, or berry-flavored malt beverages, liquor flavored malt beverages, e. g. , vodka-, rum-, or tequila-flavored malt liquor, or coffee flavored malt beverages, such as caffeine-flavored malt liquor, and the like.

### BREAKDOWN OF GRAIN-BASED MATERIAL E.G. FOR BIOFUEL PRODUCTION

The enzyme having xylanase activity, e.g. the GH10 xylanase enzyme (such as the parent or modified GH10 xylanase enzyme) or a fragment thereof, of the present invention (or composition comprising the enzyme) may be used to breakdown (degrade) AXinsol and AXsol during grain processing from e.g. grain-based material. The grain-based material may be whole grains (e.g. whole wheat, barley, rye, triticale or corn grains or mixtures thereof) or portions of the whole grains, or mixtures thereof.

In one embodiment the enzyme having xylanase activity, e.g. the GH10 xylanase enzyme (such as the parent or modified GH10 xylanase enzyme) or a fragment thereof, of the present invention (or composition comprising the enzyme) may be used to breakdown (degrade) AXinsol and AXsol in grain-based materials or whole grains.

For the avoidance of doubt the whole grains can be mechanically broken.

The grain-based material may be broken down or degraded to glucose. The glucose may subsequently be used as a feedstock for any fermentation process, e.g. for biofuel (e.g. bioethanol) production and/or biochemicals (e.g., bio-based isoprene) production.

The grain-based material may be feedstock for a biofuel (e.g. bioethanol) production process.

Today most fuel ethanol is produced from corn (maize) grain, which is milled or grinded, treated with amylase enzymes to hydrolyse starch to sugars, fermented, and distilled. While substantial progress has been made in reducing costs of ethanol production, substantial challenges remain. Improved techniques are still needed to reduce the cost of biofuel feedstocks for ethanol production. For example, in grain-based ethanol production degradation of arabinoxylans may increase accessibility of starch.

The present invention provides a modified xylanase for use in the breakdown of hemicelluloses, e.g. arabinoxylan - particularly AXinsol and AXsol.

By way of example only, in the European fuel alcohol industry, small grains like wheat, barley and rye are common raw materials, in the US corn is mainly used. Wheat, barley and rye contain, next to starch, high levels of non-starch polysaccharide polymers (NSP), like cellulose, beta-glucan and hemicellulose.

The ratio in which the different NSPs are represented differ for each feedstock. The table below shows the different amounts of NSPs in wheat, barley and rye compared to some other feedstocks.

**Table 1: Non-starch Polysaccharides present in different feedstocks (g kg⁻¹ dry matter)**

| | Corn | Wheat | Rye | Barley | | Oats | |
|---|---|---|---|---|---|---|---|
| | | | | Hulled | Hulless | Hulled | Hulless |
| Beta-Glucan | 1 | 8 | 16 | 42 | 42 | 28 | 41 |
| Cellulose | 22 | 17-20 | 15-16 | 43 | 10 | 82 | 14 |
| Soluble and Non-soluble NCP¹ | 75 | 89-99 | 116-136 | 144 | 114 | 150 | 113 |
| Total NSP | 97 | 107-119 | 132-152 | 186 | 124 | 232 | 116 |

| | | | | | | | |
|---|---|---|---|---|---|---|---|
| Non Cellulosic Polysaccharides: pentosans, (arabino)xylans and other hemicelluloses | | | | | | | |

NSPs can give high viscosity to grain mashes. High viscosity has a negative impact on ethanol production since it will limit the solid concentration that can be used in mashing and it will reduce the energy efficiency of the process. In addition, residual hemicelluloses present throughout the process may contribute to fouling in heat exchangers and distillation equipment. The largest impact of a high viscosity is seen when a mash is cooled to fermentation temperature (32°C). This explains that the viscosity needs to be reduced in the process anywhere before the cooling step.

In one embodiment of the present invention the method for degrading grain-based material comprises admixing the modified xylanase as disclosed herein as early as possible in the biofuel (e.g. bioethanol) production process, e.g. preferably during mixing of the grain-based material at the start of the process. One advantage of adding the modified xylanases as disclosed herein at an early stage in the process is that the enzymes breakdown initial viscosity.

In one embodiment of the present invention the method for degrading grain-based material comprises admixing the modified xylanase as disclosed herein prior to or during liquefaction, saccharification, fermentation, simultaneous saccharification and fermentation, post fermentation or a combination thereof.

Therefore in one embodiment the present invention relates to reducing viscosity when degrading grain-based materials, e.g. in biofuel (e.g. bioethanol) production processes.

The benefits of using the enzyme having xylanase activity, e.g. the GH10 xylanase enzyme (such as the parent or modified GH10 xylanase enzyme) or a fragment thereof, of the present invention and taught herein to reduce viscosity when degrading grain-based materials, e.g. in biofuel (e.g. bioethanol) production processes are multiple:
- Higher dry substance mash can be used in the process
- Higher solids content of final syrup can be obtained
- Better heat transfer, lower energy requirement
- Reduced evaporator fouling leading to reduced cleaning costs
- Increased final ethanol yields
- Improved quality of DDGS (by-product)
- Better separation between the solid and liquid part during stillage separation (after distillation). The lower viscosity increases separation efficiency.

A further significant advantage of the present invention is that use of the enzyme having xylanase activity, e.g. the GH10 xylanase enzyme (such as the parent or modified GH10 xylanase enzyme) or a fragment thereof, of the present invention in biofuel production can also result in improved (by)products from that process such as wet-cake, Distillers Dried Grains (DDG) or Distillers Dried Grains with Solubles (DDGS). Therefore one advantage of the present invention is since the wet-cake, DDG and DDGS are (by)products of biofuel (e.g. bioethanol) production the use of the present invention can result is improved quality of these (by)products. For example the arabinoxylans in the (by)products can be already dissolved during the biofuel production process.

### CEREAL (E.G. WHEAT) GLUTEN-STARCH SEPARATION

The enzyme having xylanase activity, e.g. the GH10 xylanase enzyme (such as the parent or modified GH10 xylanase enzyme) or a fragment thereof, of the present invention (or composition comprising the enzyme) of the present invention or as disclosed herein may be used to breakdown (degrade) AXinsol and AXsol during wheat starch and gluten separation.

After initial separation of the wheat bran and germ from the endosperm, fractionation of wheat endosperm flour into starch and gluten fractions is industrially applied on large scale to obtain high quality A-starch and byproducts B-starch and vital gluten.

The product of the degradation of the cereal flour (e.g. wheat flour) in the present invention is starch (high quality A-starch).

In addition, by-products B-starch and vital gluten are also produced. Each individual product is then further processed to supplement or modify food product characteristics to the market needs.

There are several wheat separation processes used by industry described in literature. These industrial processes differ mainly in the forms of the flour-water mixtures presented to the fractionation equipment (centrifuge, hydrocyclone, or screen) or in the initial reaction conditions as temperature and applying of shear *(*Abdulvahit Sayaslan, Lebensm.-Wiss. U.-Technol 37 (2004) 499-515, Wetmilling of wheat flour: industrial processes and small-sacale test methods*).*

In the method for separating a cereal flour (e.g. wheat flour) into starch and gluten fractions the method comprises admixing a cereal flour (e.g. wheat flour), water and a modified xylanase. The cereal flour, water and modified xylanase may be mixed simultaneously or sequentially. In some embodiments the cereal flour (e.g. wheat flour) and water may be admixed before admixing with the modified xylanase.

In general, cereal flour (e.g. wheat flour) is either mixed to a dough or batter, varying between 35 to 63% Dry solids, at temperatures of ∼20-45°C. The mixture is then further processed either by:
1) letting the mixture rest for some time (∼30 minutes) and sequentially washing out the starch from the mixture using a screen, centrifuge or hydrocyclone to separate the starch milk from the gluten, or
2) applying shear to the mixture, optionally diluting the mixture further and then separating the wheat flour by a hydrocyclone, or a 2- or 3-phase decanter centrifuge.

The term "dry solids" as used herein means total solids (dissolved and undissolved) of a slurry (in %) on a dry weight basis.

In one embodiment of the present invention the method or use as claimed may include the steps of mixing wheat flour to form a dough or batter between 35-63% dry solids, at a temperature of about 20 to about 45°C and separating the starch from the gluten.

The method of the present invention may further comprise:
a) resting the mixture for about 30 minutes and sequentially washing out the starch from the mixture using either a screen, a centrifuge or a hydrocyclone to separate the starch milk from the gluten; or
b) applying shear to the mixture and optionally diluting the mixture further, separating the starch from the gluten using a hydrocyclone or a 2- or 3-phase decanter centrifuge.

The present invention provides for improving the separation of the starch and the gluten by adding a enzyme having xylanase activity, e.g. the GH10 xylanase enzyme (such as the parent or modified GH10 xylanase enzyme) or a fragment thereof, of the present invention suitably during the initial mixing step of flour and water in the various processes described above used for wheat starch separation. Separation is improved by adding a modified xylanase during the initial mixing step due to viscosity reduction and the hydrolysis of AXsol and/or AXinsol interfering with the gluten particles. By degrading these poly- and oligosaccharides, gluten agglomeration is enhanced, improving the gluten yield. *(*S.A. Frederix, C.M. Courtin, J.A. Delcour, J. Cereal Sci. 40 (2004) 41-49*, Substrate selectivity and inhibitor sensitivity affect xylanase functionality in wheat flour gluten-starch separation).*

One advantage of the present invention is that it results in higher A-starch yields and/or better quality gluten (e.g. better quality vital gluten).

One advantage of the present invention is that it improves wheat gluten-starch separation.

One of the ways to evaluate gluten quality is by monitoring gluten agglomeration. When a certain amount of friction through kneading of the dough or mixing of the batter is applied, gluten particles tend to agglomerate into larger particles that form a polymeric network, called "vital gluten". "Vital gluten" can be added to food products to improve properties of baked goods such as dough strength, shelf-life and bread volume (L. Day, M.A. Augustin, I.L. Batey and C. W. Wrigley; Wheat-gluten uses and industry needs; Trends in Food Science & Technology 17 (2006) 82-90).

In the bakery industry, the quality and quantity of the gluten in a wheat flour is determined by the ICC standard assay No. 155 (AACC 38-12) using a Glutomatic. In this device, a dough is formed from wheat flour (10.0 gr) mixed with a small amount of 2% NaCl solution (4.2 - 4.8 ml). After 20 seconds of mixing step, the dough is continuously kneaded while being washed for 5 minutes with a 2% NaCl solution at room temperature (∼22°C) pumped through the mixing cup at a flow rate of ∼70 ml/minute. During this washing step, the wash water containing starch is collected and the gluten particles form a gluten ball within the Glutomatic sieve holder.

The quality of the gluten is measured by evaluating the gluten agglomeration. This is done by centrifuging the gluten ball in a special centrifuge containing a small sieve. The gluten particles that pass this sieve are weighed (small gluten) and the total amount of gluten is weighed. The gluten index is calculated by (total wet gluten - small wet gluten)/total wet gluten. The more gluten agglomeration is improved, the smaller the small gluten fraction will be and the higher the gluten index value is. A high gluten index, with a theoretical maximum of 100%, indicates a high quality gluten ball.

Another value to quantify the amount of gluten is the dried gluten yield (%). This value is calculated by dividing the grams of total dried gluten by the total amount of dry flour which was used in the experiment. The more dried gluten is recovered, the better the separation is.
This industrial assay is currently under adaptation to simulate a dough separation process used in industry.

### DOSAGES

Preferably, the enzyme having xylanase activity, e.g. the GH10 xylanase enzyme (such as the parent or modified GH10 xylanase enzyme) or a fragment thereof, of the present invention is present in the xylan-containing material (e.g. feedstuff) in the range of about 500XU/kg to about 16,000XU/kg xylan-containing material (e.g. feed), more preferably about 750XU/kg feed to about 8000XU/kg xylan-containing material (e.g. feed), preferably about 1500XU/kg feed to about 3000XU/kg xylan-containing material (e.g. feed), preferably about 2000XU/kg feed to about 2500XU/kg xylan-containing material (e.g. feed), and even more preferably about 1000XU/kg xylan-containing material (e.g. feed) to about 4000XU/kg xylan-containing material (e.g. feed).

In one embodiment the enzyme having xylanase activity, e.g. the GH10 xylanase enzyme (such as the parent or modified GH10 xylanase enzyme) or a fragment thereof, of the present invention is present in the xylan-containing material (e.g. feedstuff) at more than about 500XU/kg xylan-containing material (e.g. feed), suitably more than about 600XU/kg xylan-containing material (e.g. feed), suitably more than about 700XU/kg xylan-containing material (e.g. feed), suitably more than about 800XU/kg xylan-containing material (e.g. feed), suitably more than about 900XU/kg xylan-containing material (e.g. feed), suitably more than about 1000XU/kg xylan-containing material (e.g. feed), suitably more than about 2000XU/kg, suitably more than about 2500XU/kg, suitably more than about 3000XU/kg xylan-containing material (e.g. feed),

In one embodiment the enzyme having xylanase activity, e.g. the GH10 xylanase enzyme (such as the parent or modified GH10 xylanase enzyme) or a fragment thereof, of the present invention is present in the xylan-containing material (e.g. feedstuff) at a concentration of between about 2000XU/kg to about 2500XU/kg.

In one embodiment the enzyme having xylanase activity, e.g. the GH10 xylanase enzyme (such as the parent or modified GH10 xylanase enzyme) or a fragment thereof, of the present invention is present in the xylan-containing material (e.g. feedstuff) at less than about 16,000XU/kg xylan-containing material (e.g. feed), suitably less than about 8000XU/kg xylan-containing material (e.g. feed), suitably less than about 7000XU/kg xylan-containing material (e.g. feed), suitably less than about 6000XU/kg xylan-containing material (e.g. feed), suitably less than about 5000XU/kg xylan-containing material (e.g. feed), suitably less than about 4000XU/kg xylan-containing material (e.g. feed).

Preferably, the enzyme having xylanase activity, e.g. the GH10 xylanase enzyme (such as the parent or modified GH10 xylanase enzyme) or a fragment thereof, of the present invention may be present in a feed additive composition in range of about 100XU/g to about 320,000XU/g composition, more preferably about 300XU/g composition to about 160,000XU/g composition, and even more preferably about 500XU/g composition to about 50,000 XU/g composition, and even more preferably about 500XU/g composition to about 40,000 XU/g composition.

In one embodiment the enzyme having xylanase activity, e.g. the GH10 xylanase enzyme (such as the parent or modified GH10 xylanase enzyme) or a fragment thereof, of the present invention is present in the feed additive composition at more than about 100XU/g composition, suitably more than about 200XU/g composition, suitably more than about 300XU/g composition, suitably more than about 400XU/g composition, suitably more than about 500XU/g composition.

In one embodiment the enzyme having xylanase activity, e.g. the GH10 xylanase enzyme (such as the parent or modified GH10 xylanase enzyme) or a fragment thereof, of the present invention is present in the feed additive composition at less than about 320,000XU/g composition, suitably less than about 160,000XU/g composition, suitably less than about 50,000XU/g composition, suitably less than about 40,000XU/g composition, suitably less than about 30000XU/g composition.

The xylanase activity can be expressed in xylanase units (XU) measured at pH 5.0 with AZCL-arabinoxylan (azurine-crosslinked wheat arabinoxylan, Xylazyme tablets, Megazyme) as substrate. Hydrolysis by endo-(1-4)-β-D-xylanase (xylanase) produces water soluble dyed fragments, and the rate of release of these (increase in absorbance at 590 nm) can be related directly to enzyme activity. The xylanase units (XU) are determined relatively to an enzyme standard (Danisco Xylanase, available from Danisco Animal Nutrition) at standard reaction conditions, which are 40 °C, 5 min reaction time in Mcllvaine buffer, pH 5.0.

The xylanase activity of the standard enzyme is determined as amount of released reducing sugar end groups from an oat-spelt-xylan substrate per min at pH 5.3 and 50°C. The reducing sugar end groups react with 3, 5-Dinitrosalicylic acid and formation of the reaction product can be measured as increase in absorbance at 540 nm. The enzyme activity is quantified relative to a xylose standard curve (reducing sugar equivalents). One xylanase unit (XU) is the amount of standard enzyme that releases 0.5 µmol of reducing sugar equivalents per min at pH 5.3 and 50°C.

In one embodiment suitably the enzyme is classified using the E.C. classification above, and the E.C. classification designates an enzyme having that activity when tested in the assay taught herein for determining 1 XU.

Preferably, the enzyme having xylanase activity, e.g. the GH10 xylanase enzyme (such as the parent or modified GH10 xylanase enzyme) or a fragment thereof, of the present invention is present in the mixing step of a wheat starch separation process in the dough or batter in the range of about 0.01 kg/MT DS dough or batter to about 0.60 kg/MT DS, more preferably about 0.05 kg/MT DS to about 0.45 kg/MT DS dough or batter, and even more preferably about 0.10 kg/MT DS to about 0.25 kg/MT DS dough or batter.

In some embodiments (particularly in the wheat starch separation embodiment) the enzyme having xylanase activity, e.g. the GH10 xylanase enzyme (such as the parent or modified GH10 xylanase enzyme) or a fragment thereof, of the present invention may be dosed in the range of about 0.019 g protein/MT DS wheat flour (which is equivalent to 0.019 mg/kg DS) to about 119 g protein/MT DS wheat flour (which is equivalent to 119 mg/kg DS - where DS means dry solids content and MT means metric ton.

In some embodiments (particularly in the wheat starch separation embodiment) the enzyme having xylanase activity, e.g. the GH10 xylanase enzyme (such as the parent or modified GH10 xylanase enzyme) or a fragment thereof, of the present invention may be dosed at about 1.19 g protein/MT DS wheat flour (which is equivalent to about 1.19 mg/kg DS) - where DS means dry solids content and MT means metric ton.

In some embodiments (particularly in the wheat starch separation embodiment) the enzyme having xylanase activity, e.g. the GH10 xylanase enzyme (such as the parent or modified GH10 xylanase enzyme) or a fragment thereof, of the present invention may be dosed in the range of about 9 to about 120000 units/kg wheat flour, suitably between about 500-2400 units/kg wheat flour, suitably between about 900-1200 units/kg wheat flour (wherein 1 unit is defined as the amount of enzyme required to generate 1 micromole of xylose reducing sugar equivalents per minute under the conditions of the birch wood assay of Example 4).

In some embodiments (particularly in degrading grain-based material) the enzyme having xylanase activity, e.g. the GH10 xylanase enzyme (such as the parent or modified GH10 xylanase enzyme) or a fragment thereof, of the present invention may be dosed in the range of about 0.29 g/protein/MT DS wheat (which is equivalent to 0.29 mg/kg DS) to about 0290 g/protein/MT DS wheat (which is equivalent to 290 mg/kg DS).

In some embodiments (particularly in degrading grain-based material) the xylanase may be dosed at 2.9 g/protein/MT DS wheat (which is equivalent to 2.9 mg/kg DS).

In some embodiments (particularly in degrading grain-based material) the xylanase may be dosed in the range of about 22 to about 285000 units/kg, suitably about 1100 to about 5700 units/kg, suitably about 2200 to about 2850 units/kg (wherein 1 unit is defined as the amount of enzyme required to generate 1 micromole of xylose reducing sugar equivalents per minute under the conditions of the birch wood assay of Example 4).

The enzyme having xylanase activity, e.g. the GH10 xylanase enzyme (such as the parent or modified GH10 xylanase enzyme) or a fragment thereof, of the present invention and/or composition comprising the enzyme according to the present invention may be designed for one-time dosing or may be designed for use (e.g. feeding) on a daily basis.

The optimum amount of the enzyme having xylanase activity, e.g. the GH10 xylanase enzyme (such as the parent or modified GH10 xylanase enzyme) or a fragment thereof, of the present invention and/or composition comprising the enzyme to be used in the present invention will depend on the product to be treated and/or the method of contacting the product with the composition and/or the intended use for the same.

The amount of enzyme having xylanase activity, e.g. the GH10 xylanase enzyme (such as the parent or modified GH10 xylanase enzyme) or a fragment thereof, of the present invention used in the compositions should be a sufficient amount to be effective.

The amount of enzyme having xylanase activity, e.g. the GH10 xylanase enzyme (such as the parent or modified GH10 xylanase enzyme) or a fragment thereof, of the present invention used in the compositions should be a sufficient amount to be effective and to remain sufficiently effective in for example improving the performance of an animal fed feed products containing said composition. This length of time for effectiveness should extend up to at least the time of utilisation of the product (e.g. feed additive composition or feed containing same).

### FORMULATION

In one embodiment the enzyme having xylanase activity, e.g. the GH10 xylanase enzyme (such as the parent or modified GH10 xylanase enzyme) or a fragment thereof, of the present invention may be formulated as a liquid, a dry powder or a granule.

The dry powder or granules may be prepared by means known to those skilled in the art, such as, in top-spray fluid bed coater, in a buttom spray Wurster or by drum granulation (e.g. High sheer granulation), extrusion, pan coating or in a microingredients mixer.

For some embodiments the enzyme having xylanase activity, e.g. the GH10 xylanase enzyme (such as the parent or modified GH10 xylanase enzyme) or a fragment thereof, of the present invention may be coated, for example encapsulated.

In one embodiment the coating protects the modified enzyme from heat and may be considered a thermoprotectant.

In one embodiment the feed additive composition is formulated to a dry powder or granules as described in WO2007/044968 (referred to as TPT granules) or WO1997/016076 or WO1992/012645.

In one embodiment the feed additive composition may be formulated to a granule for feed compositions comprising: a core; an active agent; and at least one coating, the active agent of the granule retaining at least 50% activity, at least 60% activity, at least 70% activity, at least 80% activity after conditions selected from one or more of a) a feed pelleting process, b) a steam-heated feed pretreatment process, c) storage, d) storage as an ingredient in an unpelleted mixture, and e) storage as an ingredient in a feed base mix or a feed premix comprising at least one compound selected from trace minerals, organic acids, reducing sugars, vitamins, choline chloride, and compounds which result in an acidic or a basic feed base mix or feed premix.

With regard to the granule at least one coating may comprise a moisture hydrating material that constitutes at least 55% w/w of the granule; and/or at least one coating may comprise two coatings. The two coatings may be a moisture hydrating coating and a moisture barrier coating. In some embodiments, the moisture hydrating coating may be between 25% and 60% w/w of the granule and the moisture barrier coating may be between 2% and 15% w/w of the granule. The moisture hydrating coating may be selected from inorganic salts, sucrose, starch, and maltodextrin and the moisture barrier coating may be selected from polymers, gums, whey and starch.

The granule may be produced using a feed pelleting process and the feed pretreatment process may be conducted between 70°C and 95°C for up to several minutes, such as between 85°C and 95°C.

In one embodiment the feed additive composition may be formulated to a granule for animal feed comprising: a core; an active agent, the active agent of the granule retaining at least 80% activity after storage and after a steam-heated pelleting process where the granule is an ingredient; a moisture barrier coating; and a moisture hydrating coating that is at least 25% w/w of the granule, the granule having a water activity of less than 0.5 prior to the steam-heated pelleting process.

The granule may have a moisture barrier coating selected from polymers and gums and the moisture hydrating material may be an inorganic salt. The moisture hydrating coating may be between 25% and 45% w/w of the granule and the moisture barrier coating may be between 2% and 10% w/w of the granule.

The granule may be produced using a steam-heated pelleting process which may be conducted between 85°C and 95°C for up to several minutes.

In some embodiments the enzyme may be diluted using a diluent, such as starch powder, lime stone or the like.

In one embodiment, the enzyme having xylanase activity, e.g. the GH10 xylanase enzyme (such as the parent or modified GH10 xylanase enzyme) or a fragment thereof, of the present invention or composition comprising the enzyme is in a liquid formulation suitable for consumption preferably such liquid consumption contains one or more of the following: a buffer, salt, sorbitol and/or glycerol.

In another embodiment the enzyme having xylanase activity, e.g. the GH1 0 xylanase enzyme (such as the parent or modified GH10 xylanase enzyme) or a fragment thereof, of the present invention or composition comprising the enzyme may be formulated by applying, e.g. spraying, the enzyme(s) onto a carrier substrate, such as ground wheat for example.

In one embodiment the enzyme having xylanase activity, e.g. the GH10 xylanase enzyme (such as the parent or modified GH10 xylanase enzyme) or a fragment thereof, of the present invention or composition comprising the enzyme according to the present invention may be formulated as a premix. By way of example only the premix may comprise one or more feed components, such as one or more minerals and/or one or more vitamins.

In one embodiment the enzyme having xylanase activity, e.g. the GH10 xylanase enzyme (such as the parent or modified GH10 xylanase enzyme) or a fragment thereof, for use in the present invention are formulated with at least one physiologically acceptable carrier selected from at least one of maltodextrin, limestone (calcium carbonate), cyclodextrin, wheat or a wheat component, sucrose, starch, Na₂SO₄, Talc, PVA, sorbitol, benzoate, sorbiate, glycerol, sucrose, propylene glycol, 1,3-propane diol, glucose, parabens, sodium chloride, citrate, acetate, phosphate, calcium, metabisulfite, formate and mixtures thereof.

### PACKAGING

In one embodiment the enzyme having xylanase activity, e.g. the GH10 xylanase enzyme (such as the parent or modified GH10 xylanase enzyme) or a fragment thereof, of the present invention and/or composition comprising same (e.g. feed additive composition) and/or premix and/or feed or feedstuff according to the present invention is packaged.

In one preferred embodiment the feed additive composition and/or premix and/or feed or feedstuff is packaged in a bag, such as a paper bag.

In an alternative embodiment the feed additive composition and/or premix and/or feed or feedstuff may be sealed in a container. Any suitable container may be used.

### FORMS

The enzyme having xylanase activity, e.g. the GH10 xylanase enzyme (such as the parent or modified GH10 xylanase enzyme) or a fragment thereof, of the present invention or composition comprising the enzyme (e.g. the feed additive composition) of the present invention and other components and/or the feedstuff comprising same may be used in any suitable form.

The enzyme having xylanase activity, e.g. the GH10 xylanase enzyme (such as the parent or modified GH10 xylanase enzyme) or a fragment thereof, of the present invention or composition comprising same (e.g. feed additive composition) of the present invention may be used in the form of solid or liquid preparations or alternatives thereof. Examples of solid preparations include powders, pastes, boluses, capsules, pellets, tablets, pills, capsules, ovules, solutions or suspensions, dusts, and granules which may be wettable, spray-dried or freeze-dried. Examples of liquid preparations include, but are not limited to, aqueous, organic or aqueous-organic solutions, suspensions and emulsions.

The composition comprising the enzyme having xylanase activity, e.g. the GH10 xylanase enzyme (such as the parent or modified GH10 xylanase enzyme) or a fragment thereof, of the present invention may contain flavouring or colouring agents, for immediate-, delayed-, modified-, sustained-, pulsed- or controlled-release applications.

By way of example, if the composition of the present invention is used in a solid, e.g. pelleted form, it may also contain one or more of: excipients such as microcrystalline cellulose, lactose, sodium citrate, calcium carbonate, dibasic calcium phosphate and glycine; disintegrants such as starch (preferably corn, potato or tapioca starch), sodium starch glycollate, croscarmellose sodium and certain complex silicates; granulation binders such as polyvinylpyrrolidone, hydroxypropylmethylcellulose (HPMC), hydroxypropylcellulose (HPC), sucrose, gelatin and acacia; lubricating agents such as magnesium stearate, stearic acid, glyceryl behenate and talc may be included.

Examples of nutritionally acceptable carriers for use in preparing the forms include, for example, water, salt solutions, alcohol, silicone, waxes, petroleum jelly, vegetable oils, polyethylene glycols, propylene glycol, liposomes, sugars, gelatin, lactose, amylose, magnesium stearate, talc, surfactants, silicic acid, viscous paraffin, perfume oil, fatty acid monoglycerides and diglycerides, petroethral fatty acid esters, hydroxymethyl-cellulose, polyvinylpyrrolidone, and the like.

Preferred excipients for the forms include lactose, starch, a cellulose, milk sugar or high molecular weight polyethylene glycols.

For aqueous suspensions and/or elixirs, the composition of the present invention may be combined with various sweetening or flavouring agents, colouring matter or dyes, with emulsifying and/or suspending agents and with diluents such as water, propylene glycol and glycerin, and combinations thereof.

### SUBJECT

The term "subject", as used herein, means an animal that is to be or has been administered with a modified xylanase according to the present invention or a feed additive composition according to the present invention or a feedstuff comprising said feed additive composition according to the present invention.

The term "subject", as used herein, means an animal.

In one embodiment, the subject is a mammal, bird, fish or crustacean including for example livestock or a domesticated animal (e.g. a pet).

In one embodiment the "subject" is livestock.

The term "livestock", as used herein refers to any farmed animal. Preferably, livestock is one or more of ruminants such as cattle (e.g. cows or bulls (including calves)), mono-gastric animals such as poultry (including broilers, chickens and turkeys), pigs (including piglets), birds, aquatic animals such as fish, agastric fish, gastric fish, freshwater fish such as salmon, cod, trout and carp, e.g. koi carp, marine fish such as sea bass, and crustaceans such as shrimps, mussels and scallops), horses (including race horses), sheep (including lambs).

In another embodiment the "subject" is a domesticated animal or pet or an animal maintained in a zoological environment.

The term "domesticated animal or pet or animal maintained in a zoological environment" as used herein refers to any relevant animal including canines (e.g. dogs), felines (e.g. cats), rodents (e.g. guinea pigs, rats, mice), birds, fish (including freshwater fish and marine fish), and horses.

### PERFORMANCE

As used herein, "animal performance" may be determined by the feed efficiency and/or weight gain of the animal and/or by the feed conversion ratio and/or by the digestibility of a nutrient in a feed (e.g. amino acid digestibility) and/or digestible energy or metabolizable energy in a feed and/or by nitrogen retention and/or by animals ability to avoid the negative effects of necrotic enteritis and/or by the immune response of the subject.

Preferably "animal performance" is determined by feed efficiency and/or weight gain of the animal and/or by the feed conversion ratio.

By "improved animal performance" it is meant that there is increased feed efficiency, and/or increased weight gain and/or reduced feed conversion ratio and/or improved digestibility of nutrients or energy in a feed and/or by improved nitrogen retention and/or by an improved immune response in the subject resulting from the use of feed additive composition of the present invention in feed in comparison to feed which does not comprise said feed additive composition.

Preferably, by "improved animal performance" it is meant that there is increased feed efficiency and/or increased weight gain and/or reduced feed conversion ratio.

As used herein, the term "feed efficiency" refers to the amount of weight gain per unit of feed when the animal is fed ad-libitum or a specified amount of feed during a period of time.

By "increased feed efficiency" it is meant that the use of a feed additive composition according the present invention in feed results in an increased weight gain per unit of feed intake compared with an animal fed without said feed additive composition being present.

### FEED CONVERSION RATIO (FCR)

As used herein, the term "feed conversion ratio" refers to the amount of feed fed to an animal to increase the weight of the animal by a specified amount.

An improved feed conversion ratio means a lower feed conversion ratio.

By "lower feed conversion ratio" or "improved feed conversion ratio" it is meant that the use of a feed additive composition in feed results in a lower amount of feed being required to be fed to an animal to increase the weight of the animal by a specified amount compared to the amount of feed required to increase the weight of the animal by the same amount when the feed does not comprise said feed additive composition.

### NUTRIENT DIGESTIBILITY

Nutrient digestibility as used herein means the fraction of a nutrient that disappears from the gastro-intestinal tract or a specified segment of the gastro-intestinal tract, e.g. the small intestine. Nutrient digestibility may be measured as the difference between what is administered to the subject and what comes out in the faeces of the subject, or between what is administered to the subject and what remains in the digesta on a specified segment of the gastro intestinal tract, e.g. the ileum.

Nutrient digestibility as used herein may be measured by the difference between the intake of a nutrient and the excreted nutrient by means of the total collection of excreta during a period of time; or with the use of an inert marker that is not absorbed by the animal, and allows the researcher calculating the amount of nutrient that disappeared in the entire gastro-intestinal tract or a segment of the gastro-intestinal tract. Such an inert marker may be titanium dioxide, chromic oxide or acid insoluble ash. Digestibility may be expressed as a percentage of the nutrient in the feed, or as mass units of digestible nutrient per mass units of nutrient in the feed.

Nutrient digestibility as used herein encompasses starch digestibility, fat digestibility, protein digestibility, and amino acid digestibility.

Energy digestibility as used herein means the gross energy of the feed consumed minus the gross energy of the faeces or the gross energy of the feed consumed minus the gross energy of the remaining digesta on a specified segment of the gastro-intestinal tract of the animal, e.g. the ileum. Metabolizable energy as used herein refers to apparent metabolizable energy and means the gross energy of the feed consumed minus the gross energy contained in the faeces, urine, and gaseous products of digestion. Energy digestibility and metabolizable energy may be measured as the difference between the intake of gross energy and the gross energy excreted in the faeces or the digesta present in specified segment of the gastro-intestinal tract using the same methods to measure the digestibility of nutrients, with appropriate corrections for nitrogen excretion to calculate metabolizable energy of feed.

### COMBINATION WITH OTHER COMPONENTS

The enzyme having xylanase activity, e.g. the GH10 xylanase enzyme (such as the parent or modified GH10 xylanase enzyme) or a fragment thereof, of the present invention may be used in combination with other components.

In one embodiment the enzyme having xylanase activity, e.g. the GH10 xylanase enzyme (such as the parent or modified GH10 xylanase enzyme) or a fragment thereof, of the present invention may be used in combination with a probiotic or a direct fed microbial (DFM), e.g. a direct fed bacteria.

The combination of the present invention comprises the enzyme having xylanase activity, e.g. the GH10 xylanase enzyme (such as the parent or modified GH10 xylanase enzyme) or a fragment thereof, of the present invention or a composition comprising the xylanase, e.g. a feed additive composition, and another component which is suitable for human or animal consumption and is capable of providing a medical or physiological benefit to the consumer.

In one embodiment the "another component" may be one or more further enzymes (e.g. further feed enzymes or brewing or malting enzymes, or grain processing enzymes or wheat gluten-starch separation enzymes).

Suitable additional enzymes for use in the present invention may be one or more of the enzymes selected from the group consisting of: endoglucanases (E.C. 3.2.1.4); celliobiohydrolases (E.C. 3.2.1.91), β-glucosidases (E.C. 3.2.1.21), cellulases (E.C. 3.2.1.74), lichenases (E.C. 3.1.1.73), lipases (E.C. 3.1.1.3), lipid acyltransferases (generally classified as E.C. 2.3.1.x), phospholipases (E.C. 3.1.1.4, E.C. 3.1.1.32 or E.C. 3.1.1.5), phytases (e.g. 6-phytase (E.C. 3.1.3.26) or a 3-phytase (E.C. 3.1.3.8), amylases, alpha-amylases (E.C. 3.2.1.1), other xylanases (E.C. 3.2.1.8, E.C. 3.2.1.32, E.C. 3.2.1.37, E.C. 3.1.1.72, E.C. 3.1.1.73), glucoamylases (E.C. 3.2.1.3), hemicellulases, proteases (e.g. subtilisin (E.C. 3.4.21.62) or a bacillolysin (E.C. 3.4.24.28) or an alkaline serine protease (E.C. 3.4.21.x) or a keratinase (E.C. 3.4.x.x)), debranching enzymes, cutinases, esterases and/or mannanases (e.g. a β-mannanase (E.C. 3.2.1.78)).

In one embodiment (particularly for feed applications) the other component may be one or more of the enzymes selected from the group consisting of an amylase (including α-amylases (E.C. 3.2.1.1), G4-forming amylases (E.C. 3.2.1.60), β-amylases (E.C. 3.2.1.2) and γ-amylases (E.C. 3.2.1.3)); and/or a protease (e.g. subtilisin (E.C. 3.4.21.62) or a bacillolysin (E.C. 3.4.24.28) or an alkaline serine protease (E.C. 3.4.21.x) or a keratinase (E.C. 3.4.x.x)) and/or a phytase (e.g. a 6-phytase (E.C.3.1.3.26) or a 3-phytase (E.C. 3.1.38)).

In one embodiment (particularly for feed applications) the other component may be a combination of an amylase (e.g. α-amylases (E.C. 3.2.1.1)) and a protease (e.g. subtilisin (E.C. 3.4.21.62)).

In one embodiment (particularly for feed applications) the other component may be a β-glucanase, e.g. an endo-1,3(4)-β-glucanases (E.C. 3.2.1.6).

In one embodiment (particularly for feed applications) the other component may be a phytase (e.g. a 6-phytase (E.C.3.1.3.26) or a 3-phytase (E.C. 3.1.38).

In one embodiment (particularly for feed applications) the other component may be a mannanases (e.g. a β-mannanase (E.C. 3.2.1.78)).

In one embodiment (particularly for feed applications) the other component may be a lipase lipase (E.C. 3.1.1.3), a lipid acyltransferase (generally classified as E.C. 2.3.1.x), or a phospholipase (E.C. 3.1.1.4, E.C. 3.1.1.32 or E.C. 3.1.1.5), suitably a lipase (E.C. 3.1.1.3).

In one embodiment (particularly for feed applications) the other component may be a protease (e.g. subtilisin (E.C. 3.4.21.62) or a bacillolysin (E.C. 3.4.24.28) or an alkaline serine protease (E.C. 3.4.21.x) or a keratinase (E.C. 3.4.x.x)).

In one embodiment the additional component may be a stabiliser or an emulsifier or a binder or carrier or an excipient or a diluent or a disintegrant.

The term "stabiliser" as used here is defined as an ingredient or combination of ingredients that keeps a product (e.g. a feed product) from changing over time.

The term "emulsifier" as used herein refers to an ingredient (e.g. a feed ingredient) that prevents the separation of emulsions. Emulsions are two immiscible substances, one present in droplet form, contained within the other. Emulsions can consist of oil-in-water, where the droplet or dispersed phase is oil and the continuous phase is water; or water-in-oil, where the water becomes the dispersed phase and the continuous phase is oil. Foams, which are gas-in-liquid, and suspensions, which are solid-in-liquid, can also be stabilised through the use of emulsifiers.

As used herein the term "binder" refers to an ingredient (e.g. a feed ingredient) that binds the product together through a physical or chemical reaction. During "gelation" for instance, water is absorbed, providing a binding effect. However, binders can absorb other liquids, such as oils, holding them within the product. In the context of the present invention binders would typically be used in solid or low-moisture products for instance baking products: pastries, doughnuts, bread and others. Examples of granulation binders include one or more of: polyvinylpyrrolidone, hydroxypropylmethylcellulose (HPMC), hydroxypropylcellulose (HPC), sucrose, maltose, gelatin and acacia.

"Carriers" mean materials suitable for administration of the enzyme and include any such material known in the art such as, for example, any liquid, gel, solvent, liquid diluent, solubilizer, or the like, which is non-toxic and which does not interact with any components of the composition in a deleterious manner.

The present invention provides a method for preparing a composition (e.g. a feed additive composition) comprising admixing an enzyme of the present invention with at least one physiologically acceptable carrier selected from at least one of maltodextrin, limestone (calcium carbonate), cyclodextrin, wheat or a wheat component, sucrose, starch, Na₂SO₄, Talc, PVA, sorbitol, benzoate, sorbiate, glycerol, sucrose, propylene glycol, 1,3-propane diol, glucose, parabens, sodium chloride, citrate, acetate, phosphate, calcium, metabisulfite, formate and mixtures thereof.

Examples of "excipients" include one or more of: microcrystalline cellulose and other celluloses, lactose, sodium citrate, calcium carbonate, dibasic calcium phosphate, glycine, starch, milk sugar and high molecular weight polyethylene glycols.

Examples of "disintegrants" include one or more of: starch (preferably corn, potato or tapioca starch), sodium starch glycollate, croscarmellose sodium and certain complex silicates.

Examples of "diluents" include one or more of: water, ethanol, propylene glycol and glycerin, and combinations thereof.

The other components may be used simultaneously (e.g. when they are in admixture together or even when they are delivered by different routes) or sequentially (e.g. they may be delivered by different routes) to the xylanase of the present invention.

Preferably, when the feed additive composition of the present invention is admixed with another component(s), the DFM remains viable.

In one embodiment preferably the feed additive composition according to the present invention does not comprise chromium or organic chromium
In one embodiment preferably the feed additive according to the present invention does not contain glucanase.

In one embodiment preferably the feed additive according to the present invention does not contain sorbic acid.

### ISOLATED

In one aspect, preferably the amino acid sequence, or nucleic acid, or enzyme according to the present invention is in an isolated form. The term "isolated" means that the sequence or enzyme or nucleic acid is at least substantially free from at least one other component with which the sequence, enzyme or nucleic acid is naturally associated in nature and as found in nature. The sequence, enzyme or nucleic acid of the present invention may be provided in a form that is substantially free of one or more contaminants with which the substance might otherwise be associated. Thus, for example it may be substantially free of one or more potentially contaminating polypeptides and/or nucleic acid molecules.

### PURIFIED

In one aspect, preferably the sequence, enzyme or nucleic acid according to the present invention is in a purified form. The term "purified" means that the given component is present at a high level. The component is desirably the predominant component present in a composition. Preferably, it is present at a level of at least about 90%, or at least about 95% or at least about 98%, said level being determined on a dry weight/dry weight basis with respect to the total composition under consideration.

### NUCLEOTIDE SEQUENCE

The scope of the present invention encompasses nucleotide sequences encoding proteins having the specific properties as defined herein.

The term "nucleotide sequence" as used herein refers to an oligonucleotide sequence or polynucleotide sequence, and variant, homologues, fragments and derivatives thereof (such as portions thereof). The nucleotide sequence may be of genomic or synthetic or recombinant origin, which may be double-stranded or single-stranded whether representing the sense or anti-sense strand.

The term "nucleotide sequence" in relation to the present invention includes genomic DNA, cDNA, synthetic DNA, and RNA. Preferably it means DNA, more preferably cDNA sequence coding for the present invention.

In one embodiment the term "nucleotide sequence" means cDNA.

In a preferred embodiment, the nucleotide sequence when relating to and when encompassed by the *per se* scope of the present invention does not include the native nucleotide sequence according to the present invention when in its natural environment and when it is linked to its naturally associated sequence(s) that is/are also in its/their natural environment. For ease of reference, we shall call this preferred embodiment the "non-native nucleotide sequence". In this regard, the term "native nucleotide sequence" means an entire nucleotide sequence that is in its native environment and when operatively linked to an entire promoter with which it is naturally associated, which promoter is also in its native environment. However, the amino acid sequence encompassed by scope the present invention can be isolated and/or purified post expression of a nucleotide sequence in its native organism. Preferably, however, the amino acid sequence encompassed by scope of the present invention may be expressed by a nucleotide sequence in its native organism but wherein the nucleotide sequence is not under the control of the promoter with which it is naturally associated within that organism.

Typically, the nucleotide sequence encompassed by the scope of the present invention is prepared using recombinant DNA techniques (i.e. recombinant DNA). However, in an alternative embodiment of the invention, the nucleotide sequence could be synthesised, in whole or in part, using chemical methods well known in the art (see Caruthers MH et al., (1980) Nuc Acids Res Symp Ser 215-23 and Horn T et al., (1980) Nuc Acids Res Symp Ser 225-232).

### PREPARATION OF THE NUCLEOTIDE SEQUENCE

A nucleotide sequence encoding either a protein which has the specific properties as defined herein or a protein which is suitable for modification may be identified and/or isolated and/or purified from any cell or organism producing said protein. Various methods are well known within the art for the identification and/or isolation and/or purification of nucleotide sequences. By way of example, PCR amplification techniques to prepare more of a sequence may be used once a suitable sequence has been identified and/or isolated and/or purified.

By way of further example, a genomic DNA and/or cDNA library may be constructed using chromosomal DNA or messenger RNA from the organism producing the enzyme. If the amino acid sequence of the enzyme is known, labelled oligonucleotide probes may be synthesised and used to identify enzyme-encoding clones from the genomic library prepared from the organism. Alternatively, a labelled oligonucleotide probe containing sequences homologous to another known enzyme gene could be used to identify enzyme-encoding clones. In the latter case, hybridisation and washing conditions of lower stringency are used.

Alternatively, enzyme-encoding clones could be identified by inserting fragments of genomic DNA into an expression vector, such as a plasmid, transforming enzyme-negative bacteria with the resulting genomic DNA library, and then plating the transformed bacteria onto agar plates containing a substrate for enzyme (i.e. arabinoxylan), thereby allowing clones expressing the enzyme to be identified.

In a yet further alternative, the nucleotide sequence encoding the enzyme may be prepared synthetically by established standard methods, e.g. the phosphoroamidite method described by Beucage S.L. et al., (1981) Tetrahedron Letters 22, p 1859-1869, or the method described by Matthes et al., (1984) EMBO J. 3, p 801-805. In the phosphoroamidite method, oligonucleotides are synthesised, e.g. in an automatic DNA synthesiser, purified, annealed, ligated and cloned in appropriate vectors.

The nucleotide sequence may be of mixed genomic and synthetic origin, mixed synthetic and cDNA origin, or mixed genomic and cDNA origin, prepared by ligating fragments of synthetic, genomic or cDNA origin (as appropriate) in accordance with standard techniques. Each ligated fragment corresponds to various parts of the entire nucleotide sequence. The DNA sequence may also be prepared by polymerase chain reaction (PCR) using specific primers, for instance as described in US 4,683,202 or in Saiki R K et al., (Science (1988) 239, pp 487-491).

### AMINO ACID SEQUENCES

The scope of the present invention also encompasses amino acid sequences of enzymes having the specific properties as defined herein.

As used herein, the term "amino acid sequence" is synonymous with the term "polypeptide" and/or the term "protein". In some instances, the term "amino acid sequence" is synonymous with the term "peptide". In some instances, the term "amino acid sequence" is synonymous with the term "enzyme".

The amino acid sequence may be prepared/isolated from a suitable source, or it may be made synthetically or it may be prepared by use of recombinant DNA techniques.

Preferably the amino acid sequence when relating to and when encompassed by the *per se* scope of the present invention is not a native enzyme. In this regard, the term "native enzyme" means an entire enzyme that is in its native environment and when it has been expressed by its native nucleotide sequence.

### SEQUENCE IDENTITY OR SEQUENCE HOMOLOGY

The present invention also encompasses the use of sequences having a degree of sequence identity or sequence homology with amino acid sequence(s) of a polypeptide having the specific properties defined herein or of any nucleotide sequence encoding such a polypeptide (hereinafter referred to as a "homologous sequence(s)"). Here, the term "homologue" means an entity having a certain homology with the subject amino acid sequences and the subject nucleotide sequences. Here, the term "homology" can be equated with "identity".

The homologous amino acid sequence and/or nucleotide sequence should provide and/or encode a polypeptide which retains the functional activity and/or enhances the activity of the enzyme.

In the present context, in some embodiments a homologous sequence is taken to include an amino acid or a nucleotide sequence which may be at least 97.7% identical, preferably at least 98 or 99% identical to the subject sequence.

In some embodiments a homologous sequence is taken to include an amino acid or a nucleotide sequence which may be at least 85% identical, preferably at least 90 or 95% identical to the subject sequence.

Typically, the homologues will comprise the same active sites etc. as the subject amino acid sequence for instance. Although homology can also be considered in terms of similarity (i.e. amino acid residues having similar chemical properties/functions), in the context of the present invention it is preferred to express homology in terms of sequence identity.

In one embodiment, a homologous sequence is taken to include an amino acid sequence or nucleotide sequence which has one or several additions, deletions and/or substitutions compared with the subject sequence.

In the present context, "the subject sequence" relates to the nucleotide sequence or polypeptide/amino acid sequence according to the invention.

Preferably, the % sequence identity with regard to a polypeptide sequence is determined using SEQ ID No. 1 as the subject sequence in a sequence alignment. In one embodiment, the polypeptide subject sequence is selected from the group consisting of SEQ ID No. 1, SEQ ID No. 26, SEQ ID No. 27, SEQ ID No. 3, SEQ ID No 28, SEQ ID No. 29, or SEQ ID No. 5.

Preferably, the % sequence identity with regard to a nucleotide sequence is determined using SEQ ID No. 2 as the subject sequence in the sequence alignment. In one embodiment, the subject sequence for nucleotide sequences may be selected from the group consisting of SEQ ID No. 2, SEQ ID No. 24, SEQ ID No. 25, SEQ ID No. 4, SEQ ID No. 30, SEQ ID No. 31, SEQ ID No. 6, SEQ ID No 32 or SEQ ID No. 33.

A "parent nucleic acid" or "parent amino acid" means a nucleic acid sequence or amino acid sequence, encoding or coding for the parent polypeptide, respectively.

In one embodiment the present invention relates to a protein whose amino acid sequence is represented herein or a protein derived from this (parent) protein by substitution, deletion or addition of one or several amino acids, such as 2, 3, 4, 5, 6, 7, 8, 9 amino acids, or more amino acids, such as 10 or more than 10 amino acids in the amino acid sequence of the parent protein and having the activity of the parent protein.

Suitably, the degree of identity with regard to an amino acid sequence is determined over at least 20 contiguous amino acids, preferably over at least 30 contiguous amino acids, preferably over at least 40 contiguous amino acids, preferably over at least 50 contiguous amino acids, preferably over at least 60 contiguous amino acids, preferably over at least 100 contiguous amino acids, preferably over at least 200 contiguous amino acids.

In one embodiment the present invention relates to a nucleic acid sequence (or gene) encoding a protein whose amino acid sequence is represented herein or encoding a protein derived from this (parent) protein by substitution, deletion or addition of one or several amino acids, such as 2, 3, 4, 5, 6, 7, 8, 9 amino acids, or more amino acids, such as 10 or more than 10 amino acids in the amino acid sequence of the parent protein and having the activity of the parent protein.

In the present context, in one embodiment a homologous sequence or foreign sequence is taken to include a nucleotide sequence which may be at least 97.7% identical, preferably at least 98 or 99% identical to a nucleotide sequence encoding a polypeptide of the present invention (the subject sequence).

In another embodiment, a homologous sequence is taken to include a nucleotide sequence which may be at least 85% identical, preferably at least 90 or 95% identical to a nucleotide sequence encoding a polypeptide of the present invention (the subject sequence).

Typically, the homologues will comprise the same sequences that code for the active sites etc. as the subject sequence. Although homology can also be considered in terms of similarity (i.e. amino acid residues having similar chemical properties/functions), in the context of the present invention it is preferred to express homology in terms of sequence identity.

Homology comparisons can be conducted by eye, or more usually, with the aid of readily available sequence comparison programs. These commercially available computer programs can calculate % homology or % identity between two or more sequences.

% homology or % identity may be calculated over contiguous sequences, i.e. one sequence is aligned with the other sequence and each amino acid in one sequence is directly compared with the corresponding amino acid in the other sequence, one residue at a time. This is called an "ungapped" alignment. Typically, such ungapped alignments are performed only over a relatively short number of residues.

Although this is a very simple and consistent method, it fails to take into consideration that, for example, in an otherwise identical pair of sequences, one insertion or deletion will cause the following amino acid residues to be put out of alignment, thus potentially resulting in a large reduction in % homology or % identity when a global alignment is performed. Consequently, most sequence comparison methods are designed to produce optimal alignments that take into consideration possible insertions and deletions without penalising unduly the overall homology score. This is achieved by inserting "gaps" in the sequence alignment to try to maximise local homology.

However, these more complex methods assign "gap penalties" to each gap that occurs in the alignment so that, for the same number of identical amino acids, a sequence alignment with as few gaps as possible - reflecting higher relatedness between the two compared sequences - will achieve a higher score than one with many gaps. "Affine gap costs" are typically used that charge a relatively high cost for the existence of a gap and a smaller penalty for each subsequent residue in the gap. This is the most commonly used gap scoring system. High gap penalties will of course produce optimised alignments with fewer gaps. Most alignment programs allow the gap penalties to be modified. However, it is preferred to use the default values when using such software for sequence comparisons.

Calculation of maximum % homology or % identity therefore firstly requires the production of an optimal alignment, taking into consideration gap penalties. A suitable computer program for carrying out such an alignment is the Vector NTI (Invitrogen Corp.). Examples of software that can perform sequence comparisons include, but are not limited to, the BLAST package (see Ausubel et al 1999 Short Protocols in Molecular Biology, 4th Ed - Chapter 18), BLAST 2 (see FEMS Microbiol Lett 1999 174(2): 247-50; FEMS Microbiol Lett 1999 177(1): 187-8 and tatiana@ncbi.nlm.nih.gov), FASTA (Altschul et al 1990 J. Mol. Biol. 403-410) and AlignX for example. At least BLAST, BLAST 2 and FASTA are available for offline and online searching (see Ausubel et al 1999, pages 7-58 to 7-60), such as for example in the GenomeQuest search tool (www.genomequest.com).

Although the final % homology or % identity can be measured in terms of identity, the alignment process itself is typically not based on an all-or-nothing pair comparison. Instead, a scaled similarity score matrix is generally used that assigns scores to each pairwise comparison based on chemical similarity or evolutionary distance. An example of such a matrix commonly used is the BLOSUM62 matrix - the default matrix for the BLAST suite of programs. Vector NTI programs generally use either the public default values or a custom symbol comparison table if supplied (see user manual for further details). For some applications, it is preferred to use the default values for the Vector NTI package.

Alternatively, percentage homologies may be calculated using the multiple alignment feature in Vector NTI (Invitrogen Corp.), based on an algorithm, analogous to CLUSTAL (Higgins DG & Sharp PM (1988), Gene 73(1), 237-244).

Once the software has produced an optimal alignment, it is possible to calculate % homology, preferably % sequence identity. The software typically does this as part of the sequence comparison and generates a numerical result.

Should Gap Penalties be used when determining sequence identity, then preferably the following parameters are used for pairwise alignment:

| FOR BLAST | |
|---|---|
| GAP OPEN | 9 |
| GAP EXTENSION | 2 |

| FOR CLUSTAL | DNA | PROTEIN |
|---|---|---|
| Weight Matrix | IUB | Gonnet 250 |
| GAP OPENING | 15 | 10 |
| GAP EXTEND | 6.66 | 0.1 |

In one embodiment, CLUSTAL may be used with the gap penalty and gap extension set as defined above.

Suitably, the degree of identity with regard to a nucleotide sequence or protein sequence is determined over at least 20 contiguous nucleotides/amino acids, preferably over at least 30 contiguous nucleotides/amino acids, preferably over at least 40 contiguous nucleotides/amino acids, preferably over at least 50 contiguous nucleotides/amino acids, preferably over at least 60 contiguous nucleotides/amino acids, preferably over at least 100 contiguous nucleotides/amino acids.

Suitably, the degree of identity with regard to a nucleotide sequence is determined over at least 100 contiguous nucleotides, preferably over at least 200 contiguous nucleotides, preferably over at least 300 contiguous nucleotides, preferably over at least 400 contiguous nucleotides, preferably over at least 500 contiguous nucleotides, preferably over at least 600 contiguous nucleotides, preferably over at least 700 contiguous nucleotides, preferably over at least 800 contiguous nucleotides .

Suitably, the degree of identity with regard to a nucleotide sequence may be determined over the whole sequence taught herein.

Suitably, the degree of identity with regard to a nucleotide sequence may be determined over the whole sequence taught herein as the mature sequence, e.g. SEQ ID No. 2 or SEQ ID No. 24 or SEQ ID No. 25 or SEQ ID No. 4, SEQ ID No. 30, SEQ ID No. 31, SEQ ID No. 6, SEQ ID No 32 or SEQ ID No. 33. Suitably, the degree of identity with regard to a nucleotide sequence may be determined over the whole sequence as taught herein as SEQ ID No. 2.

Suitably, the degree of identity with regard to a protein (amino acid) sequence is determined over at least 100 contiguous amino acids, preferably over at least 200 contiguous amino acids, preferably over at least 300 contiguous amino acids.

Suitably, the degree of identity with regard to an amino acid or protein sequence may be determined over the whole sequence taught herein.

Suitably, the degree of identity with regard to an amino acid or protein sequence may be determined over the whole sequence taught herein as the mature sequence, e.g. SEQ ID No. 1, SEQ ID No. 26, SEQ ID No. 27, SEQ ID No. 3, SEQ ID No. 28, SEQ ID No. 29, or SEQ ID No. 5. Suitably, the degree of identity with regard to an amino acid or protein sequence may be determined over the whole sequence taught herein as SEQ ID No. 1.

In the present context, the term "query sequence" means a homologous sequence or a foreign sequence, which is aligned with a subject sequence in order to see if it falls within the scope of the present invention. Accordingly, such query sequence can for example be a prior art sequence or a third party sequence.

In one preferred embodiment, the sequences are aligned by a global alignment program and the sequence identity is calculated by identifying the number of exact matches identified by the program divided by the length of the subject sequence.

In one embodiment, the degree of sequence identity between a query sequence and a subject sequence is determined by 1) aligning the two sequences by any suitable alignment program using the default scoring matrix and default gap penalty, 2) identifying the number of exact matches, where an exact match is where the alignment program has identified an identical amino acid or nucleotide in the two aligned sequences on a given position in the alignment and 3) dividing the number of exact matches with the length of the subject sequence.

In yet a further preferred embodiment, the global alignment program is selected from the group consisting of CLUSTAL and BLAST (preferably BLAST) and the sequence identity is calculated by identifying the number of exact matches identified by the program divided by the length of the subject sequence.

The sequences may also have deletions, insertions or substitutions of amino acid residues result in a functionally equivalent substance. Deliberate amino acid substitutions may be made on the basis of similarity in polarity, charge, solubility, hydrophobicity, hydrophilicity, and/or the amphipathic nature of the residues. For example, negatively charged amino acids include aspartic acid and glutamic acid; positively charged amino acids include lysine and arginine; and amino acids with uncharged polar head groups having similar hydrophilicity values include leucine, isoleucine, valine, glycine, alanine, asparagine, glutamine, serine, threonine, phenylalanine, and tyrosine.

Conservative substitutions may be made, for example according to the Table below. Amino acids in the same block in the second column and preferably in the same line in the third column may be substituted for each other:

| | | |
|---|---|---|
| ALIPHATIC | Non-polar | G A P |
| | | I L V |
| | Polar - uncharged | C S T M |
| | | N Q |
| | Polar - charged | D E |
| | | K R |
| AROMATIC | | H F W Y |

The present invention also encompasses homologous substitution (substitution and replacement are both used herein to mean the interchange of an existing amino acid residue, with an alternative residue) that may occur i.e. like-for-like substitution such as basic for basic, acidic for acidic, polar for polar etc. Non-homologous substitution may also occur i.e. from one class of residue to another or alternatively involving the inclusion of unnatural amino acids such as ornithine (hereinafter referred to as Z), diaminobutyric acid ornithine (hereinafter referred to as B), norleucine ornithine (hereinafter referred to as O), pyriylalanine, thienylalanine, naphthylalanine and phenylglycine.

Replacements may also be made by unnatural amino acids include; alpha* and alpha-disubstituted* amino acids, N-alkyl amino acids*, lactic acid*, halide derivatives of natural amino acids such as trifluorotyrosine*, p-Cl-phenylalanine*, p-Br-phenylalanine*, p-I-phenylalanine*, L-allyl-glycine*, β-alanine*, L-α-amino butyric acid*, L-γ-amino butyric acid*, L-α-amino isobutyric acid*, L-ε-amino caproic acid^{#}, 7-amino heptanoic acid*, L-methionine sulfone^{#*}, L-norleucine*, L-norvaline*, p-nitro-L-phenylalanine*, L-hydroxyproline^{#}, L-thioproline*, methyl derivatives of phenylalanine (Phe) such as 4-methyl-Phe*, pentamethyl-Phe*, L-Phe (4-amino)^{#}, L-Tyr (methyl)*, L-Phe (4-isopropyl)*, L-Tic (1,2,3,4-tetrahydroisoquinoline-3-carboxyl acid)*, L-diaminopropionic acid^{#} and L-Phe (4-benzyl)*. The notation * has been utilised for the purpose of the discussion above (relating to homologous or non-homologous substitution), to indicate the hydrophobic nature of the derivative whereas # has been utilised to indicate the hydrophilic nature of the derivative, #* indicates amphipathic characteristics.

Variant amino acid sequences may include suitable spacer groups that may be inserted between any two amino acid residues of the sequence including alkyl groups such as methyl, ethyl or propyl groups in addition to amino acid spacers such as glycine or β-alanine residues. A further form of variation, involves the presence of one or more amino acid residues in peptoid form, will be well understood by those skilled in the art. For the avoidance of doubt, "the peptoid form" is used to refer to variant amino acid residues wherein the α-carbon substituent group is on the residue's nitrogen atom rather than the α-carbon. Processes for preparing peptides in the peptoid form are known in the art, for example Simon RJ et al., PNAS (1992) 89(20), 9367-9371 and Horwell DC, Trends Biotechnol. (1995) 13(4), 132-134.

In one embodiment the xylanase for use in the present invention may comprise a polypeptide sequence shown as SEQ ID No. 17, SEQ ID No. 18, SEQ ID No. 19, SEQ ID No. 20, or SEQ ID No. 21 with a conservative substitution of at least one of the amino acids.

Suitably there may be at least 2 conservative substitutions, such as at least 3 or at least 4 or at least 5.

Suitably there may be less than 15 conservative substitutions, such as less than 12, less than 10, or less than 8 or less than 5.

The nucleotide sequences for use in the present invention may include within them synthetic or modified nucleotides. A number of different types of modification to oligonucleotides are known in the art. These include methylphosphonate and phosphorothioate backbones and/or the addition of acridine or polylysine chains at the 3' and/or 5' ends of the molecule. For the purposes of the present invention, it is to be understood that the nucleotide sequences described herein may be modified by any method available in the art. Such modifications may be carried out in order to enhance the *in vivo* activity or life span of nucleotide sequences of the present invention.

The present invention also encompasses the use of nucleotide sequences that are complementary to the sequences presented herein, or any derivative, fragment or derivative thereof. If the sequence is complementary to a fragment thereof then that sequence can be used as a probe to identify similar coding sequences in other organisms etc.

Polynucleotides which are not 100% homologous to the sequences of the present invention but fall within the scope of the invention can be obtained in a number of ways. Other variants of the sequences described herein may be obtained for example by probing DNA libraries made from a range of individuals, for example individuals from different populations. In addition, other homologues may be obtained and such homologues and fragments thereof in general will be capable of selectively hybridising to the sequences shown in the sequence listing herein. Such sequences may be obtained by probing cDNA libraries made from or genomic DNA libraries from other animal species, and probing such libraries with probes comprising all or part of any one of the sequences in the attached sequence listings under conditions of medium to high stringency. Similar considerations apply to obtaining species homologues and allelic variants of the polypeptide or nucleotide sequences of the invention.

Variants and strain/species homologues may also be obtained using degenerate PCR which will use primers designed to target sequences within the variants and homologues encoding conserved amino acid sequences within the sequences of the present invention. Conserved sequences can be predicted, for example, by aligning the amino acid sequences from several variants/homologues. Sequence alignments can be performed using computer software known in the art. For example the GCG Wisconsin PileUp program is widely used.

The primers used in degenerate PCR will contain one or more degenerate positions and will be used at stringency conditions lower than those used for cloning sequences with single sequence primers against known sequences.

Alternatively, such polynucleotides may be obtained by site directed mutagenesis of characterised sequences. This may be useful where for example silent codon sequence changes are required to optimise codon preferences for a particular host cell in which the polynucleotide sequences are being expressed. Other sequence changes may be desired in order to introduce restriction enzyme recognition sites, or to alter the property or function of the polypeptides encoded by the polynucleotides.

Polynucleotides (nucleotide sequences) of the invention may be used to produce a primer, e.g. a PCR primer, a primer for an alternative amplification reaction, a probe e.g. labelled with a revealing label by conventional means using radioactive or non-radioactive labels, or the polynucleotides may be cloned into vectors. Such primers, probes and other fragments will be at least 15, preferably at least 20, for example at least 25, 30 or 40 nucleotides in length, and are also encompassed by the term polynucleotides of the invention as used herein.

Polynucleotides such as DNA polynucleotides and probes according to the invention may be produced recombinantly, synthetically, or by any means available to those of skill in the art. They may also be cloned by standard techniques.

In general, primers will be produced by synthetic means, involving a stepwise manufacture of the desired nucleic acid sequence one nucleotide at a time. Techniques for accomplishing this using automated techniques are readily available in the art.

Longer polynucleotides will generally be produced using recombinant means, for example using a PCR (polymerase chain reaction) cloning techniques. The primers may be designed to contain suitable restriction enzyme recognition sites so that the amplified DNA can be cloned into a suitable cloning vector.

### AMINO ACID NUMBERING

In the present invention, a specific numbering of amino acid residue positions in the xylanases used in the present invention may be employed. By alignment of the amino acid sequence of a sample xylanases with the xylanase of the present invention (particularly SEQ ID No. 1) it is possible to allot a number to an amino acid residue position in said sample xylanase which corresponds with the amino acid residue position or numbering of the amino acid sequence shown in SEQ ID No. 1 of the present invention.

### HYBRIDISATION

The present invention also encompasses sequences that are complementary to the nucleic acid sequences of the present invention or sequences that are capable of hybridising either to the sequences of the present invention or to sequences that are complementary thereto.

The term "hybridisation" as used herein shall include "the process by which a strand of nucleic acid joins with a complementary strand through base pairing" as well as the process of amplification as carried out in polymerase chain reaction (PCR) technologies.

The present invention also encompasses the use of nucleotide sequences that are capable of hybridising to the sequences that are complementary to the sequences presented herein, or any fragment or derivative thereof.

The term "variant" also encompasses sequences that are complementary to sequences that are capable of hybridising to the nucleotide sequences presented herein.

Preferably, the term "variant" encompasses sequences that are complementary to sequences that are capable of hybridising under stringent conditions (e.g. 50°C and 0.2xSSC {1xSSC = 0.15 M NaCl, 0.015 M Na₃citrate pH 7.0}) to the nucleotide sequences presented herein.

More preferably, the term "variant" encompasses sequences that are complementary to sequences that are capable of hybridising under high stringency conditions (e.g. 65°C and 0.1xSSC {1xSSC = 0.15 M NaCl, 0.015 M Na₃citrate pH 7.0}) to the nucleotide sequences presented herein.

The present invention also relates to nucleotide sequences that can hybridise to the nucleotide sequences of the present invention (including complementary sequences of those presented herein).

The present invention also relates to nucleotide sequences that are complementary to sequences that can hybridise to the nucleotide sequences of the present invention (including complementary sequences of those presented herein).

Preferably hybridisation is analysed over the whole of the sequences taught herein.

### EXPRESSION OF ENZYMES

The nucleotide sequence for use in the present invention may be incorporated into a recombinant replicable vector. The vector may be used to replicate and express the nucleotide sequence, in protein/enzyme form, in and/or from a compatible host cell.

Expression may be controlled using control sequences e.g. regulatory sequences.

The protein produced by a host recombinant cell by expression of the nucleotide sequence may be secreted or may be contained intracellularly depending on the sequence and/or the vector used. The coding sequences may be designed with signal sequences which direct secretion of the substance coding sequences through a particular prokaryotic or eukaryotic cell membrane.

### HOST CELLS FOR PARENT AND MODIFIED XYLANASE ENZYMES AND CULTURE CONDITIONS FOR ENZYME PRODUCTION

After DNA sequences that encode the parent or modified xylanase enzymes have been cloned into DNA constructs, the DNA is used to transform microorganisms. The microorganism to be transformed for the purpose of expressing a modified xylanase according to the present invention can be chosen from a wide variety of host cells. The sections below are provided as examples of host cells/microorganisms and are not meant to limit the scope of host cells that can be employed in practicing aspects of the present invention.

### (i) Filamentous Fungi

Aspects of the present invention include filamentous fungi which have been modified, selected and cultured in a manner effective to result in desired modified xylanase production or expression relative to the corresponding non-transformed parental filamentous fungi.

Examples of species of parental filamentous fungi that may be treated and/or modified for desired xylanase expression include, but are not limited to *Trichoderma, Penicillium sp., Humicola sp.,* including *Humicola insolens; Aspergillus sp.,* including *Aspergillus niger, Chrysosporium sp., Myceliophthora sp., Fusarium sp., Hypocrea sp.,* and *Emericella sp.*

Cells expressing a desired modified xylanase are cultured under conditions typically employed to culture the parental fungal line. Generally, cells are cultured in a standard medium containing physiological salts and nutrients, such as described in Pourquie, J. et al., Biochemistry and Genetics of Cellulose Degradation, eds. Aubert, J. P. et al., Academic Press, pp. 71-86, 1988 and Ilmen, M. et al., Appl. Environ. Microbiol. 63:1298-1306, 1997. Standard culture conditions are known in the art, e.g., cultures are incubated at 28°C in shaker cultures or fermenters until desired levels of desired modified xylanase expression are achieved.

Culture conditions for a given filamentous fungus can be found, for example, in the scientific literature and/or from the source of the fungi such as the American Type Culture Collection (ATCC). After fungal growth has been established, the cells are exposed to conditions effective to cause or permit the expression of a desired modified xylanase.

In cases where a desired parent or modified xylanase coding sequence is under the control of an inducible promoter, the inducing agent, e.g., a sugar, metal salt or antibiotic, is added to the medium at a concentration effective to induce expression of the desired modified xylanase.

In one embodiment, the strain is an *Aspergillus niger* strain, which is a useful strain for obtaining overexpressed protein. For example *A. niger var awamori* dgr246 is known to secrete elevated amounts of secreted cellulases (Goedegebuur et al, Curr. Genet (2002) 41: 89-98). Other strains of *Aspergillus niger var awamori* such as GCDAP3, GCDAP4 and GAP3-4 are known (Ward et al, 1993, Appl. Microbiol. Biotechnol. 39:738-743).

In another embodiment, the strain is a *Trichoderma reesei* strain, which is a useful strain for obtaining overexpressed protein. For example, RL-P37, described by Sheir-Neiss, et al., Appl. Microbiol. Biotechnol. 20:46-53 (1984) is known to secrete elevated amounts of cellulase enzymes. Functional equivalents of RL-P37 include *Trichoderma reesei* strain RUT-C30 (ATCC No. 56765) and strain QM9414 (ATCC No. 26921). It is contemplated that these strains would also be useful in over-expressing variant GA.

Where it is desired to obtain a parent or modified xylanase in the absence of potentially detrimental native xylanase activity, it is useful to obtain a host cell strain which has had one or more glucoamylase genes deleted prior to introduction of a DNA construct or plasmid containing the DNA fragment encoding the desired GA variant. Such strains may be prepared in any convenient manner, for example by the method disclosed in U.S. Patent No. 5,246,853 and WO 92/06209. By expressing a desired modified xylanase in a host microorganism that is missing one or more xylanase genes (e.g., the endogenous xylanase gene of a host cell), identification and subsequent purification procedures, where desired, are simplified.

Gene deletion may be accomplished by inserting a form of the desired gene to be deleted or disrupted into a plasmid by methods known in the art. The deletion plasmid is then cut at an appropriate restriction enzyme site(s), internal to the desired gene coding region, and the gene coding sequence or part thereof replaced with a selectable marker. Flanking DNA sequences from the locus of the gene to be deleted or disrupted, for example from about 0.5 to about 2.0 kb, may remain on either side of the selectable marker gene. An appropriate deletion plasmid will generally have unique restriction enzyme sites present therein to enable the fragment containing the deleted gene, including flanking DNA sequences, and the selectable marker gene to be removed as a single linear piece.

In certain embodiments, more than one copy of DNA encoding a desired parent or modified xylanase may be present in a host strain to facilitate overexpression of the modified xylanase. For example, a host cell may have multiple copies of a desired parent or modified xylanase integrated into the genome or, alternatively, include a plasmid vector that is capable of replicating autonomously in the host organism.

### (ii) Yeast

The present invention also contemplates the use of yeast as a host cell for desired xylanase enzyme production. Several other genes encoding hydrolytic enzymes have been expressed in various strains of the yeast S. *cerevisiae.* These include sequences encoding for two endoglucanases (Penttila et al., 1987), two cellobiohydrolases (Penttila et al., 1988) and one beta-glucosidase from *Trichoderma reesei* (Cummings and Fowler, 1996), a xylanase from *Aureobasidlium pullulans* (Li and Ljungdahl, 1996), an alpha-amylase from wheat (Rothstein et al., 1987), etc.

### (iii) Other

It is further contemplated that in some embodiments, expression systems in host cells other than filamentous fungal cells or yeast cells may be employed, including, plant, algae, insect cell or bacterial cell expression systems. Certain of the bacterial host cells can, for example, be one that is also an ethanologen, such as an engineered Zymomonas moblis, which is not only capable of expressing the enzyme(s)/variant(s) of interest but also capable of metabolizing certain monomeric and other fermentable sugars, turning them into ethanol. The selection of a host cell may be determined by the desires of the user of the modified xylanase described herein, and thus no limitation in that regard is intended.

### UTILITY OF MODIFIED XYLANASE ENZYMES IN FERMENTATION BY-PRODUCT RECOVERY

As detailed above, xylanases are very important commercial enzymes used in a wide variety of applications involving fermentation processes that generate valuable by-products, e.g., oil. Some fermentation processes are designed to produce high fructose corn sweeteners, which comprise over 50% of the sweetener market in the United States, as well as in processes for the direct production of glucose. In general, glucoamylases may be, and commonly are, used with alpha-amylases in starch hydrolyzing processes to hydrolyze starch to dextrins and then to glucose.

Given the commercial importance of fermentation by-products, it can be appreciated that the desired parent or mofidied xylanase-encoding nucleic acids, the desired parent or modified xylanase polypeptides and compositions comprising the same find utility in a wide variety applications. The improved property or properties of the parent or modified xylanase described herein can be exploited in many ways. For example, parent or modified xylanase with improved performance in oil recovery, under conditions of thermal stress or reduced viscosity can be used to increase by-product recovery (e.g., oil recovery). Other improved properties of parent or modified xylanase polypeptides can be exploited, including GA variants having altered pH optima, increased stability or activity at a specific pH, increased specific activity for a substrate, and/or high level expression in a host cell of interest.

A grain-based material, e.g., starch, processing accessory enzyme containing a desired parent or modified xylanase as described herein finds use in ethanol production. Ethanol from this process can be further used as an octane enhancer or directly as a fuel in lieu of gasoline, which is advantageous, because ethanol as a fuel source is more environmentally friendly than petroleum derived products. It is known that the use of ethanol will improve air quality and possibly reduce local ozone levels and smog. Moreover, utilization of ethanol in lieu of gasoline can be of strategic importance in buffering the impact of sudden shifts in non-renewable energy and petro-chemical supplies.

Separate saccharification and fermentation is a process whereby starch present in a feedstock, e.g., corn, is converted to glucose and subsequently an ethanologen (e.g., a yeast strain) convert the glucose into ethanol. Simultaneous saccharification and fermentation (SSF) is a process whereby starch present in a feedstock is converted to glucose and, at the same time and in the same reactor, an ethanologen converts the glucose into ethanol. Thus, the parent or modified xylanase of the invention find use for improving recovery of fermentation by-products, e.g., oi recovery, in both of these processes involving the degradation of starch-containing feedstock to generate ethanol.

In some embodiments, a parent or modified xylanase as described herein is expressed in an ethanologen whereby the enzymatic activity of the parent or modified xylanase expressed by the ethanologen can improve by-product recovery amounts. This may be achieved either with our without addition of exogenous modified xylanase.

Modified xylanase as described herein find use in generating host cells for producing biochemical products of interest. As such, aspects of the present disclosure include methods of producing a biochemical by obtaining a host cell expressing a modified xylanase as described herein and culturing the host cell under conditions to produce the biochemical of interest. The host cell may include additional modifications to promote production of the desired biochemical, e.g., to express homologous or heterologous genes, additional variant genes, and/or to delete or otherwise inactivate the expression of one or more endogenous genes. Biochemicals of interest include, but are not limited to alcohols (ethanol, methanol, butanol, etc.) and other organic compounds, including volatile organic molecules (e.g., isoprene).

It is noted that parent or modified xylanases facilitating improved by-product recovery find use, for example, in areas where the enzyme activity is required to be neutralized at lower temperatures, so that other enzymes that may be present are left unaffected. In addition, the enzymes may find utility in the limited conversion of cellulosics, for example, in combination with glucoamylase in controlling the degree of crystallinity or of cellulosic chain-length. After reaching the desired extent of conversion, the saccharifying temperature can be raised above the survival temperature of the de-stabilized modified xylanase (and glucoamylase).

### EXPRESSION VECTOR

The term "expression vector" means a construct capable of *in vivo* or *in vitro* expression.

Preferably, the expression vector is incorporated into the genome of a suitable host organism. The term "incorporated" preferably covers stable incorporation into the genome.

The nucleotide sequence of the present invention may be present in a vector in which the nucleotide sequence is operably linked to regulatory sequences capable of providing for the expression of the nucleotide sequence by a suitable host organism.

The vectors for use in the present invention may be transformed into a suitable host cell as described below to provide for expression of a polypeptide of the present invention.

The choice of vector e.g. a plasmid, cosmid, or phage vector will often depend on the host cell into which it is to be introduced.

The vectors for use in the present invention may contain one or more selectable marker genes-such as a gene, which confers antibiotic resistance e.g. ampicillin, kanamycin, chloramphenicol or tetracyclin resistance. Alternatively, the selection may be accomplished by co-transformation (as described in WO91/17243).

Vectors may be used *in vitro,* for example for the production of RNA or used to transfect, transform, transduce or infect a host cell.

Thus, in a further embodiment, the invention provides a method of making nucleotide sequences of the present invention by introducing a nucleotide sequence of the present invention into a replicable vector, introducing the vector into a compatible host cell, and growing the host cell under conditions which bring about replication of the vector.

The vector may further comprise a nucleotide sequence enabling the vector to replicate in the host cell in question. Examples of such sequences are the origins of replication of plasmids pUC19, pACYC177, pUB110, pE194, pAMB1 and pIJ702.

### REGULATORY SEQUENCES

In some applications, the nucleotide sequence for use in the present invention is operably linked to a regulatory sequence which is capable of providing for the expression of the nucleotide sequence, such as by the chosen host cell. By way of example, the present invention covers a vector comprising the nucleotide sequence of the present invention operably linked to such a regulatory sequence, i.e. the vector is an expression vector.

The term "operably linked" refers to a juxtaposition wherein the components described are in a relationship permitting them to function in their intended manner. A regulatory sequence "operably linked" to a coding sequence is ligated in such a way that expression of the coding sequence is achieved under condition compatible with the control sequences.

The term "regulatory sequences" includes promoters and enhancers and other expression regulation signals.

The term "promoter" is used in the normal sense of the art, e.g. an RNA polymerase binding site.

Enhanced expression of the nucleotide sequence encoding the enzyme of the present invention may also be achieved by the selection of heterologous regulatory regions, e.g. promoter, secretion leader and terminator regions.

Preferably, the nucleotide sequence according to the present invention is operably linked to at least a promoter.

Other promoters may even be used to direct expression of the polypeptide of the present invention.

Examples of suitable promoters for directing the transcription of the nucleotide sequence in a bacterial, fungal or yeast host are well known in the art.

The promoter can additionally include features to ensure or to increase expression in a suitable host. For example, the features can be conserved regions such as a Pribnow Box or a TATA box.

### CONSTRUCTS

The term "construct" - which is synonymous with terms such as "conjugate", "cassette" and "hybrid" - includes a nucleotide sequence for use according to the present invention directly or indirectly attached to a promoter.

An example of an indirect attachment is the provision of a suitable spacer group such as an intron sequence, such as the Sh1-intron or the ADH intron, intermediate the promoter and the nucleotide sequence of the present invention. The same is true for the term "fused" in relation to the present invention which includes direct or indirect attachment. In some cases, the terms do not cover the natural combination of the nucleotide sequence coding for the protein ordinarily associated with the wild type gene promoter and when they are both in their natural environment.

The construct may even contain or express a marker, which allows for the selection of the genetic construct.

For some applications, preferably the construct of the present invention comprises at least the nucleotide sequence of the present invention operably linked to a promoter.

### HOST CELLS

The term "host cell" - in relation to the present invention includes any cell that comprises either the nucleotide sequence or an expression vector as described above and which is used in the recombinant production of a protein having the specific properties as defined herein.

In one embodiment the organism is an expression host.

Thus, a further embodiment of the present invention provides host cells transformed or transfected with a nucleotide sequence that expresses the protein of the present invention. The cells will be chosen to be compatible with the said vector and may for example be prokaryotic (for example bacterial), fungal or yeast cells.

Examples of suitable bacterial host organisms are gram positive or gram negative bacterial species.

In one embodiment the xylanases taught herein are expressed in the expression host *Trichoderma reesei.*

In some embodiments the expression host for the xylanases taught herein may be one or more of the following fungal expression hosts: *Fusarium* spp. (such as *Fusarium oxysporum*); *Aspergillus* spp. (such as *Aspergillus niger, A. oryzae, A. nidulans,* or *A. awamori*) or *Trichoderma* spp. (such as *T. reesei).*

In some embodiments the expression host may be one or more of the following bacterial expression hosts: *Streptomyces* spp. or *Bacillus* spp. (e.g. *Bacillus subtilis* or B. *licheniformis*).

The use of suitable host cells - such as yeast and fungal host cells - may provide for post-translational modifications (e.g. myristoylation, glycosylation, truncation, lipidation and tyrosine, serine or threonine phosphorylation) as may be needed to confer optimal biological activity on recombinant expression products of the present invention.

### ORGANISM

The term "organism" in relation to the present invention includes any organism that could comprise the nucleotide sequence coding for the polypeptide according to the present invention and/or products obtained therefrom, and/or wherein a promoter can allow expression of the nucleotide sequence according to the present invention when present in the organism.

In one embodiment the organism is an expression host.

Suitable organisms may include a prokaryote, fungus, yeast or a plant.

The term "transgenic organism" in relation to the present invention includes any organism that comprises the nucleotide sequence coding for the polypeptide according to the present invention and/or the products obtained therefrom, and/or wherein a promoter can allow expression of the nucleotide sequence according to the present invention within the organism. Preferably the nucleotide sequence is incorporated in the genome of the organism.

The term "transgenic organism" does not cover native nucleotide coding sequences in their natural environment when they are under the control of their native promoter which is also in its natural environment.

Therefore, the transgenic organism of the present invention includes an organism comprising any one of, or combinations of, the nucleotide sequence coding for the polypeptide according to the present invention, constructs according to the present invention, vectors according to the present invention, plasmids according to the present invention, cells according to the present invention, tissues according to the present invention, or the products thereof.

For example the transgenic organism may also comprise the nucleotide sequence coding for the polypeptide of the present invention under the control of a heterologous promoter.

### TRANSFORMATION OF HOST CELLS/ORGANISM

As indicated earlier, the host organism can be a prokaryotic or a eukaryotic organism. Examples of suitable prokaryotic hosts include E. *coli, Streptomyces* spp. and *Bacillus spp., e.g. Bacillus subtilis.*

Teachings on the transformation of prokaryotic hosts is well documented in the art, for example see Sambrook et al (Molecular Cloning: A Laboratory Manual, 2nd edition, 1989, Cold Spring Harbor Laboratory Press). If a prokaryotic host is used then the nucleotide sequence may need to be suitably modified before transformation - such as by removal of introns.

Filamentous fungi cells may be transformed using various methods known in the art - such as a process involving protoplast formation and transformation of the protoplasts followed by regeneration of the cell wall in a manner known. The use of *Aspergillus* as a host microorganism is described in EP 0 238 023.

Transformation of prokaryotes, fungi and yeasts are generally well known to one skilled in the art.

A host organism may be a fungus - such as a mould. Examples of suitable such hosts include any member belonging to the genera *Trichoderma* (e.g. T. reesei), *Thermomyces, Acremonium, Fusarium, Aspergillus, Penicillium, Mucor, Neurospora* and the like.

In one embodiment, the host organism may be a fungus. In one preferred embodiment the host organism belongs to the genus *Trichoderma, e.g. T. reesei*).

### CULTURING AND PRODUCTION

Host cells transformed with the nucleotide sequence of the present invention may be cultured under conditions conducive to the production of the encoded polypeptide and which facilitate recovery of the polypeptide from the cells and/or culture medium.

The medium used to cultivate the cells may be any conventional medium suitable for growing the host cell in questions and obtaining expression of the polypeptide.

The protein produced by a recombinant cell may be displayed on the surface of the cell.

The protein may be secreted from the host cells and may conveniently be recovered from the culture medium using well-known procedures.

### SECRETION

Often, it is desirable for the protein to be secreted from the expression host into the culture medium from where the protein may be more easily recovered. According to the present invention, the secretion leader sequence may be selected on the basis of the desired expression host. Hybrid signal sequences may also be used with the context of the present invention.

### LARGE SCALE APPLICATION

In one preferred embodiment of the present invention, the amino acid sequence is used for large scale applications.

Preferably the amino acid sequence is produced in a quantity of from 1g per litre to about 100g per litre of the total cell culture volume after cultivation of the host organism.

Suitably the amino acid sequence may be produced in a quantity of from 30g per litre to about 90g per litre of the total cell culture volume after cultivation of the host organism.

### GENERAL RECOMBINANT DNA METHODOLOGY TECHNIQUES

The present invention employs, unless otherwise indicated, conventional techniques of chemistry, molecular biology, microbiology, recombinant DNA and immunology, which are within the capabilities of a person of ordinary skill in the art. Such techniques are explained in the literature. See, for example, J. Sambrook, E. F. Fritsch, and T. Maniatis, 1989, Molecular Cloning: A Laboratory Manual, Second Edition, Books 1-3, Cold Spring Harbor Laboratory Press; Ausubel, F. M. et al. (1995 and periodic supplements; Current Protocols in Molecular Biology, ch. 9, 13, and 16, John Wiley & Sons, New York, N.Y.); B. Roe, J. Crabtree, and A. Kahn, 1996, DNA Isolation and Sequencing: Essential Techniques, John Wiley & Sons; M. J. Gait (Editor), 1984, Oligonucleotide Synthesis: A Practical Approach, Irl Press; and, D. M. J. Lilley and J. E. Dahlberg, 1992, Methods of Enzymology: DNA Structure Part A: Synthesis and Physical Analysis of DNA Methods in Enzymology, Academic Press.

The invention will now be described, by way of example only, with reference to the following Figures and Examples.

### EXAMPLES

### EXAMPLE 1

### Materials and methods

### Plasmid and library construction

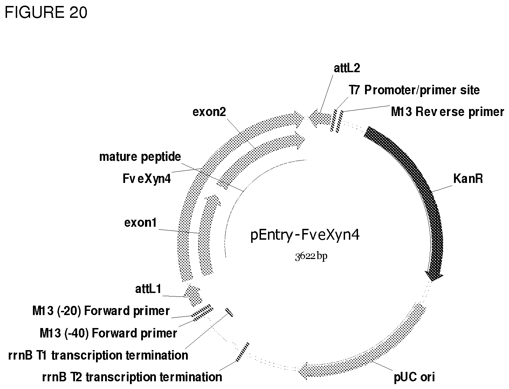

A DNA sequence containing the coding region for xylanase 4 (the family GH10) from the filamentous fungus *Fusarium verticilloides,* FveXyn4, was amplified from the genomic DNA with the gene specific primers extended with the attB1 and attB2 sites to allow for the Gateway® BP recombination cloning into the pDonor221 vector (Invitrogen, USA). The pEntry-FveXyn4 plasmid, as shown in Figure 20 was used by the vendors BaseClear (Netherlands) and Geneart GmH (Germany) as template for construction of combinatorial libraries.

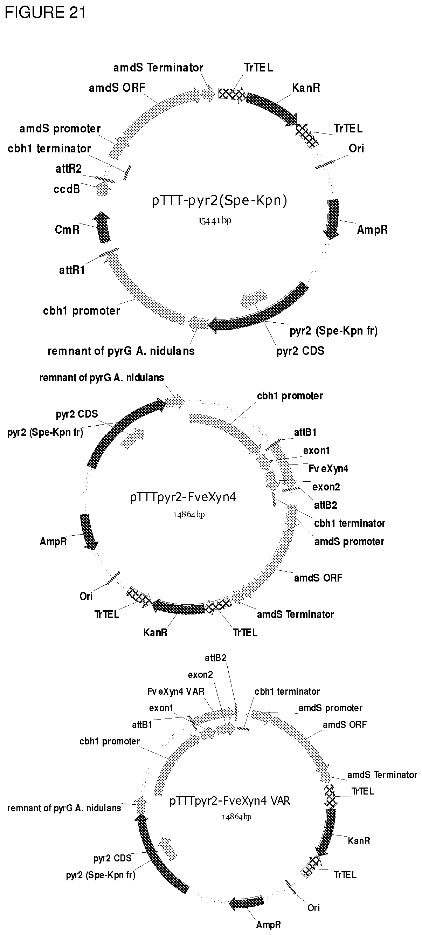

Variants of FveXyn4 was generated either as combinatorial libraries or by introduction of specific mutations and were designed to included different numbers and combinations of the mutations presented in Table 1. Variant A, B, C, D, and E were included in these variants. Combinatorial variants were generated via the Gateway® recombination technique (Invitrogen, USA) with the destination vector pTTTpyr2 (Figure 21). The resulting expression plasmids pTTTpyr2-FveXyn4_VAR expressing Xyn4 with different mutations were amplified in the *Escherichia coli* DH5a strain, purified, sequenced, arrayed individually in 96 MTPs and used for fungal transformation as described further. The expression vector contains the *T. reesei cbhl* promoter and terminator regions allowing for a strong inducible expression of a gene of interest, the *Aspergillus nidulans amdS* and *T. reesei pyr2* selective markers conferring growth of transformants on minimal medium with acetamide in the absence of uridine. The plasmids are maintained autonomously in the fungal cell due to *T. reesei* derived telomere regions. Usage of replicative plasmids results in increased frequencies of transformation and circumvents problems of locus-dependent expression observed with integrative fungal transformation. Specific mutations were introduced into the genomic sequence of the *Fusarium verticilloides* xylanase Xyn4 via a *de novo* gene synthesis (GeneArt GmbH, Germany). Synthetic variants were then cloned by the vendor into the destination vector pTTT-pyr2 via a Gateway recombination technique (Invitrogen, Carlsbad, CA, USA).

### Fungal strains, growth media and transformation

Expression plasmids (5-10 ul) were transformed using a PEG-protoplast method into a *T*. *reesei* strain deleted for major cellulases and xylanase 2 (*Δcbh1 Δcbh2 Δegl1 Δegl2 Δegl3 Δegl4 Δegl5 Δegl6 Δbgl1 Δman1 Δxyn2 Prdiv: iRNAxyn1 xyn3: amdS pyr2-).* Additional downregulation of the endogenous xylanase 1 and 3 background was further achieved via introducing into the host strain genome an iRNA interference cassette targeting to shut down the *xyn1* and *xyn3* expression simultaneously. All high throughput transformations were performed robotically in a 24 well MTP format using Biomek robots (Beckman Coulter, USA). Plasmids with variants were received from the vendors in 96 well MTPs arrayed according to a predetermined layout. Transformation mixtures containing approximately 1 µg of DNA and 5x 10⁶ protoplasts in a total volume of 50 µl were treated with 200 ul of 25% PEG solution, diluted with 1 volumes of 1.2M sorbitol/10mM Tris, pH7.5/ 10mM CaCl₂ solution, rearranged robotically into 24 well MTPs and mixed with 1 ml of 3% agarose Minimal Medium containing 1M sorbitol and 10 mM NH4Cl. After growth of transformants, spores from each well were pooled and repatched on fresh 24 well MTPs with MM containing acetamide for additional selective pressure. Once sporulated, spores were harvested and used for inoculation of liquid cultures either in a 24-well MTP format or shake flasks in the following production medium: 37 g/L glucose, 1 g/L sophorose, 9 g/L casmino acids, 10 g/L (NH₄)₂SO₄, 5 g/L KH₂PO₄, 1 g/L CaCl₂x2H₂O, 1 g/L MgSO₄x7H₂O, 33 g/L 1,4-Piperazinebis(propanesulfonic acid), pH 5.5, 2.5 ml/L of 400X *T. reesei* trace elements (175 g/L citric acid, 200 g/L FeSO4x7H₂O, 16 g/L ZnSO4x7H₂O, 3.2 g/L CuSO4x5H₂O, 1.4 g/L MnSO4xH₂O, 0.8 g/L boric acid). 1 ml of production medium was added to produce variants in 24 well MTPs. For shake flasks, volumes were scaled up.

Plates were grown for 6 days at 28°C and 80% humidity with shaking at 200 rpm. Culture supernatants were harvested by vacuum filtration and used to assay their performance as well as expression level.

For larger scale production fermentation in a 6 Liter autoclaveable Continuers Stirred Reactor was conducted. Shake flasks were inoculated with spores and incubated with shaking for 3 days at 28°C in the following shake flask medium: 5 g/L (NH₄)₂SO₄, 4.5 g/L KH₂PO₄, 1 g/L MgSO₄x7H₂O, 14.4 g/L citric acid x1H₂O, 1 g/L CaCl₂x2H₂O, 27.5 g/L glucose, 1 drop antifoam agent (EROL DF 6000K). The pH was adjusted with NaOH (2M) to 5.5 and media was autoclaved 20 minutes at 122°C. After cooling 2.5 ml/L of 400X T. reesei trace elements (175 g/L citric acid, 200 g/L FeSO4x7H₂O, 16 g/L ZnSO4x7H₂O, 3.2 g/L CuSO4x5H₂O, 1.4 g/L 0.8 g/L boric acid) was added. Cells from the shake flask was used to inoculate the bioreactor containing the following Bioreactor medium: 4.7 g/L KH₂PO₄, 1 g/L MgSO₄x7H₂O, 4.3 g/L (NH₄)₂SO₄, 45 g/L glucose, 0.7 g/L CaCl₂x2H₂O, 0.3 g/L antifoam agent (EROL DF 6000K), 2.5 ml/L of 400X T. reesei trace elements (175 g/L citric acid, 200 g/L FeSO4x7H₂O, 16 g/L ZnSO4x7H₂O, 3.2 g/L CuSO4x5H₂O, 1.4 g/L 0.8 g/L boric acid). Temperature was controlled at 34°C; pH was continuously controlled by adding 20% ammoniumhydroxide. Dissolved oxygen was controlled to minimum 40% saturation by varying the stirring rate. Off gas carbon dioxide and oxygen content were measured. When the initial glucose was depleted a constant feeding of a glucose/sophorose was started. At the same time temperature was reduced to and controlled at 28°C, pH was increased to and controlled at 4.5. The fermentation was terminated after 140 hours. Broth was removed from the tank, and cells were removed by filtration. After cell separation the filtrate was concentrated by ultrafiltration. Finally, the concentrate was sterile filtered and used for pelleting stability studies.

### Enzyme samples

Xylanase activity of culture supernatants from MTP were measured using the method for measurement of xylanase activity as described below. Culture supernatants were diluted 20 and 130 times in 25 mM sodium acetate, 250 mM NaCl, pH 4.0. 25 µL diluted enzyme sample was mixed with 150 µL 0.5 % WE-AX substrate, pH 5.0 and incubated at 30 °C for 15 min with shaking. After incubation, 45.4 µL reaction sample was mixed with 135 µL PAHBAH working solution and incubated at 95 °C for 5 min before cooled to 20 °C for 10 sec. 100 µL sample was transferred to a microtiter plate well and the plate was read at 410 nm.

The activity was calculated as the mean of three replicates subtracted a blank including 25 mM sodium acetate, 250 mM NaCl, pH 4.0 instead of enzyme. Protein concentration of the samples were calculated based on a standard curve of purified FveXyn4. All samples were diluted to 50 ppm in 25 mM sodium acetate, 250 mM NaCl, pH 4.0. These normalised samples were used as enzyme stock solution in assays described below.

Protein concentration in the enzyme stock solution was measured by HPLC as described below.

Xylanase activity of sterile filtered concentrates from large scale production was measured by the following activity assay. 0.5 g of each concentrate was weighed in 100 ml volumetric flasks followed by filling to volume with Mcllvaine buffer, pH 5.0. Samples were diluted to app. 6 XU/ml using Mcllvaine buffer, pH 5.0. 100 µl of diluted sample was added to 1ml of Mcllvaine buffer, pH 5.0 in test tubes and equilibrated at 50°C for 2 min. A Xylazyme tablet (100 mg) was added to initiate the reaction and samples were incubated at 40°C for 10 min before the reaction was stopped by adding 10 ml of 2 % Tris, pH 12.0. The solution was mixed using vortex, left to stand for 5 min and mixed again before centrifuged at 3500 rpm for 10 min. Absorbance of the supernatant was measured at 590 nm. Each sample was measured in duplicate. Xylanase activity was quantified relatively to an enzyme standard (Danisco Xylanase, available from Danisco Animal Nutrition).

The benchmark enzyme Econase® XT is a commercially available and was extracted from commercial dry formulated samples. The xylanase component from Econase® XT commercial dry formulated samples was extracted in a 33% (w/w) slurry using Mcllvain buffer, pH 5.0. The extract was cleared using centrifugation (3000 RCF for 10 min) and filtered using a PALL Acrodisc PF syringe filter (0.8/0.2 µm Supor membrane) and subsequently heated 20 min at 70°C. After removable of precipitation by centrifugation (38 724 RCF for 15 min) the buffer was replaced by passage through a Sephadex G25 column (PD10 from Pharmacia) equilibrated with 20 mM Na Citrate, 20 mM NaCl, pH 3.4. Purification of the xylanase component was performed using Source 15S resin, followed by elution with a linear increasing salt gradient (NaCl in 20 mM Na Citrate buffer pH 3.4).

Econase XT® is an endo-1,4-β-xylanase (EC 3.2.1.8) produced by the strain Trichoderma reesei RF5427 (CBS 114044), available from ABVista.

Protein concentration was determined by measuring absorption at 280nm. The extinction coefficients were estimates from the amino acid sequences. For Econase XT the absorption at 280nm of 1 mg/ml was calculated to be 2.84 AU.

### Protein Determination by HPLC

A MTP (Agilent Part no. 5042-1385) containing 100 µL enzyme stock solution with an approximate concentration of 50 ppm per well was used for the High Performance Liquid Chromatography (HPLC) protein determination method. An Agilent 1260 or 1290 (Hewlett Packard) HPLC equipped with an Acuity UPLC BEH 125 SEC (Waters) column was used to separate remaining contaminants. Sample was eluted from the column using 25 mM sodium phosphate buffer pH 6.8 containing 250 mM sodium chloride. Absorbance was measured at 220 nm, integrated using ChemStation software (Agilent Technologies) and the protein concentration of samples was determined based on a standard curve of purified FveXyn4 protein/enzyme having the amino acid sequence of SEQ ID No. 1.

### Measurement of xylanase activity

The xylanase activity of enzyme samples was determined by measuring amount of reducing sugars released from hydrolysed wheat WE-AX (water extractable arabinoxylan). The amount of reducing sugars was measured by PAHBAH-method. Briefly, by heat and alkaline conditions the reducing end groups react with the colorless PAHBAH (4-Para-Hydroxybenzoic Acid Hydrazide), whereby PAHBAH is oxidized and absorbance is measured at 410 nm (Lever, 1972).

0.5 % WE-AX substrate, pH 5.0 was prepared by moisterising 0.25 g soluble wheat arabinoxylan (e.g. Megazyme, high viscosity ∼43 cSt, P-WAXYH) with 2.5 ml 96 % Ethanol, before adding 50 ml 0.1 M sodium acetate, pH 5.0. Standard activity determination was carried out at pH 5.0. For measurement at other pH values, the 50 ml 0.1 M sodium acetate, pH 5.0 was substituted with the indicated buffer. The solution was heated under stirring to boiling, and cooled under stirring to RT.

PAHBAH working solution was prepared by mixing 5% PAHBAH (4-Hydroxybenzhydrazide, e.g. Sigma-Aldrich H9882) stock solution in 0.5 M HCl with 0.5 M NaOH at a 1:4 (v/v) ratio. The solution was prepared on the day of analysis and protected from light.

Enzyme samples were diluted in the indicated buffer to a concentration of 1 ppm prior to analysis. 25 µL diluted enzyme sample was mixed with 150 µL 0.5 % WE-AX substrate, pH 5.0 and incubated at 30 °C for 15 min with shaking. After incubation, 45.4 µL reaction sample was mixed with 135 µL PAHBAH working solution and incubated at 95 °C for 5 min before cooled to 20 °C for 10 sec. 100 µL sample was transferred to a microtiter plate well and the plate was read at 410 nm.

The activity was calculated as the mean of three replicates subtracted a blank including appropriate dilution buffer instead of enzyme.

### Assay for measurement of thermostability

The thermal denaturation profiles of the FveXyn4 variants was measured by diluting and pre-incubating the enzyme samples in 25 mM MES buffer (0.00125% Tween 80 - 25mM MES buffer, pH 6.0, 0.00125% (V:V) Tween 80), pH 6.0 for 10 min at varying temperatures (63, 65.5, 66.7, 68.2, 70.6, 73.5, 76.8, 79.7, 81.9, 83.5, 84.6, and 85 °C, respectively) and subsequently measuring the residual activity by the xylanase activity method described above. Activity measured without pre-incubation was set to 100 % and the residual activity of each variant at each temperature was calculated as relative to this. Tm value is calculated from the thermal denaturation profiles as the temperature at which 50 % residual activity is obtained.

### pH profile

The pH profile of the FveXyn4 variants was studied by measuring activity at pH 4.0, 5.0 and 6.0. Activity was measured essentially as described in the xylanase activity method described above. Enzyme samples were diluted in 0.1 M Na-Acetate, pH 4.0, 0.1 M Na-Acetate, pH 5.0, 0.1 % BSA (e.g. Sigma A7906), or Mcllvaine buffer, pH 6.0, for activity at pH 4.0, 5.0 or 6.0, respectively prior to analysis. 0.5 % WE-AX substrate at pH 4.0 and 6.0 was prepared as described for 0.5 % WE-AX substrate, pH 5.0, though 0.1 M sodium acetate, pH 5.0 was substituted with 0.1 M Na-Acetate, pH 4.0 or Mcllvaine buffer, pH 6.0, respectively.

### Pentosan solubilisation (AXinsol solubilisation)

The substrate used for measuring pentosan solubilisation by FveXyn4 variants was corn DDGS. 100 mg cDDGS with particle size < 212 µm was transferred to a 2 ml Eppendorf tube and the precise weight was recorded. 750 µl incubation buffer (200 mM HEPES, 100 mM NaCl, 2 mM CaCl₂, pH 6.0) and 900 µl chloramphenicol (40 µg/ml in incubation buffer) was added. Increasing enzyme dosages was added to make a total volume of 1.8 ml.

Each sample was assayed in doublets in parallel with a control sample (without enzyme). The samples were incubated at 40°C with shaking. After 18 hours of incubation the supernatant was filtered using 96 wells filterplates (Pall Corporation, AcroPrep 96 Filter Plate, 1.0 µm Glass, NTRL, 1 mL well). After filtration the samples were stored at 4°C until analysis for total amount of C5 sugars, arabinose and xylose.

### Quantification of C5 sugars (pentosans)

The total amount of pentoses brought into solution was measured using the method of Rouau and Surget (1994) with a continuous flow injection apparatus. The supernatants were treated with acid to hydrolyse polysaccharides to monosugars. Phloroglucinol (1, 3, 5-trihydroxybenzen) was added for reaction with monopentoses and monohexoses, which forms a coloured complex.

By measuring the difference in absorbance at 550 nm compared to 510 nm, the amount of pentoses in the solution was calculated using a standard curve. Unlike the pentose-phloroglucinol complex, the absorbance of the hexose-phloroglucinol complex is constant at these wavelengths. Glucose was added to the phloroglucinol solution to create a constant glucose signal and further ensure no interference from hexose sugars. The pentose concentration in the samples was determined using a xylose standard curve.

### Viscosity reduction in in vitro animal model assay

Viscosity reduction on wheat was determined using a modified version of the procedure described by Bedford & Classen (1993 Poultry Sci., 72, 137-143). 3.6 mL of pepsin solution (2000 U/mL in 0.1 N HCI) was mixed with 2.4 g wheat prior to addition of the indicated amount of xylanase (FveXyn4 variants) followed by 45 min incubation at 40 °C. 1.2 ml pancreatin solution (8 mg/mL in 1 M MES, pH 6.8) was then mixed into the slurry resulting in a final pH of 6.0. The sample was allowed to incubate for 60 min at 40 °C with mixing after 30 and 60 min. The sample was then placed on ice for 5 min to stop the reaction and centrifuged 10 min at 3320 RCF followed by filtration through a 0.45 µm filter to obtain a clear supernatant. Sample viscosity was then measured at 20°C using a Brookfield digital viscometer (model DV-I+, Brookfield Engineering Laboratories, Stoughton, MA 02172, USA) fitted with a CPE-40 cone and plate. Each data point is the average of three repetitions.

### Pelleting stability

Pelleting trials were performed in full scale at Technological Institute, Kolding, Denmark. Each sterile filtered xylanase concentrate was formulated on wheat and mixed into a corn/soy feed mix (61.1 % Corn, 31.52 % Hipro Soya 48, 4.00 % Soya Oil, 0.40 % Sodium Bicarbonate, 0.25 % Vitamins/Minerals Leghennen, 0.20 % DL-methionine, 1.46 % Dicalcium Phosphate, 1.16 % Limestone). A premix was prepared by mixing the xylanase variants formulated on wheat into 10 kg corn/soy feed mix and mixed for 10 min. The premix was then added to 120 kg feed and mixed for 10 min before conditioning. Feed was conditioned for 30 sec at 90 and 95 °C before pelleting. The mash and resulting feed pellets were grinded using a Perten laboratory mill (it is important that all samples are ground the same), before xylanase activity in the samples were analyzed according to either the extract or slurry method described below using azurine cross linked arabinoxylan from wheat (e.g. Xylazyme tablets, Megazyme, Ireland) as substrate.

Extract method: 5.0 g of ground sample was mixed with 50 ml Mcllvaine buffer, pH 5.0 and stirred on a magnetic stirrer for 10 min. The extract was filtered through a glass fiber filter and diluted 3-6 times in 50 ml Mcllvaine buffer, pH 5.0. 100 µl diluted extract was mixed with 400 µL Mcllvaine buffer, pH 5.0 and equilibrated at 50°C for 2 min. A Xylazyme tablet (60 mg) was added to initiate the reaction and samples were incubated at 50°C for 60 min before the reaction was stopped by adding 5 ml of 2 % Tris, pH 12.0. The solution was mixed using vortex, left to stand for 5 min and mixed again before centrifuged at 3500 rpm for 10 min. Absorbance of the supernatant was measured at 590 nm. Each sample was measured in duplicate.

Xylanase activity was quantified using a xylanase standard curve prepared for each of the xylanase variant on blank (no enzyme) mash and 90 °C feed. The respective wheat formulated xylanase was extracted for 10 min in Mc Ilvaine buffer, pH 5.0 to obtain a concentration of 160 XU/mL. The extract was filtered through a glass fiber filter and hereafter 0, 200, 400, 600, 800, and 1000 µL extract was added to 5.0 g samples of ground blank mash and 90 °C feed. Xylanase activity in these standard samples was measured as described in the extract method above. Each standard curve was prepared once.

Slurry method: 1.0 g of ground sample was mixed with 50 ml Mcllvaine buffer, pH 5.0 and stirred on a magnetic stirrer in a water bath at 50 °C for 2 min. A Xylazyme tablet (100 mg) was added to initiate the reaction and samples were incubated with stirring at 50 °C for 20 min (30 min for SV107). After incubation the samples were filtered through a glass fiber filter and absorbance of the supernatant was measured at 590 nm. Each sample was measured in duplicate.

Xylanase activity was quantified using a xylanase standard curve prepared for each of the xylanase variants on blank mash feed (no enzyme). The respective wheat formulated xylanase was extracted for 10 min in Mc Ilvaine buffer pH 5.0 to obtain a concentration of 30 XU/mL. The extract was filtered through a glass fiber filter and hereafter 0, 200, 400, 600, 800, and 1000 µL was added to 1.0 g samples of ground blank mash feed. Xylanase activity in these standard samples was measured as described in the slurry method above. The standard curve was prepared once.

Recovery measured in mash feed was set to 100 % and the residual activity of 90 and 95 °C feed was calculated as relative to this.

### Results and Discussion

Following a significant undertaking five variants of the backbone xylanase FveXyn4 were identified.

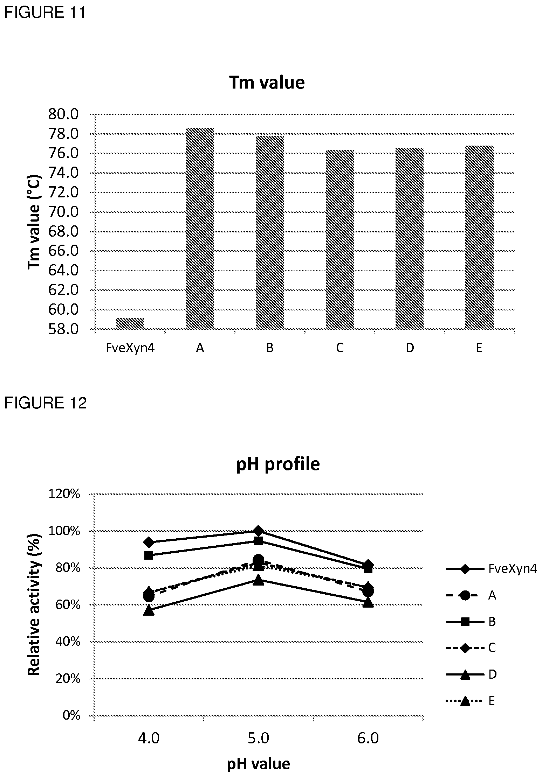

The five variants are all extremely more thermostable than the benchmark/parental molecule, FveXyn4, as shown in Figure 11.

Further characterization of the variant on biochemical and performance properties important for a xylanase to be used in for example feed applications identified these variants as being ones which were both thermostable and had good performance/biochemical activity.

| **Table 1.** Overview of mutations in the five variants of FveXyn4 | |
|---|---|
| **Varian ts** | **Mutations** |
| **A** | N7D_N25P_T33V_S57Q_N62T_K79Y_S89G_T103M_V115L_N147Q_G181Q_S193 Y_A217Q_G219P_T298Y |
| **B** | N7D_N25P_T33V_S57Q_N62T_G64T_K79Y_T103M_V115L_N147Q_G181Q_S193 Y_A217Q_G219P_T298Y |
| **C** | N7D_N25P_T33V_K79Y_S89G_A217Q_T298Y |
| **D** | N7D_T33V_S57Q_N62T_G64T_K79Y_S89G_A217Q_T298Y |
| **E** | N7D_N25P_T33V_G64T_K79Y_S89G_A217Q_T298Y |
| Numbering is based on the mature sequence of FveXyn4. SEQ ID No. 1. | |

Figure 11 shows the Tm value of the 5 variants A, B, C, D, and E compared to FveXyn4. Tm value is measured as the temperature at which 50 % residual activity is obtained after 10 min incubation.

Figure 12 shows pH profile of the five variants measured at pH 4.0, 5.0 and 6.0 and all data are relative to wild type at pH 5.0. All five variants have a pH profile that is ideal for use in, for example, feed applications.

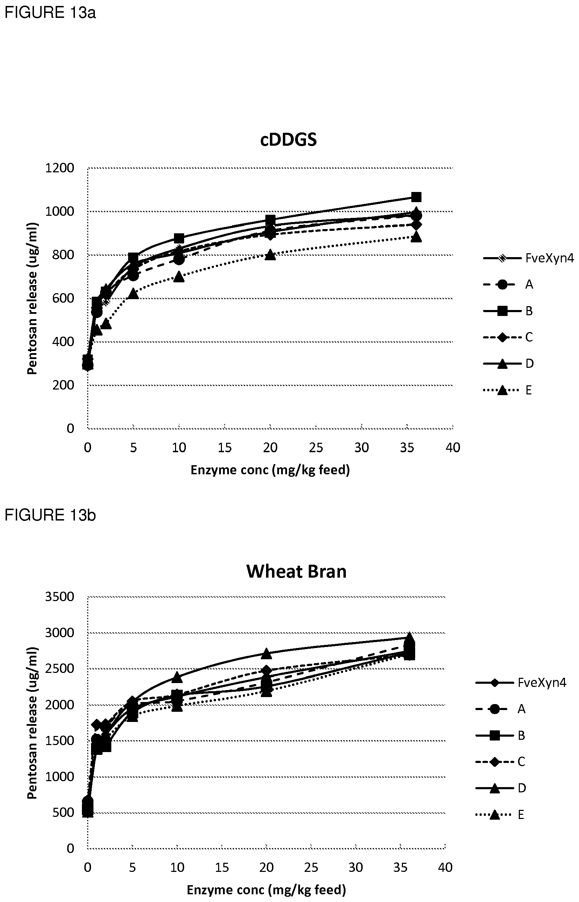

Figures 13a and 13b show dose response curves on pentosan solubilisation from corn DDGS and wheat bran, respectively by the five variants. All five variants show a high ability to solubilize arabinoxylan (pentosan) from both DDGS and wheat bran and all on the same level as the wt molecule. All five variants are very suitable for use in for example animal feed.

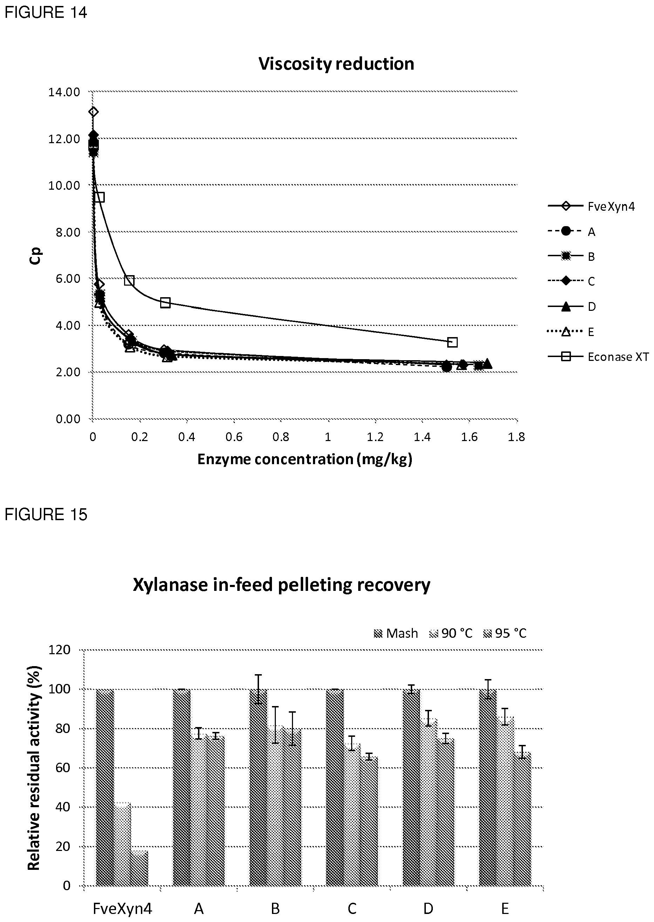

Figure 14 shows viscosity reduction in the "Viscosity reduction in *in vitro* animal model assay" taught in Example 1. All five variants show a high ability to reduce viscosity and all on the same level as the wt molecule and were much better than the benchmark Econase XT

Figure 15 shows in-feed xylanase recovery after pelleting at 90 and 95 °C. All five variants show high recovery of xylanase after pelleting and significantly higher than wild type.

### EXAMPLE 2

### Cloning of Fusarium verticillioides backbone (parent) xylanase (FveXyn4)

Genomic DNA isolated from a strain of *Fusarium verticillioides* was used for amplifying a xylanase gene. The sequence of the cloned gene, called the *FveXyn4* gene, is depicted in SEQ ID No. 2. The mature protein encoded by the *FveXyn4* gene is depicted in SEQ ID No. 1. The protein product of gene *FveXyn4* belongs to glycosyl hydrolase family 10 (GH10) based on the PFAM search (http://pfam.sanger.ac.uk/).

### EXAMPLE 3

### Expression of FveXyn4 backbone (parent) protein

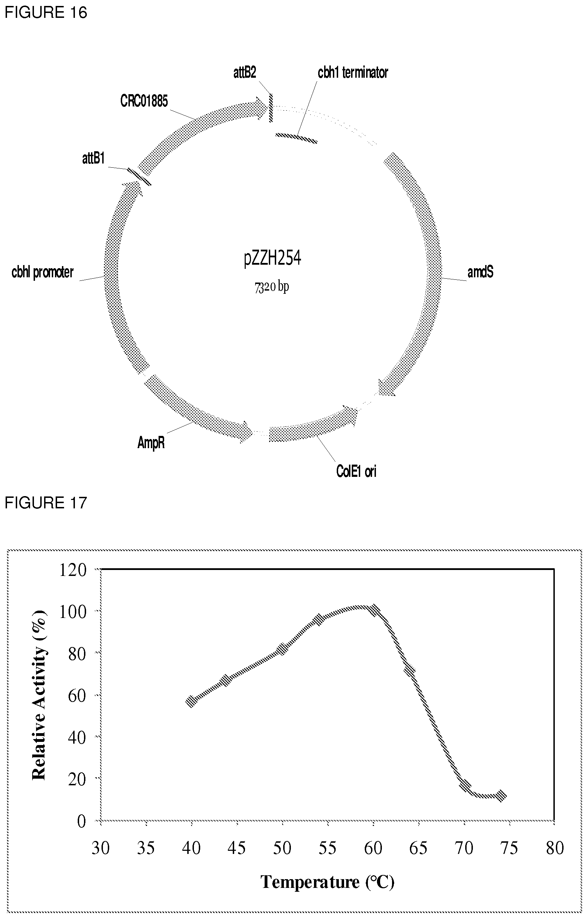

The *FveXyn4* gene was amplified from genomic DNA of *Fusarium verticillioides* using the following primers: Primer 1 5'-caccATGAAGCTGTCTTCTTTCCTCTA-3' (SEQ ID No. 22), and Primer 2 5'-TTTTTAGCGGAGAGCGTTGACAACAGC-3' (SEQ ID No. 23). The PCR product was cloned into pENTR/D-TOPO vector (Invitrogen K2400) to generate the *FveXyn4* pEntry plasmid. The expression plasmid pZZH254 was obtained by Gateway cloning reaction between the *FveXyn4* pEntry plasmid and pTrex3gM expression vector (described in US 2011/0136197 A1) using Gateway® LR Clonase® II enzyme kit (Invitrogen 11791). A map of plasmid pZZH254 is provided as Figure 16. The sequence of the *FveXyn4* gene was confirmed by DNA sequencing (SEQ ID No. 2). The plasmid pZZH254 was transformed into a quad deleted *Trichoderma reesei* strain (described in WO 05/001036) using biolistic method (Te'o VS et al., J Microbiol Methods, 51:393-9, 2002).

Following sequence confirmation, protoplasts of a quad deleted *T. reesei* strain (described in WO 05/001036) were transformed with the expression plasmid pZZH254 using the PEG protoplast method (Penttila et al, Gene, 61:155-164, 1987). For protoplast preparation, spores were grown for about 10 hours at 24°C in *Trichoderma* Minimal Medium MM (20 g/L glucose, 15 g/L KH₂PO₄, pH 4.5, 5 g/L (NH₄)₂SO₄, 0.6 g/L MgSO₄x7H₂O, 0.6 g/L CaCl₂x2H₂O, 1 ml of 1000X *T. reesei* Trace elements solution (175 g/L Citric Acid anhydrous, 200 g/L FeSO₄x7H₂O, 16 g/L ZnSO₄x7H₂O, 3.2 g/L CuSO₄, 1.4 g/L and 0.8 g/L Boric Acid). Germinating spores were harvested by centrifugation and treated with 30 mg/mL Vinoflow FCE (Novozymes, AG Switzerland) solution for from 7 hours to overnight at 30 °C at 100 rpm to lyse the fungal cell walls. Protoplasts were washed in 0.1 M Tris HCl buffer (pH 7) containing 0.6 M sorbitol and resuspended in 10 mM Tris HCl buffer (pH 7.5) containing 1.2 M sorbitol and 10 mM calcium chloride. For PEG transformation, approximately 1 µg of DNA and 1-5 x 10⁷ protoplasts in a total volume of 200 µl were treated with 2 ml of 25% PEG solution, diluted with 2 volumes of 1.2 M sorbitol/10 mM Tris, pH 7.5/10 mM CaCl₂ solution. Transformants were selected on a medium containing acetamide as a sole source of nitrogen (acetamide 0.6 g/L; cesium chloride 1.68 g/L; glucose 20 g/L; potassium dihydrogen phosphate 15 g/L; magnesium sulfate heptahydrate 0.6 g/L; calcium chloride dihydrate 0.6 g/L; iron (II) sulfate 5 mg/L; zinc sulfate 1.4 mg/L; cobalt (II) chloride 1 mg/L; manganese (II) sulfate 1.6 mg/L; agar 20 g/L; pH 4.25). Transformed colonies (about 50-100) appeared in about 1 week. After growth on acetamide plates, the spores were collected and reselected on acetamide plates. After 5 days, the spores were collected using 10% glycerol, and 1 x 10⁸ spores were inoculated in a 250 ml shake flask with 30 ml Glucose/Sophorose defined medium for protein expression. Protein expression was confirmed by SDS-PAGE. The spore suspension was subsequently grown in a 7 L fermentor in a defined medium containing 60% glucose-sophorose feed. Glucose/Sophorose defined medium (per liter) consists of (NH₄)2SO₄ 5 g, PIPPS buffer 33 g, Casamino Acids 9 g, KH₂PO₄ 4.5 g, CaCl₂ (anhydrous) 1 g, MgSO₄.7H₂O 1 g, pH to 5.5 adjusted with 50% NaOH with Milli-Q H₂O to bring to 966.5 mL. After sterilization, the following were added: 26 mL 60% Glucose/Sophrose, and 400X *T. reesei* Trace Metals 2.5 mL. FveXyn4 was purified from concentrated fermentation broth of a 7L fermentor culture using two chromatography columns. Concentrated fermentation broth buffered in 20 mM sodium phosphate buffer pH 6.0 containing 1 M ammonium sulfate was loaded on a hydrophobic interaction chromatography column (Sepharose Phenyl FF, 26/10). The protein was eluted from the column using a linear gradient of equilibration/wash buffer to 20 mM sodium phosphate buffer pH 6.0. The fraction containing FveXyn4 protein was loaded onto a gel filtration column (HiLoad Superdex 75 pg 26/60), and the mobile phase used was 20 mM sodium phosphate, pH 7.0, containing 0.15 M NaCl. The purified protein was concentrated using a 3K Amicon Ultra-15 device and the concentrated protein fraction was used in further studies.

The nucleotide sequence of *FveXyn4* gene is set forth as SEQ ID No. 24. The signal sequence is shown in bold (upper case), and the predicted intron is shown in bold and lowercase.

The amino acid sequence of FveXyn4 protein is set forth as SEQ ID No. 26. The signal sequence predicted by SignalP-NN software is shown underlined. This is the pre-pro-protein.

The amino acid sequence of the mature form of FveXyn4 protein is set forth as SEQ ID No. 1. This is the active form of the enzyme. SEQ ID No. 27 shows the pro-protein, i.e. before post-translational modification. Depending on the host the post-translation modification may vary and therefore the present invention also encompasses mature, active forms of SEQ ID No. 27.

### EXAMPLE 4

### Xylanase Activity of FveXyn4 (a parent xylanase)

FveXyn4 belongs to the glycosyl hydrolase 10 family (GH10, CAZy number). The beta 1-4 xylanase activity of FveXyn4 was measured using 1% xylan from birch wood (Sigma 95588) or 1% arabinoxylan from wheat flour (Megazyme P-WAXYM) as substrates. The assay was performed in 50 mM sodium citrate pH 5.3, 0.005% Tween-80 buffer at 50 °C for 10 minutes.

The released reducing sugar was quantified by reaction with 3, 5-Dinitrosalicylic acid and measurement of absorbance at 540 nm. The enzyme activity is quantified relative to a xylose standard curve. In this assay, one xylanase unit (U) is defined as the amount of enzyme required to generate 1 micromole of xylose reducing sugar equivalents per minute under the conditions of the assay.

### EXAMPLE 5

### Temperature Profile of FveXyn4 (a parent xylanase)

The temperature optimum of purified FveXyn4 (a parent enzyme) was determined by assaying for xylanase activity at temperatures varying between 40°C and 75°C for 10 minutes in 50mM sodium citrate buffer at pH 5.3. The activity was reported as relative activity where the activity at the temperature optimum was set to 100%. The temperature profile of FveXyn4 is shown in Figure 17. FveXyn4 was found to have an optimum temperature of 60°C, and was found to retain greater than 70% of maximum activity between 45°C and 64°C.

### EXAMPLE 6

### Viscosity reduction in grain-based material (e.g. for biofuel production)

### Wheat viscosity reduction

In the European fuel alcohol industry, small grains like wheat, barley and rye are common raw materials, in contrast to the US where mainly corn is used. These small grains contain, next to starch, high levels of non-starch polysaccharide polymers (NSP), like cellulose, beta-glucan and hemicellulose.

The ratio in which the different NSPs are represented differ for each feedstock. Table 1 shows the different amounts of NSPs in wheat, barley and rye compared to some other feedstocks.

**Table 1: Non-starch Polysaccharides present in different feedstocks (g kg⁻¹ dry matter)^{1,2}**

| | Corn | Wheat | Rye | Barley | | Oats | |
|---|---|---|---|---|---|---|---|
| | | | | Hulled | Hulless | Hulled | Hulless |
| Beta-Glucan | 1 | 8 | 16 | 42 | 42 | 28 | 41 |
| Cellulose | 22 | 17-20 | 15-16 | 43 | 10 | 82 | 14 |
| Soluble and Non-soluble NCP³ | 75 | 89-99 | 116-136 | 144 | 114 | 150 | 113 |
| Total NSP | 97 | 107-119 | 132-152 | 186 | 124 | 232 | 116 |

| | | | | | | | |
|---|---|---|---|---|---|---|---|
| ¹(Bach Knudsen, 1997) Carbohydrate and lignin contents of plant materials used in animal feeding. Anim. Feed Sci. Technol., 67 (4): 319-338 ²Englyst, H. N., Anderson, V. and Cummings, J. H., 1983. Starch and non-starch polysaccharides in some cereal foods. J. Sci. Food Agric., 34: 1434-1440. ³Non Cellulosic Polysaccharides: pentosans, (arabino)xylans and other hemicelluloses NSPs give high viscosity to grain mashes. High viscosity has a negative impact on ethanol production since it will limit the solid concentration that can be used in mashing and it will reduce the energy efficiency of the process. In addition, residual hemicelluloses present throughout the process may contribute to fouling in heat exchangers and distillation equipment. The largest impact of a high viscosity is seen when a mash is cooled to fermentation temperature (32°C). This explains that the viscosity needs to be reduced in the process anywhere before the cooling step. Depending on the process used, enzymes are needed that operate at 60°C and/or 85°C. | | | | | | | |

Viscosity reducing enzymes can be added in different stages of the ethanol production process: mixing and/or saccharification/fermentation. Preferably the enzymes are added in mixing to breakdown initial viscosity.

The benefits of using viscosity reduction enzymes in the ethanol production process are multiple:
- Higher dry substance mash can be used in the process
- Higher solids content of final syrup can be obtained
- Better heat transfer, lower energy requirement
- Reduced evaporator fouling leading to reduced cleaning costs
- Increased final ethanol yields
- Improved quality of DDGS

### Methods

A Rapid Visco Analyzer (RVA 4500) from Perten Instruments was used to measure viscosity profiles of a wheat mash. This wheat mash was prepared according to following protocol:
Prepare 60 grams of 30% DS (34.65% 'as is') wheat slurry (for simultaneous runs on two RVA's) as follows:
- Weigh 20.80 grams of wheat
- In a 100 ml beaker glass, weigh 39.20 grams of tap water and add 137µl 4N H₂SO₄
- Add the wheat to the water and stir for 5 minutes at maximum speed (approx. 500 rpm) with an overhead stirrer
- Transfer 25.0 grams of slurry to an RVA cup, add 50-fold diluted enzymes and start RVA run (check if starting pH is around 5.3)
- Check pH at end of RVA run (5.6-5.7)

In each experiment (25 grams of slurry), xylanase was dosed at 25 µg protein (per 8.66 g wheat 'as is'), corresponding to2.9 µg protein / g wheat 'as is'. SPEZYME® CL was dosed at 0.15 kg/MT wheat 'as is' (2.2 AAU/g 'as is' or 2.6 AAU/g DS).

A standard wheat liquefaction was mimicked in the RVA. Pretreatment was performed for 20 minutes at 60°C, followed by a liquefaction step for 30 minutes at 85°C. After pretreatment and liquefaction, slurry was cooled down to 32°C, to determine viscosity at fermentation conditions. Liquefaction pH was kept between 5.3-5.7.

I n this experiment, the performance of FveXyn4 was compared to Variant A, B, C, D and E.

| | **Viscosity (mPa*****s)** | | | | | | |
|---|---|---|---|---|---|---|---|
| | Blank (n=2) | FveXyn4 | Variant A | Variant B | Variant C | Variant D | Variant E |
| After pretreatment (1200 sec. process time) | 533 ± 16 | 206 | 220 | 232 | 224 | 235 | 240 |
| After liquefaction (3120 sec. process time) | 347 ± 16 | 122 | 125 | 135 | 130 | 141 | 145 |
| At fermentation temperature (3660 sec. process time) | 765 ± 20 | 250 | 257 | 282 | 275 | 302 | 298 |

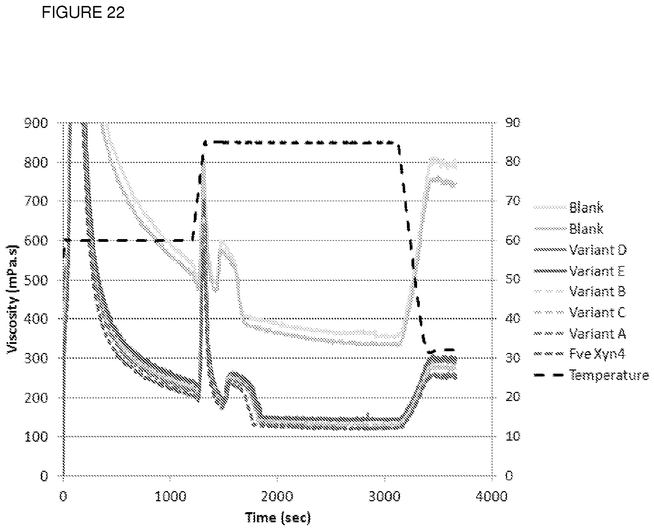

The results are shown above and in Figure 22.

These data show that FveXyn4 and all variants perform very similar, showing viscosity reduction of 55-67% compared to blank (only SPEZYME® CL).

### EXAMPLE 7

### Wheat gluten-starch separation

Separation of wheat flour into starch and gluten fractions is industrially applied on large scale to obtain high quality A-starch and byproducts B-starch and vital gluten. Separation is improved by addition of xylanases.

### 7.1 Materials and Methods

The following assay simulates the wheat starch separation of a batter process at 40 °C. In this assay, industrial wheat flour (Cargill) is added to preheated tap water (50°C) to create a 35%DS slurry by mixing 1 minute in a kitchen blender (Braun). pH of the slurry remains as is' at ∼6.1. 100 gram of this slurry is transferred to the Haake VT550 viscometer, which is calibrated at 40°C. After 1 minute of incubation, the enzyme solution Is added to the slurry. Meanwhile, the viscosity profile is monitored before and after enzyme addition for 15 minutes in total. After incubation, triplicate samples of the incubated slurry and one sample of t₀ slurry is taken for a spin test. Each spin test sample has a total weight of 22.5 g, which contains 15.8-15.9 g slurry sample added to 6.6-6.7 g of disposable centrifuge tube (15ml). All samples are centrifuged in a Hermle Z400 centrifuge for 15 minutes at 3500 rpm. Brix values
are determined from the syrup of the centrifuged samples..

### EXAMPLE 8

### Cloning of a backbone (parent) Fusarium oxysporum Xylanase FoxXyn2

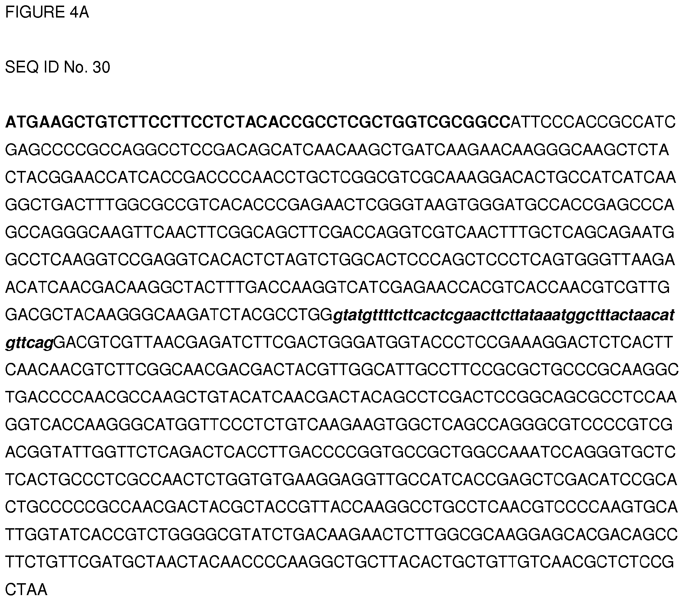

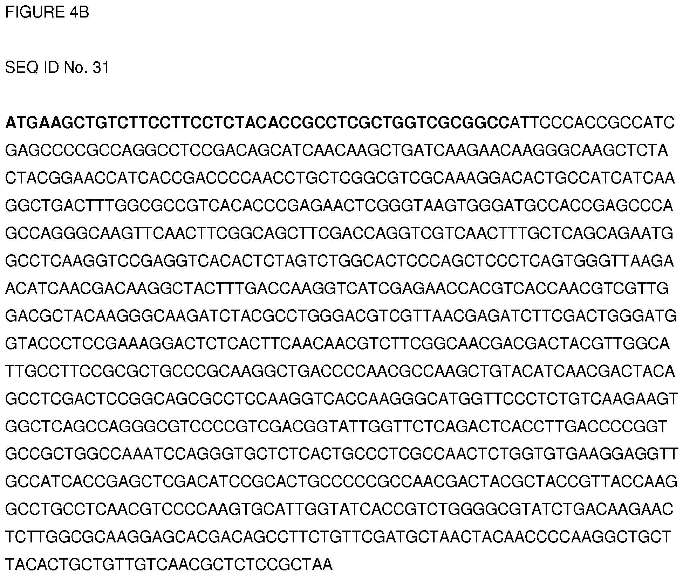

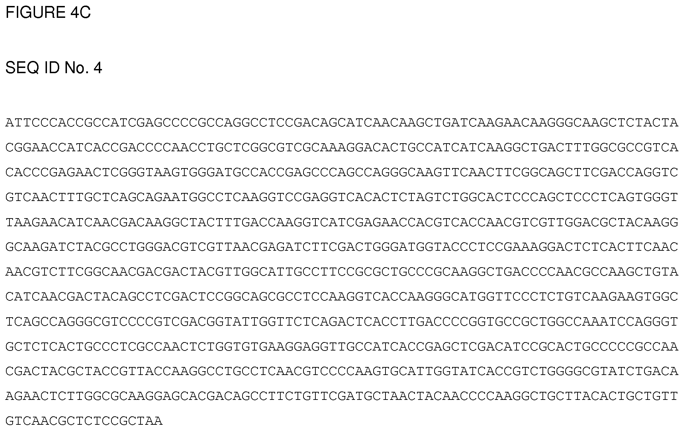

The nucleotide sequence of the *FoxXyn2* gene isolated from *Fusarium oxysporum* is set forth as SEQ ID No. 30 (Figure 4A). The signal sequence is shown in bold, and the predicted intron is shown in italics and lowercase.

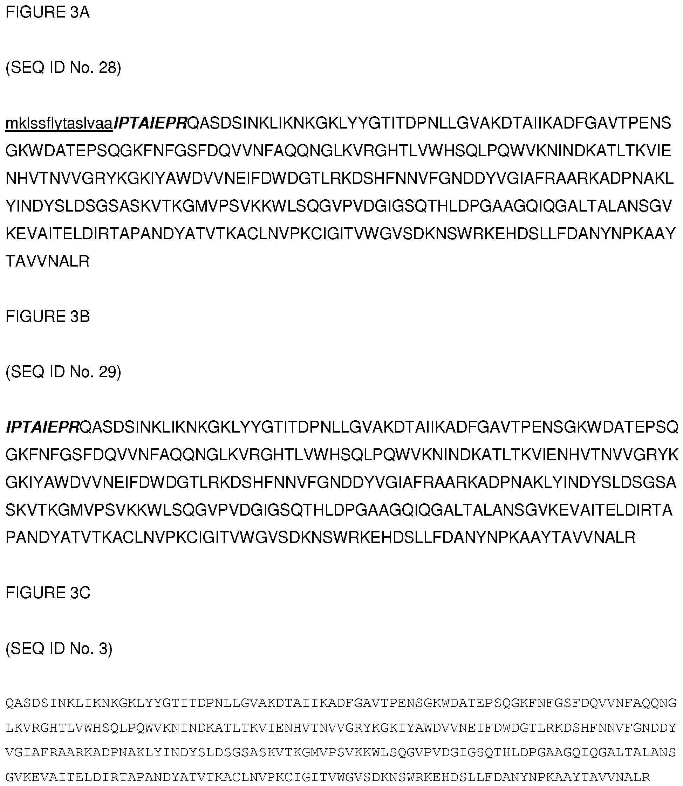

The amino acid sequence of FoxXyn2 protein is set forth as SEQ ID No. 29 (Figure 3A). The signal sequence is shown in italics.

The amino acid sequence of the mature form of FoxXyn2 protein is set forth as SEQ ID No. 31 or SEQ ID No. 4 (Figures 3B and 3C).

The protein product of gene *FoxXyn2* belongs to glycosyl hydrolase family 10. This suggests that FoxXyn2 is a secreted glycosyl hydrolase.

### EXAMPLE 9

### Expression of backbone (parent) FoxXyn2 protein

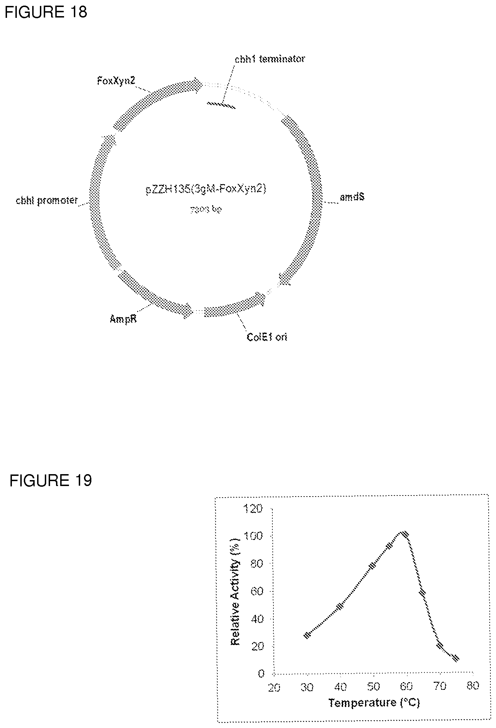

The *FoxXyn2* gene was amplified from genomic DNA of *Fusarium oxysporum* using the following primers: Primer 1 5'- ccgcggccgcaccATGAAGCTGTCTTCCTTCCTCTACACC-3' (SEQ ID NO:34), and Primer 25'-ccggcgcgcccttaTTAGCGGAGAGCGTTGACAACAG -3' (SEQ ID NO:35). After digested with *Not I* and *Asc I,* the PCR product was cloned into pTrex3gM expression vector (described in US 2011/0136197 A1) digested with the same restriction enzymes, and the resulting plasmid was labeled pZZH135. A plasmid map of pZZH135 is provided in Figure 18. The sequence of the *FoxXyn2* gene was confirmed by DNA sequencing.

The plasmid pZZH135 was transformed into a quad deleted *Trichoderma reesei* strain (described in WO 05/001036,) using biolistic method (taught in Te'o VS et al., J Microbiol Methods, 51:393-9, 2002). The protein isolated from the culture supernatant after filtration was used to perform SDS-PAGE analysis and xylanase activity assay to confirm enzyme expression.

The nucleotide sequence of *FoxXyn2* gene from expression plasmid pZZH135 is set forth as SEQ ID No. 4. The amino acid sequence of the mature form of FoxXyn2 protein is set forth as SEQ ID No. 3.

FoxXyn2 protein was purified from culture supernatant using affinity chromatography resin Blue Sepharose, 6FF, and samples were used for biochemical characterization as described in subsequent examples.

### EXAMPLE 10

### Xylanase Activity of backbone (parent) FoxXyn2

FoxXyn2 belongs to the glycosyl hydrolase 10 family (GH10, CAZy number). The beta 1-4 xylanase activity of FoxXyn2 was measured using 1% xylan from birch wood (Sigma 95588) or 1% arabinoxylan from wheat flour (Megazyme P-WAXYM) as substrates. The assay was performed in 50 mM sodium citrate pH 5.3, 0.005% Tween-80 buffer at 50 °C for 10 minutes.

The released reducing sugar was quantified by reaction with 3, 5-Dinitrosalicylic acid and measurement of absorbance at 540 nm. The enzyme activity is quantified relative to a xylose standard curve. In this assay, one xylanase unit (U) is defined as the amount of enzyme required to generate 1 micromole of xylose reducing sugar equivalents per minute under the conditions of the assay.

### EXAMPLE 11

### Temperature Profile of FoxXyn2

The temperature optimum of purified FoxXyn2 was determined by assaying for xylanase activity at temperatures varying between 45 °C and 94 °C for 10 minutes in 50mM sodium citrate buffer at pH 5.3. The activity was reported as relative activity where the activity at the temperature optimum was set to 100%. The temperature profile of FoxXyn2 is shown in Figure 19. FoxXyn2 was found to have an optimum temperature of 60°C, and was found to retain greater than 50% of maximum activity between 40°C and 65°C.

### EXAMPLE 12

### Thermostability

Thermostability of the FveXyn4 wildtype (wt) and variants of FveXyn4 was measured at 63°C (see data in Table 2). It is clearly seen that all variants representing different numbers (2-4) and combinations of mutations has higher residual activity at 63°C compared to FveXyn4 and it can be concluded that these variants are all more thermostable than FveXyn4 wildtype (e.g. shown herein as SEQ ID No. 1, or SEQ ID No. 27).

### Materials and methods

Variants of FveXyn4 were obtained from combinatorial libraries or by introduction of specific mutations as described in Example 1. The thermostability of the FveXyn4 variants was measured by diluting and pre-incubating the enzyme samples in 25 mM MES buffer (0.00125% Tween 80 - 25mM MES buffer, pH 6.0, 0.00125% (V:V) Tween 80), pH 6.0 for 10 min at 63°C. After incubation the residual activity was measured by the xylanase activity method described in Example 1. Activity measured without pre-incubation was set to 100% and the residual activity of each variant at the respective temperature was calculated as relative to this.

Table 2 shows 14 combinatorial variants of FveXyn4 with significantly increased thermostability as compared to FveXyn4 wt. Thermostability is measured as residual activity after pre-incubation at 63°C for 10 min as described in above in the Materials and methods section of this Example 12.

| **Table 2** | | |
|---|---|---|
| **Variant** | **Mutations** | **Residual activity at 63 °C** |
| FveXyn4 | WT | 0.04 |
| 1 | K79F_A217Q_T298F | 0.89 |
| 2 | N7D_T33V_A217Q_T298F | 0.80 |
| 3 | N7D_K79F_T298F | 0.79 |
| 4 | T33V_K79F_A217Q | 0.77 |
| 5 | N7D_T33V_T298Y | 0.76 |
| 6 | T33V_A217Q_T298Y | 0.67 |
| 7 | N7D_ A217Q_T298F | 0.65 |
| 8 | N7D_T33V_A217Q | 0.60 |
| 9 | K79F_T298F | 0.59 |
| 10 | N7D_K79F | 0.58 |
| 11 | T33V_K79F | 0.57 |
| 12 | T33V_T298Y | 0.55 |
| 13 | N7D_T33V | 0.40 |
| 14 | T33V_A217Q | 0.35 |

### EXAMPLE 13

### Oil Recovery: Effect of GH10 xylanase FveXyn4 (SEQ ID NO: 1) and Modified Variants Thereof on Oil Recovery in Corn Ethanol Fermentations

The GH 10 xylanases FveXyn4 (SEQ ID NO: 1) and Variant E (SEQ ID NO: 21) were found to increase free oil availability at the end of fermentation when added to at the start of laboratory scale corn ethanol fermentations.

### Materials and Methods:

### Enzyme preparations:

FveXyn xylanase
Variant E xylanase
DISTILLASE® SSF (DuPont Industrial Biosciences)

### Simultaneous Saccharification and Fermentation (SSF)

The concentrations of the FveXyn4 and Variant E xylanases were determined using a standard BCA assay.

Corn liquefact for experiments was obtained from Big River Resources, Dyersville. The starting amount of dry solids in the liquefact was measured, and the pH was adjust to pH 4.8. Fermax™ Gold yeast was added at 0.1 % w/w and all fermentations received DISTILLASE® SSF at a dose of 0.325 GAU/g DS (see Table 3). In fermentations with xylanases, the xylanases were added at 0.25 mg/g DS (see Table 3). The fermentations were run at 32°C for 64 hours, with constant mixing. Each condition was run at 15 g scale, in triplicate.

**TABLE 3.**

| **Enzyme** | **Dose** |
|---|---|
| Control (DISTILLASE® SSF) | 0.325 GAU/g DS |
| FveXyn4 | 0.25 mg/g DS |
| Variant E | 0.25 mg/g DS |

### Oil measurement:

After 64 hours, the samples were cooled to stop the fermentation and samples were immediately processed.

In one replicate the resulting mash was mixed vigorously with 2 mL hexane, then centrifuged at 10000 rpm for 2 minutes to separate the hexane-oil layer from the solids and aqueous layers. After centrifugation, 10 uL of the topmost hexane-oil layer was pipette into a crimp-cap glass HPLC vial containing 290 uL hexane and mixed. The amount of the oil extracted from the whole mash into the hexane layer is measured by HPLC with a light scattering detector to give the Total Oil present at the end of fermentation.

In the other two replicates, the remaining mash was first centrifuged at 10000 rpm for 2 minutes to enable physical separation of the solid and liquid phases. Without disturbing the separated mash, 2 mL hexane was added to the top, and the mixture was gently swirled for 2 minutes on an orbital mixer. After mixing, 10 uL of the topmost hexane-oil layer was pipette into a crimp-cap glass HPLC vial containing 290 uL hexane and mixed. The amount of oil extracted from the supernatant layer is measured directly (no column) by HPLC with a light scattering detector to give the available oil at the end of fermentation.

The % oil recovered for each condition is calculated by dividing the available oil by the total oil. A standard curve was created with sample of hexane containing known amounts of corn oil.

### Results:

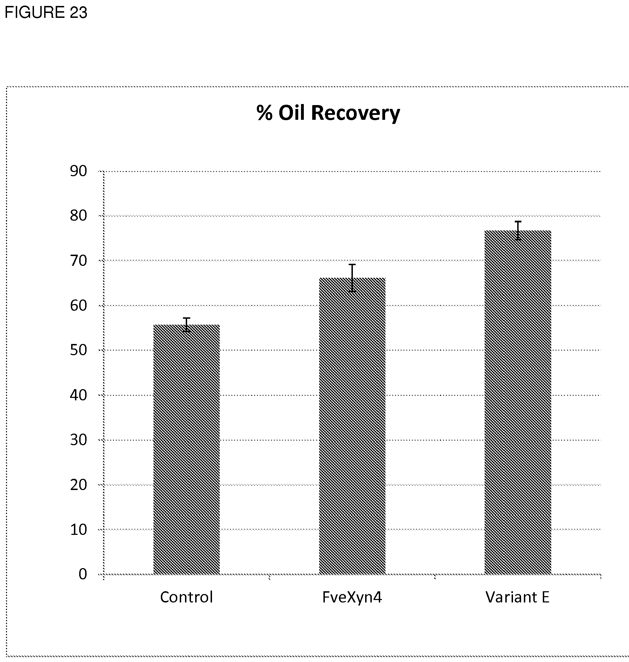

The results are shown in Figure 23. At equal doses (0.25 mg/g DS), all three experimental xylanases tested increased the % oil recovered at the end of fermentation when compared to the control without any xylanase. Variant E had the biggest positive effect on oil availability, which exceeded expectations based on its specific activity vs the parent xylanase FveXyn4. Variant E has lower specific activity than FveXyn4 at pH 5.0 (2600 U/mg vs 3300 U/mg, respectively), and has less ability than WT to solubilize corn DDGs (91% vs WT). See Table 4.

**TABLE 4.**

| Xylanases | SPAC pH 5.0 (U/mg) | cDDGs solubilization (PI) |
|---|---|---|
| FveXyn4 | 3300 | 1 |
| Variant E | 2600 | 0.91 |
| | | Dosing 36 mg/kg) |

### EXAMPLE 14

FveXyn4 has been demonstrated to enhance oil availability in corn germ & fiber mixtures.

### Materials and Methods:

Substrates: millled corn germ and flaked corn fiber from Ingredion Inc.

### Enzymes:

FERMGEN® (acid fungal protease from DuPont Industrial Biosciences)
XLATHIN® XYLANASE (Verenium Corporation)
FveXyn4 xylanase
Solvent: Hexane (for oil extraction)

HPLC with CAD detector was used for oil quantification. A standard curve was created with sample of hexane containing known amounts of corn oil.

Concentrations of the enzyme samples were determined using a standard BCA protein assay. XYLATHIN® xylanase and FveXyn4 xylanase were diluted to 2 ppm (2 mg/L) before dosing.

In glass tubes, 15 ml of 25mM Acetate buffer (pH 5.0), 5% (w/w) corn germ and 6.5% (w/w) corn fiber was added. Each tube was heated in a water bath to 85 °C and held for 30 minutes and then allowed to cool to room temperature. To each tube 45ul of FERMGEN® was added, and 6ul of the diluted xylanase was added. The tubes were the incubated, with shaking, at 35 °C for 18 hrs. After reaction, the contents of each tube was poured off into a 50ml polystyrene tube and spun at 10000 rpm for 5 minutes. To the top of each sample, 2ml of hexane was then added carefully, and the samples were slowly swirled without disturbing the pellet for two minutes on an orbital mixer. A 200- fold dilution of the hexane layer was done and the samples were loaded onto the HPLC to be quantified by a standard curve previously obtained. This measurement gives available oil after reaction.

To one of the samples, the hexane was vigorously shaken for a few minutes to obtain total oil extracted by hexane.

The % oil recovered for each condition is calculated by dividing the available oil by the total oil.

### Results:

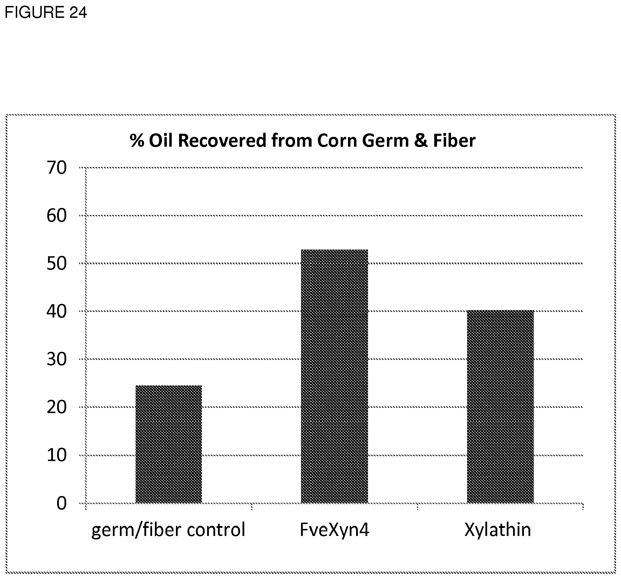

As shown in Figure 24, at equal protein doses and in combination with FERMGEN®, both xylanases tested increased the % oil recovered from the mixture of corn germ and corn fiber when compared to a no-xylanase control. FveXyn4 (GH10) improved % oil recovery the most (52% vs 25% oil recovered when no xylanase control), outperforming XYLATHIN® xylanase which exhibited only 40% oil recovered.

## Claims

1. A xylanase enzyme having at least 70% amino acid sequence identity to the amino acid sequence of SEQ ID NO: 21, the xylanase enzyme comprising amino acids 7D, 33V, 79Y, 217Q and 298Y at positions corresponding to the amino acid numbering of SEQ ID NO: 21 and wherein the xylanase enzyme has xylanase activity and is capable of increased oil recovery from grain-based material as compared to the xylanase enzyme of SEQ ID NO: 1.

2. The xylanase enzyme according to claim 1 wherein the increased oil recovery is measurable at end of fermentation oil recovery.

3. The xylanase enzyme according to claim 1 or claim 2, wherein the xylanase enzyme comprises one of the following combinations of amino acids at positions corresponding to the amino acid numbering of SEQ ID NO: 21:
A: 7D, 25P, 33V, 57Q, 62T, 79Y, 89G, 103M, 115L, 147Q, 181Q, 193Y, 217Q, 219P and 298Y;
B: 7D, 25P, 33V, 57Q, 62T, 64T, 79Y, 103M, 115L, 147Q, 181Q, 193Y, 217Q, 219P and T298Y; or
C: 7D, 25P, 33V, 79Y, 89G, 217Q, and 298Y; or
D: 7D, 33V, 57Q, 62T, 64T, 79Y, 89G, 217Q and T298Y; or
E: 7D, 25P, 33V, 64T, 79Y, 89G, 217Q and 298Y.

4. The xylanase enzyme according to any one of the preceding claims, wherein the enzyme has at least 80% amino acid sequence identity to the amino acid sequence of SEQ ID NO: 21.

5. The xylanase enzyme according to any one of the preceding claims, wherein the enzyme has at least 90% amino acid sequence identity to the amino acid sequence of SEQ ID NO: 21.

6. The xylanase enzyme according to any one of the preceding claims, wherein the enzyme has at least 95% amino acid sequence identity to the amino acid sequence of SEQ ID NO: 21.

7. A process of recovering oil, comprising:
(a) liquefying a grain-based material into dextrins with an alpha-amylase;
(b) saccharifying the dextrins using a carbohydrate source generating enzyme to form a sugar;
(c) fermenting the sugar in a fermentation medium into a fermentation product using a fermenting organism;
(d) distilling the fermentation product to form a whole stillage;
(e) separating the whole stillage into thin stillage and wet cake; and
(f) recovering the oil from the thin stillage, wherein at least one or more of liquefying, saccharifying and fermenting comprise admixing a xylanase of any one of claims 1 to 6.

8. The process of claim 7, further comprising a step of steeping and/or a post fermentation processing step wherein said modified xylanase enzyme is admixed.

9. A process of recovering oil in a simultaneous saccharification and fermentation (SSF) process, comprising:
(a) admixing an alpha-amylase, a carbohydrate source generating enzyme and a fermenting organism to a grain-based material in a fermentation medium;
(b) performing SSF to form a sugar in the fermentation medium and further fermenting the sugar into a fermentation product using the fermenting organism;
(c) distilling the fermentation product to form a whole stillage;
(d) separating the whole stillage into thin stillage and wet cake; and
(e) recovering the oil from the thin stillage, wherein performing SSF comprises admixing a xylanase of any one of claims 1 to 6.

10. A process of recovering oil in a granular starch hydrolyzing enzyme (GSHE) process, comprising:
(a) contacting a grain-based material comprising granular starch at a temperature at or below the gelatinization temperature of the granular starch with one or more GSHE enzyme and a xylanase of any one of claims 1 to 6;
(b) allowing the alpha amylase, GSHE enzyme and xylanase to act for a period of time between 2 and 100 hours to obtain a fermentable sugar syrup;
(c) fermenting the sugar syrup into a fermentation product using a fermenting organism;
(d) distilling the fermentation product to form a whole stillage;
(e) separating the whole stillage into thin stillage and wet cake; and
(f) recovering the oil from the thin stillage.

11. The process of claim 10, wherein said sugar syrup is selected from the group consisting of a disaccharide, oliogosaccharide, polysaccharide, glucose, and maltose and/or the grain-based material comprises grain and/or cereal.

12. The process of any one of claims 7 to 11, wherein the xylanase enzyme is used in combination with one or more of the enzymes selected from endoglucanases (E.C. 3.2.1.4), celliobiohydrolases (E.C. 3.2.1.91), 13-glucosidases (E.C. 3.2.1.21), cellulases 35 (E.C. 3.2.1.74), lichenases (E.C. 3.1.1.73), lipases (E.C. 3.1.1.3), lipid acyltransferases (generally classified as E.C. 2.3.1.x), phospholipases (E.C. 3.1.1.4, E.C. 3.1.1.32 or E.C. 3.1.1.5), phytases (e.g. 6-phytase (E.C. 3.1.3.26) or a 3-phytase (E.C. 3.1.3.8), amylases, alpha-amylases (E.C. 3.2.1.1), other xylanases (E.C. 3.2.1.8, E.C. 3.2.1.32, E.C. 3.2.1.37, E.C. 3.1.1.72, E.C. 3.1.1.73), glucoamylases (E.C. 3.2.1.3), hemicellulases, proteases (e.g. subtilisin (E.C. 3.4.21.62) or a bacillolysin (E.C. 3.4.24.28) or an alkaline serine protease (E.C. 3.4.21.x) or a keratinase (E.C. 3.4.x.x) or 5 an acid stable protease), debranching enzymes, cutinases, esterases, mannanases (e.g. a 13-mannanase (E.C. 3.2.1.78)) or any combinations thereof.

## Patentansprüche

1. Xylanaseenzym, das mindestens 70 % Aminosäuresequenzidentität mit der Aminosäuresequenz von SEQ ID NO: 21 aufweist, wobei das Xylanaseenzym die Aminosäuren 7D, 33V, 79Y, 217Q und 298Y an Positionen umfasst, die der Aminosäurenummerierung von SEQ ID NO: 21 entsprechend und wobei das Xylanaseenzym eine Xylanaseaktivität aufweist und einer erhöhten Ölgewinnung aus Material auf Getreidebasis im Vergleich mit dem Xylanaseenzym von SEQ ID NO: 1 fähig ist.

2. Xylanaseenzym nach Anspruch 1, wobei die erhöhte Ölgewinnung am Ende der Fermentationsölgewinnung messbar ist.

3. Xylanaseenzym nach Anspruch 1 oder Anspruch 2, wobei das Xylanaseenzym eine der folgenden Kombinationen von Aminosäuren an Positionen umfasst, die der Aminosäurenummerierung von SEQ ID NO: 21 entsprechen:
A: 7D, 25P, 33V, 57Q, 62T, 79Y, 89G, 103M, 115L, 147Q, 181Q, 193Y, 217Q, 219P und 298Y;
B: 7D, 25P, 33V, 57Q, 62T, 64T, 79Y, 103M, 115L, 147Q, 181Q, 193Y, 217Q, 219P und T298Y; oder
C: 7D, 25P, 33V, 79Y, 89G, 217Q und 298Y; oder
D: 7D, 33V, 57Q, 62T, 64T, 79Y, 89G, 217Q und T298Y;
E: 7D, 25P, 33V, 64T, 79Y, 89G, 217Q und 298Y.

4. Xylanaseenzym nach irgendeinem der vorhergehenden Ansprüche, wobei das Enzym mindestens 80 % Aminosäuresequenzidentität mit der Aminosäuresequenz von SEQ ID NO: 21 aufweist.

5. Xylanaseenzym nach irgendeinem der vorhergehenden Ansprüche, wobei das Enzym mindestens 90 % Aminosäuresequenzidentität mit der Aminosäuresequenz von SEQ ID NO: 21 aufweist.

6. Xylanaseenzym nach irgendeinem der vorhergehenden Ansprüche, wobei das Enzym mindestens 95 % Aminosäuresequenzidentität mit der Aminosäuresequenz von SEQ ID NO: 21 aufweist.

7. Verfahren zum Gewinnen von Öl, umfassend:
(a) Verflüssigen eines Materials auf Getreidebasis in Dextrine mit einer Alpha-Amylase;
(b) Saccharifizieren der Dextrine unter Anwendung eines eine Kohlehydratquelle erzeugenden Enzyms, um einen Zucker zu bilden;
(c) Fermentieren des Zuckers in einem Fermentationsmedium zu einem Fermentationsprodukt unter Anwendung eines Fermentierorganismus;
(d) Destillieren des Fermentationsprodukts, um eine Ganzschlempe zu bilden;
(e) Trennen der Ganzschlempe in eine Dünnschlempe und einen Nasskuchen; und
(f) Gewinnen des Öls aus der Dünnschlempe, wobei mindestens eines oder mehr von Verflüssigen, Saccharifizieren und Fermentieren das Zumischen einer Xylanase nach einem der Ansprüche 1 bis 6 umfasst.

8. Verfahren nach Anspruch 7, ferner einen Schritt des Weichens und/oder einen Nachfermentierungsverarbeitungsschritt umfassend, wobei das modifizierte Xylanaseenzym zugemischt wird.

9. Verfahren zum Gewinnen von Öl bei einem simultanen Saccharifizierungs- und Fermentations- (SSF) Vorgang, umfassend:
(a) Zumischen einer Alpha-Amylase, eines eine Kohlehydratequelle erzeugenden Enzyms und eines Fermentierorganismus zu Material auf Getreidebasis in einem Fermentationsmedium;
(b) Ausführen der SSF, um einen Zucker in dem Fermentationsmedium zu bilden und weiteres Fermentieren des Zuckers in ein Fermentationsprodukt unter Verwendung des Fermentierorganismus;
(c) Destillieren des Fermentationsprodukts, um eine Ganzschlempe zu bilden;
(d) Trennen der Ganzschlempe in eine Dünnschlempe und einen Nasskuchen; und
(e) Gewinnen des Öls aus der Dünnschlempe, wobei das Ausführen des SSF das Zumischen einer Xylanase nach irgendeinem der Ansprüche 1 bis 6 umfasst.

10. Verfahren zum Gewinnen von Öl bei einem Verfahren zum Hydrolysieren granulärer Stärke durch Enzym (GSHE), umfassend:
(a) Kontaktieren eines Materials auf Getreidebasis umfassend granuläre Stärke bei einer Temperatur von oder unter der Gelatinierungstemperatur der granulären Stärke mit einem oder mehr GSHE-Enzymen und einer Xylanase nach einem der Ansprüche 1 bis 6;
(b) Gestatten, dass die Alpha-Amylase, das GSHE-Enzym und die Xylanase für eine Zeitspanne zwischen 2 und 100 Stunden wirken, um einen fermentierbaren Zuckersirup zu erhalten;
(c) Fermentieren des Zuckersirups zu einem Fermentationsprodukt unter Verwendung eines Fermentierorganismus;
(d) Destillieren des Fermentationsprodukts, um eine Ganzschlempe zu bilden;
(e) Trennen der Ganzschlempe in eine Dünnschlempe und einen Nasskuchen; und
(f) Gewinnen des Öls aus der Dünnschlempe.

11. Verfahren nach Anspruch 10, wobei der Zuckersirup aus der Gruppe ausgewählt wird bestehend aus Disaccharid, Oligosaccharid, Polysaccharid, Glucose und Maltose und/oder das Material auf Getreidebasis Getreide und/oder Cerealien umfasst.

12. Verfahren nach irgendeinem der Ansprüche 7 bis 11, wobei das Xylanaseenzymin Kombination mit einem oder mehr der Enzyme verwendet wird ausgewählt unter Endoglucanasen (E.C. 3.2.1.4), Cellobiohydrolasen (E.C. 3.2.1.91), 13-Glucosidasen (E.C. 3.2.1.21), Cellulasen 35 (E.C. 3.2.1.74), Lichenasen (E.C. 3.1.1.73), Lipasen (E.C. 3.1.1.3), Lipidacyltransferasen (im Allgemeinen als E.C. 2.3.1.x klassifiziert), Phospholipasen (E.C. 3.1.1.4, E.C. 3.1.1.32 oder E.C. 3.1.1.5), Phytasen (z.B. 6-Phytasen (E.C. 3.1.3.26) oder einer 3-Phytase (E.C. 3.1.3.8), Amylasen, Alpha-Amylasen (E.C. 3.2.1.1), anderen Xylanasen (E.C. 3.2.1.8, E.C. 3.2.1.32, E.C. 3.2.1.37, E.C. 3.1.1.72, E.C. 3.1.1.73), Glucoamylasen (E.C. 3.2.1.3), Hemicellulasen, Proteasen (z. B. Subtilisin (E.C. 3.4.21.62) oder einem Bacillolysin (E.C. 3.4.24.28) oder einer alkalischen Serinprotease (E.C. 3.4.21.x) oder einer Keratinase (E.C. 3.4.x.x.) oder einer säurestabilen Protease, entzweigenden Enzymen, Cutinasen, Esterasen, Mannanasen (z B. einer 13-Mannanase (E.C. 3.2.1.78)) oder irgendeiner Kombination davon.

## Revendications

1. Enzyme xylanase ayant au moins 70 % d'identité de séquence d'acides aminés vis-à-vis de la séquence d'acides aminés de SEQ ID n°: 21, l'enzyme xylanase comprenant les acides aminés 7D, 33V, 79Y, 217Q et 298Y aux positions correspondant à la numérotation des acides aminés de SEQ ID n°: 21 et l'enzyme xylanase présentant une activité xylanase et étant capable d'accroître la récupération de l'huile d'un matériau à base de grains tel que comparé à l'enzyme xylanase de SEQ ID n°: 1.

2. Enzyme xylanase selon la revendication 1 dans laquelle la récupération accrue d'huile est mesurable à la fin de la récupération d'huile de fermentation.

3. Enzyme xylanase selon la revendication 1 ou la revendication 2, dans laquelle l'enzyme xylanase comprend l'une des combinaisons suivantes des acides aminés aux positions correspondant à la numérotation des acides aminés de SEQ ID n°: 21:
A: 7D, 25P, 33V, 57Q, 62T, 79Y, 89G, 103M, 115L, 147Q, 181Q, 193Y, 217Q, 219P et 298Y;
B: 7D, 25P, 33V, 57Q, 62T, 64T, 79Y, 103M, 115L, 147Q, 181Q, 193Y, 217Q, 219P et T298Y; ou
C: 7D, 25P, 33V, 79Y, 89G, 217Q, et 298Y; ou
D: 7D, 33V, 57Q, 62T, 64T, 79Y, 89G, 217Q et T298Y; ou
E: 7D, 25P, 33V, 64T, 79Y, 89G, 217Q et 298Y.

4. Enzyme xylanase selon l'une quelconque des revendications précédentes, dans laquelle l'enzyme présente au moins 80 % d'identité de séquence d'acides aminés vis-à-vis de la séquence d'acides aminés de SEQ ID n°: 21.

5. Enzyme xylanase selon l'une quelconque des revendications précédentes, dans laquelle l'enzyme présente au moins 90 % d'identité de séquence d'acides aminés vis-à-vis de la séquence d'acides aminés de SEQ ID n°: 21.

6. Enzyme xylanase selon l'une quelconque des revendications précédentes, dans laquelle l'enzyme présente au moins 95 % d'identité de séquence d'acides aminés vis-à-vis de la séquence d'acides aminés de SEQ ID n°: 21.

7. Procédé de récupération d'huile, comprenant:
(a) la liquéfaction d'un matériau à base de grains en dextrines avec une alpha-amylase;
(b) la saccharification des dextrines en utilisant une enzyme générant une source d'hydrate de carbone pour former un sucre;
(c) la fermentation du sucre dans un milieu de fermentation en un produit de fermentation utilisant un organisme de fermentation;
(d) la distillation du produit de fermentation pour former un résidu de distillation complet;
(e) la séparation du résidu de distillation complet en résidu de distillation fin et tourteau humide; et
(f) la récupération de l'huile du résidu de distillation fin, où au moins une ou plusieurs parmi la liquéfaction, la saccharification et la fermentation comprend le mélange par admixtion d'une xylanase selon l'une quelconque des revendications 1 à 6.

8. Procédé selon la revendication 7, comprenant en outre une étape de trempage et/ou une étape de transformation post-fermentation où ladite enzyme xylanase modifiée est mélangée par admixtion.

9. Procédé de récupération d'huile dans un procédé simultané de saccharification et de fermentation (SSF), comprenant:
(a) le mélange par admixtion d'une alpha-amylase, d'une source d'hydrate de carbone générant une enzyme et d'un organisme de fermentation à un matériau à base de grains dans un milieu de fermentation;
(b) l'exécution de SSF pour former un sucre dans le milieu de fermentation et en outre la fermentation du sucre en un produit de fermentation en utilisant l'organisme de fermentation;
(c) la distillation du produit de fermentation pour former un résidu de distillation complet;
(d) la séparation du résidu de distillation complet en résidu de distillation fin et tourteau humide; et
(e) la récupération de l'huile du résidu de distillation fin, où l'exécution de la SSF comprend le mélange par admixtion d'une xylanase selon l'une quelconque des revendications 1 à 6.

10. Procédé de récupération d'huile dans un procédé enzymatique d'hydrolyse d'amidon granuleux (GSHE), comprenant:
(a) la mise en contact d'un matériau à base de grains comprenant de l'amidon granuleux à une température située au niveau ou en-dessous de la température de gélatinisation de l'amidon granuleux avec une ou plusieurs enzymes GSHE et une xylanase selon l'une quelconque des revendications 1 à 6;
(b) le fait de permettre à l'alpha amylase, à l'enzyme GSHE et à la xylanase d'agir sur une durée comprise entre 2 et 100 heures pour obtenir un sirop de sucre fermentescible;
(c) la fermentation du sirop de sucre en un produit de fermentation en utilisant un organisme de fermentation;
(d) la distillation du produit de fermentation pour former un résidu de distillation complet;
(e) la séparation du résidu de distillation complet en résidu de distillation fin et tourteau humide; et
(f) la récupération de l'huile du résidu de distillation fin.

11. Procédé selon la revendication 10, dans lequel ledit sirop de sucre est sélectionné dans le groupe constitué d'un disaccharide, d'un oligosaccharide, d'un polysaccharide, de glucose, et de maltose et/ou le matériau à base de grains comprend du grain et/ou une céréale.

12. Procédé selon l'une quelconque des revendications 7 à 11, dans lequel l'enzyme xylanase est utilisée en combinaison avec une ou plusieurs des enzymes sélectionnées parmi les endoglucanases (E.C.3.2.1.4), les cellobiohydrolases (E.C. 3.2.1.91), les 13-glucosidases (E.C. 3.2.1.21), les 35-cellulases (E.C. 3.2.1.74), les lichénases (E.C. 3.1.1.73), les lipases (E.C. 3.1.1.3), les lipides acyltransférases (généralement classées comme E.C. 2.3.1.x), les phospholipases (E.C. 3.1.1.4, E.C. 3.1.1.32 ou E.C.3.1.1.5), les phytases (par exemple la 6-phytase (E.C. 3.1.3.26) ou une 3-phytase (E.C. 3.1.3.8)), les amylases, les alpha-amylases (E.C. 3.2.1.1), les autres xylanases (E.C. 3.2.1.8, E.C. 3.2.1.32, E.C. 3.2.1.37, E.C. 3.1.1.72, E.C. 3.1.1.73), les glucoamylases (E.C. 3.2.1.3), les hémicellulases, les protéases (par exemple la subtilisine (E.C. 3.4.21.62) ou une bacillolysine (E.C. 3.4.24.28) ou une sérine protéase alcaline (E.C. 3.4.21.x) ou une kératinase (E.C. 3.4.x.x) ou une protéase stable dans les acides), les enzymes de déramification, les cutinases, les estérases, les mannanases (par exemple une 13-mannanase (E.C. 3.2.1.78)) ou n'importe quelles combinaisons de celles-ci.
